(19) Europäisches Patentamt
European Patent Office
Office européen des brevets

(11) EP 2 133 347 A1

(12) EUROPEAN PATENT APPLICATION
published in accordance with Art. 153(4) EPC

(43) Date of publication:
**16.12.2009 Bulletin 2009/51**

(21) Application number: **08721668.5**

(22) Date of filing: **04.03.2008**

(51) Int Cl.:
***C07D 401/10*** (2006.01) ***A61K 31/397*** (2006.01)
***A61K 31/404*** (2006.01) ***A61K 31/4427*** (2006.01)
***A61K 31/497*** (2006.01) ***A61K 31/506*** (2006.01)
***A61K 31/706*** (2006.01) ***A61P 3/06*** (2006.01)
***A61P 9/10*** (2006.01) ***C07D 401/14*** (2006.01)
***C07D 403/10*** (2006.01) ***C07D 405/14*** (2006.01)
***C07D 409/14*** (2006.01) ***C07H 15/26*** (2006.01)

(86) International application number:
**PCT/JP2008/054252**

(87) International publication number:
**WO 2008/108486 (12.09.2008 Gazette 2008/37)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **06.03.2007 JP 2007055923**
**02.08.2007 JP 2007202109**

(71) Applicant: **Teijin Pharma Limited**
**Tokyo 100-0013 (JP)**

(72) Inventors:
- **NAKANO, Akira**
**Hino-shi**
**Tokyo 191-0065 (JP)**
- **NAKAJIMA, Ryota**
**Hino-shi**
**Tokyo 191-0065 (JP)**
- **KANOU, Masanobu**
**Hino-shi**
**Tokyo 191-0065 (JP)**
- **HISAMATSU, Seiichi**
**Hino-shi**
**Tokyo 191-0065 (JP)**
- **KUROKAWA, Masayuki**
**Hino-shi**
**Tokyo 191-0065 (JP)**
- **NISHIWAKI, Yasumi**
**Hino-shi**
**Tokyo 191-0065 (JP)**

(74) Representative: **Hallybone, Huw George et al**
**Carpmaels & Ransford**
**43-45 Bloomsbury Square**
**London WC1A 2RA (GB)**

# (54) 1-BIARYLAZETIDINONE DERIVATIVES

(57) The object of the present invention is to provide a compound having an excellent cholesterol-lowering effect and to provide a drug for the treatment, prevention of onset, or prevention of progress of lipid metabolism disorder, hyperlipidemia, or atherosclerosis.

A compound represented by formula (I) or a pharmaceutically acceptable salt thereof; or a medicament or a pharmaceutical composition, which contain the compound or the salt as an active ingredient.

EP 2 133 347 A1

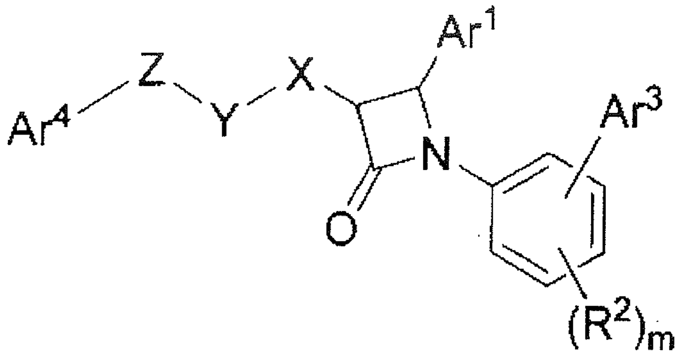

Formula (I)

## Description

[Technical Field]

The present invention relates to a novel azetidinone compound, a production method thereof, and a cholesterol-lowering agent containing the compound as an active ingredient. In particular, the present invention relates to a compound useful for the treatment, prevention of onset, or prevention of progress of lipid metabolism disorder, hyperlipidemia, or atherosclerosis.

[Background Art]

Coronary artery diseases due to atherosclerosis are the major cause of death and cardiovascular pathological conditions in Europe and the United States. Hyperlipidemic symptoms associated with increases in total cholesterol (TC) and low-density lipoprotein-cholesterol (LDL-C) levels are a major risk factor for cardiovascular atherosclerotic diseases.

Control of systemic cholesterol homeostasis depends on a total balance between the endogenous pathway and the exogenous pathway of cholesterol metabolism. In the endogenous pathway, cholesterol is biosynthesized in the liver and absorbed into the bloodstream as a lipoprotein, and in the exogenous pathway, cholesterol derived from diet and bile is absorbed from the intestine into the bloodstream as a chylomicron component. Any change occurring in either of the pathways affects the blood cholesterol level.

Statin drugs are the main stream of anti-hypercholesterolemic agents. Statin drugs inhibit β-hydroxy-β-methylglutalyl-CoA (HMG-CoA) reductase, the rate-limiting enzyme of cholesterol biosynthesis, to upregulate the expression of LDL receptor and accelerate elimination of cholesterol from the blood to exhibit a potent cholesterol-lowering effect. However, since a rate of achieving a target value for LDL-C control is not necessarily high among patients including patients with a high risk for coronary artery diseases, further lowering of cholesterol levels is desired. In addition, their activities of lowering blood triglyceride (TG) levels and increasing high-density lipoprotein-cholesterol (HDL-C) levels are not very high.

Ezetimibe ((3R,4S)-3-[(3S)-3-(4-fluorophenyl)-3-hydroxypropyl]-1-(4-fluorophenyl)-4-(4-hydroxyphenyl)azetidin-2-one) is known to be an agent for the treatment of hyperlipidemia, which suppresses absorption of cholesterol derived from diet and bile in the intestine to achieve a lowering of cholesterol levels in the blood based on the mechanism different from that of statin drugs (International Publication WO95/08532; and André J. Tremblay, Arterioscler. Thromb. Vasc. Biol. 26, 1101-1106, 2006). When absorption of cholesterol from the intestine decreases, supply of cholesterol to the liver decreases. It is considered that production of very low-density lipoprotein (VLDL) in the liver thus decreases so that the drug exhibits an effect of lowering LDL. Ezetimibe is shown to have a cholesterol-lowering effect when used alone and its efficacy as a drug used in combination with a statin drug is also shown. By the way, Ezetimibe has a limited LDL cholesterol-lowerling effect of around 15-20%, either alone or in the presence of a statin (V.Charlton-Menys, Exp. Physiol. 93, 1, 27-42, 2007).

Fibrate drugs are used as agents for the treatment of hypertriglyceremia. Fibrate drugs suppress the synthesis and secretion of TG in the liver to exhibit activities of lowering LDL-C and TG levels and elevating an HDL-C level. Their activity to reduce a total cholesterol level in the blood is not very high, however. When used with statin drugs, it is necessary for coadministration to appropriate precautions against the problem concerning adverse drug interactions.

A significant proportion of hypercholesterolemic patients cannot achieve their target cholesterol levels or cannot use a drug for a long period of time required to achieve their target levels from the viewpoint of drug interaction or drug safety. Accordingly, development of more potent and highly tolerant drugs has still been required. In addition, a blockage effect on the onset of coronary artery disease is obtained only in about 30% by the treatment of hyperlipidemia for which statin drugs are mainly used at present. Since involvement of factors other than LDL-C, that is, an increase in the blood TG level and a decrease in HDL-C level, in the onset of coronary artery disease has been shown (H Iso, Am. J. Epidemiol. 153, 490-499, 2001; and T Gordon, Arch Intern. Med. 141, 1128-1131, 1981) as the cause, therapeutic effects on these factors are also required.

Azetidinone derivatives have recently been reported to have an effect of lowering absorption of cholesterol (International Publication WO95/08532; International Publication WO93/002048; International Publication WO95/026334; International Publication WO95/035277; International Publication WO96/16037; International Publication WO2005/033100; International Publication WO2005/047248; International Publication WO2005/113495; International Publication WO2006/124713; and International Publication WO2006/121861). These compounds have structures different from that of the compound of the present invention, however.

International Publication WO2005/047248 describes data showing that an azetidinone derivative in which phenyl at 1-position of the azetidinone ring is further substituted with aryl exhibits a reduced cholesterol absorption lowering effect as compared with an azetidinone derivative in which phenyl at 1-position of the azetidinone ring is

unsubstituted or substituted with halogen.

[Disclosure of the Invention]

[Problems to be Solved by the Invention]

An object of the present invention is to provide a compound having an excellent cholesterol-lowering effect.
Another object of the present invention is to provide a compound useful for a treatment, prevention of onset, or prevention of progress of lipid metabolism disorder, hyperlipidemia, or atherosclerosis.

[Means for Solving the Problems]

The present inventors have keenly studied about compounds having a cholesterol-lowering effect and as a result, unexpectedly found that a compound represented by the following formula (I):

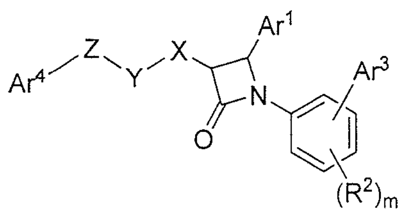

Formula (I)

which has such a feature in terms of a chemical structure that heteroaryl ($Ar^3$) is bonded to phenylene at 1-position of azetidinone and a salt thereof have excellent cholesterol-lowering effect and/or TG-lowering effect, and completed the present invention based on these findings.
In other words, the present invention relates the followings:

(1) A compound represented by the following formula (I) or a pharmaceutically acceptable salt thereof:

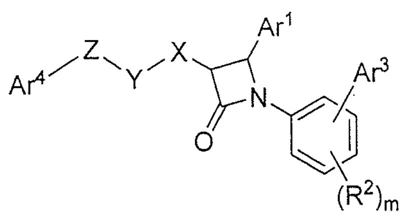

Formula (I)

wherein
X represents -$S(O)_r$- or -O-, with the proviso that r represents 0, 1, or 2;
$Ar^1$ represents aryl having 6 to 10 carbon atoms, or monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^1$;
$R^1$ represents a substituent independently selected from the group consisting of $C_1$-$C_6$ alkyl, $C_1$-$C_6$ alkyl substituted with one or more halogen, $C_1$-$C_6$ alkyl substituted with one or more -$OR^7$, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, -$NR^7SO_2R^{7a}$, -$SO_2NR^7R^{7a}$, -$S(O)_{0\text{-}2}R^7$, and glucuronide;
two independent $R^7$ or $R^7$ and $R^{7a}$ in the same $R^1$ may be bonded together to form 3 to 8-membered ring structure that may have 3 or less oxygen or nitrogen;
two adjacent independent $R^1$ may be bonded together to form 3 to 8-membered ring structure that may have 3 or less oxygen or nitrogen;
$R^2$ represents a substituent independently selected from the group consisting of $C_1$-$C_6$ alkyl, $C_1$-$C_6$ alkyl substituted with one or more halogen, halogen, cyano, and -$OR^7$;
m represents 0, 1, 2, 3, or 4;

$Ar^3$ represents monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^3$;
$R^3$ represents a substituent independently selected from the group consisting of $C_1$-$C_6$ alkyl, $C_1$-$C_6$ alkyl substituted with one or more halogen, $C_1$-$C_6$ alkyl substituted with one or more -$OR^7$, halogen, cyano, nitro, -$OR^7$, -$OCOR^7$, -$OCOOR^7$, -$OCONR^7R^{7a}$, -$COOR^7$, -$COR^7$, -$CONR^7R^{7a}$,- $NR^7R^{7a}$, -$NR^7COR^{7a}$, -$NR^7CONR^7R^{7a}$, -$NR^7SO_2R^{7a}$, -$SO_2NR^7R^{7a}$, and - $S(O)_{0-2}R^7$;
two independent $R^7$ or $R^7$ and $R^{7a}$ in the same $R^3$ may be bonded together to form 3 to 8-membered ring structure that may have 3 or less oxygen or nitrogen;
two adjacent independent $R^3$ may be bonded together to form 3 to 8-membered ring structure that may have 3 or less oxygen or nitrogen;
$Ar^4$ represents aryl having 6 to 10 carbon atoms or monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^4$;
$R^4$ represents a substituent independently selected from the group consisting of $C_1$-$C_6$ alkyl, $C_1$-$C_6$ alkyl substituted with one or more halogen, $C_1$-$C_6$ alkyl substituted with one or more -$OR^7$, $C_1$-$C_6$ alkylene, halogen, cyano, nitro, -$OR^7$, -$OCOR^7$, -$OCOOR^7$, $OCONR^7R^{7a}$, - $COOR^7$, -$COR^7$, -$CONR^7R^{7a}$, -$NR^7R^{7a}$, -$NR^7COR^{7a}$, -$NR^7CONR^7R^{7a}$, - $NR^7SO^2R^{7a}$, -$SO_2NR^7R^{7a}$, -$S(O)_{0-2}R^7$, aryl having 6 to 10 carbon atoms, which may be substituted with 1 to 5 same or different $R^8$, and monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^8$;
two independent $R^7$ or $R^7$ and $R^{7a}$ in the same $R^4$ may be bonded together to form 3 to 8-membered ring structure that may have 3 or less oxygen or nitrogen;
two adjacent independent $R^4$ may be bonded together to form 3 to 8-membered ring structure that may have 3 or less oxygen or nitrogen;
Y represents a bond or -$CR^5R^{5a}$-;
$R^5$ and $R^{5a}$ each independently represents a substituent selected from the group consisting of hydrogen, $C_1$-$C_6$ alkyl, and halogen;
Z represents -$CR^6R^{6a}$-;
$R^6$ and $R^{6a}$ each independently represents a substituent selected from the group consisting of hydrogen, $C_1$-$C_6$ alkyl, $C_1$-$C_6$ alkyl substituted with one or more halogen, halogen, -$OR^7$, -$OCOR^7$, -$OCOOR^7$, -$OCONR^7R^{7a}$, - $NR^7R^{7a}$, -$NR^7COR^{7a}$, and -$NR^7CONR^7R^{7a}$, or $R^6$ and $R^{6a}$ may together form oxo group;
$R^7$ and $R^{7a}$ each independently represents hydrogen, $C_1$-$C_6$ alkyl, $C_1$-$C_6$ alkyl substituted with one or more halogen, $C_1$-$C_6$ alkyl substituted with one or more $C_1$-$C_6$ alkoxy, $C_1$-$C_6$ alkenyl, $C_1$-$C_6$ alkynyl, aryl having 6 to 10 carbon atoms, which may be substituted with 1 to 5 same or different $R^8$, monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^8$, or $C_7$-$C_{12}$ aralkyl, which may be substituted with 1 to 5 same or different $R^8$; and
$R^8$ represents $C_1$-$C_6$ alkyl, $C_1$-$C_6$ alkyl substituted with one or more halogen, $C_1$-$C_6$ alkyl substituted with one or more $C_1$-$C_6$ alkoxy, $C_1$-$C_6$ alkoxy, or halogen.
(2) The compound or the pharmaceutically acceptable salt thereof according to (1), wherein
X represents -$S(O)_r$- or -O-, with the proviso that r represents 0, 1, or 2;
$Ar^1$ represents aryl having 6 to 10 carbon atoms, or monocyclic or bicyclic heteroaryl having 2 to 9 carbon atoms with 1 to 4 heteroatoms selected from the group consisting of oxygen, sulfur, and nitrogen, which may be substituted with 1 to 5 same or different $R^1$;
$R^1$ represents a substituent independently selected from the group consisting of $C_1$-$C_6$ alkyl, $C_1$-$C_6$ alkyl substituted with one or more halogen, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, -$NR^7SO_2R^{7a}$, and glucuronide;
$R^2$ represents a substituent independently selected from the group consisting of $C_1$-$C_6$ alkyl, $C_1$-$C_6$ alkyl substituted with one or more halogen, halogen, cyano, and- $OR^7$;
m represents 0, 1, 2, 3, or 4;
$Ar^3$ represents monocyclic or bicyclic heteroaryl having 2 to 9 carbon atoms with 1 to 4 heteroatoms selected from the group consisting of oxygen, sulfur, and nitrogen, which may be substituted with 1 to 5 same or different $R^3$;
$R^3$ represents a substituent independently selected from the group consisting of $C_1$-$C_6$ alkyl, $C_1$-$C_6$ alkyl substituted with one or more halogen, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, and -$NR^7SO_2R^{7a}$;
$Ar^4$ represents aryl having 6 to 10 carbon atoms or monocyclic or bicyclic heteroaryl having 2 to 9 carbon atoms with 1 to 4 heteroatoms selected from the group consisting of oxygen, sulfur, and nitrogen, which may be substituted with 1 to 5 same or different $R^4$;

$R^4$ represents a substituent independently selected from the group consisting of $C_1$-$C_6$ alkyl, $C_1$-$C_6$ alkyl substituted with one or more halogen, $C_1$-$C_6$ alkylene, halogen, cyano, nitro, -$OR^7$, -$OCOR^7$, -$OCOOR^7$, -$OCONR^7R^{7a}$, - $COOR^7$, -$COR^7$, -$CONR^7R^{7a}$, -$NR^7R^{7a}$, -$NR^7COR^{7a}$, -$NR^7CONR^7R^{7a}$, and -$NR^7SO_2R^{7a}$;
two independent $R^7$ or $R^7$ and $R^{7a}$ in the same $R^4$ may be bonded together to form 3 to 8-membered ring structure that may have 3 or less oxygen or nitrogen;
two adjacent independent $R^4$ may be bonded together to form 3 to 8-membered ring structure that may have 3 or less oxygen or nitrogen;
Y represents a bond or -$CR^5R^{5a}$-;
$R^5$ and $R^{5a}$ each independently represents a substituent selected from the group consisting of hydrogen, $C_1$-$C_6$ alkyl, and halogen;
Z represents -$CR^6R^{6a}$-;
$R^6$ and $R^{6a}$ each independently represents a substituent selected from the group consisting of hydrogen, $C_1$-$C_6$ alkyl, $C_1$-$C_6$ alkyl substituted with one or more halogen, halogen, -$OR^7$, -$OCOR^7$, -$OCOOR^7$, -$OCONR^7R^{7a}$, -$NR^7R^{7a}$, -$NR^7COR^{7a}$, and -$NR^7CONR^7R^{7a}$, or $R^6$ and $R^{6a}$ may together form oxo group; and
$R^7$ and $R^{7a}$ each independently represents hydrogen, $C_1$-$C_6$ alkyl, $C_1$-$C_6$ alkyl substituted with one or more halogen, or $C_1$-$C_6$ alkyl substituted with one or more $C_1$-$C_6$ alkoxy.

(3) The compound or the pharmaceutically acceptable salt thereof according to (1) or (2), wherein
$Ar^1$ represents phenyl, pyridyl, pyrimidyl, or pyridazinyl, which may be substituted with 1 to 5 same or different $R^1$, and
$R^1$ represents a substituent independently selected from the group consisting of $C_1$-$C_6$ alkyl, $C_1$-$C_6$ alkyl substituted with one or more halogen, halogen, -$OR^7$, -$COOR^7$, and glucuronide.

(4) The compound or the pharmaceutically acceptable salt thereof according to (1) or (2), wherein
$Ar^1$ represents phenyl or pyridyl, of which is substituted with at least one hydroxy.

(5) The compound or the pharmaceutically acceptable salt thereof according to (1) or (2), wherein
$Ar^1$ represents phenyl, of which 4-position is substituted with at least one hydroxy.

(6) The compound or the pharmaceutically acceptable salt thereof according to any of (1) to (5), wherein
$R^2$ represents halogen.

(7) The compound or the pharmaceutically acceptable salt thereof according to any of (1) to (5), wherein
m represents 0.

(8) The compound or the pharmaceutically acceptable salt thereof according to any of (1) to (7), wherein
$Ar^3$ represents pyridyl, pyrazyl, pyrimidyl, pyridazinyl, furanyl, thienyl, pyrrolidinyl, pyrazolyl, isoxazolyl, isothiazolyl, benzofuranyl, isobenzofuranyl, benzothienyl, indolyl, isoindolyl, benzothienyl, benzimidazolyl, or benzoxazolyl, which is bonded to 4-position of phenylene to which the $Ar^3$ is bonded and may be substituted with 1 to 5 same or different $R^3$, and
$R^3$ represents a substituent independently selected from the group consisting of $C_1$-$C_6$ alkyl, $C_1$-$C_6$ alkyl substituted with one or more halogen, halogen, cyano, -$OR^7$, -$COOR^7$, -$COR^7$, -$CONR^7R^{7a}$, and -$NR^7R^{7a}$.

(9) The compound or the pharmaceutically acceptable salt thereof according to any of (1) to (7), wherein
$Ar^3$ represents pyridyl, pyrazyl, or pyrimidyl, which is bonded to 4-position of phenylene to which the $Ar^3$ is bonded and may be substituted with 1 to 4 same or different $R^3$, and
$R^3$ represents a substituent independently selected from the group consisting of $C_1$-$C_6$ alkyl, $C_1$-$C_6$ alkyl substituted with one or more halogen, halogen, cyano, -$OR^7$, -$COR^7$, -$CONR^7R^{7a}$, and -$NR^7R^{7a}$.

(10) The compound or the pharmaceutically acceptable salt thereof according to any of (1) to (7), wherein
$Ar^3$ represents 3-pyridyl, 2-pyrazyl, or 5-pyrimidyl, which is bonded to 4-position of phenylene to which the $Ar^3$ is bonded and may be substituted with 1 to 4 same or different $R^3$.

(11) The compound or the pharmaceutically acceptable salt thereof according to any of (1) to (7), wherein
$Ar^3$ represents 3-pyridyl, which is bonded to 4-position of phenylene to which the $Ar^3$ is bonded and may be substituted with 1 to 4 same or different $R^3$, and
$R^3$ represents a substituent independently selected from the group consisting of $C_1$-$C_6$ alkyl, $C_1$-$C_6$ alkyl substituted with one or more halogen, halogen, cyano, -$OR^7$, -$COR^7$, -$CONR^7R^{7a}$, and -$NR^7R^{7a}$.

(12) The compound or the pharmaceutically acceptable salt thereof according to any of (1) to (11), wherein
X represents -S-,
Y represents -$CH_2$-, and
Z represents -CH(OH)- or -CO-.

(13) The compound or the pharmaceutically acceptable salt thereof according to any of (1) to (12), wherein
$Ar^4$ represents phenyl, pyridyl, furanyl, thienyl, thiazolyl, naphthyl, benzofuranyl, benzothienyl, or benzothiazolyl, which may be substituted with 1 to 5 same or different $R^4$, and
$R^4$ represents a substituent independently selected from the group consisting of $C_1$-$C_6$ alkyl, $C_1$-$C_6$ alkyl substituted with one or more halogen, halogen, cyano, nitro, -$OR^7$, -$CONR^7R^{7a}$, -$NR^7R^{7a}$, -$NR^7COR^{7a}$, $C_1$-$C_4$ alkyleneoxy, and $C_1$-$C_3$ alkylenedioxy.

(14) The compound or the pharmaceutically acceptable salt thereof according to any of (1) to (12), wherein $Ar^4$ represents phenyl substituted with 1 to 5 same or different $R^4$, and $R^9$ represents a substituent independently selected from the group consisting of $C_1$-$C_6$ alkyl, $C_1$-$C_6$ alkyl substituted with one or more halogen, halogen, cyano, hydroxy, $C_1$-$C_4$ alkyleneoxy, and $C_1$-$C_3$ alkylenedioxy.

(15) The compound or the pharmaceutically acceptable salt thereof according to any of (1) to (12), wherein $Ar^4$ represents unsubstantiated benzofuranyl.

(16) The compound or the pharmaceutically acceptable salt thereof according to (1) or (2), wherein
$Ar^1$ represents 4-hydroxyphenyl, 2,4-dihydroxyphenyl, 4-hydroxy-2-methylphenyl, 4-hydroxy-2-methoxyphenyl, or 2-chloro-4-hydroxyphenyl;
m represents 0;
$Ar^3$ represents pyridyl, pyrazyl, or pyrimidyl, which is bonded to 4-position of phenylene to which the $Ar^3$ is bonded and may be substituted with 1 to 4 same or different $R^3$;
$R^3$ represents a substituent independently selected from the group consisting of $C_1$-$C_6$ alkyl, $C_1$-$C_6$ alkyl substituted with one or more halogen, halogen, cyano, - $OR^7$, $-COR^7$, $-CONR^7R^{7a}$, and $-NR^7R^{7a}$;
$Ar^4$ represents phenyl substituted with 1 to 5 same or different $R^4$;
$R^4$ represents a substituent independently selected from the group consisting of $C_1$-$C_6$ alkyl, $C_1$-$C_6$ alkyl substituted with one or more halogen, halogen, cyano, hydroxy, $C_1$-$C_4$ alkyleneoxy, and $C_1$-$C_3$ alkylenedioxy;
X represents -S-;
Y represents $-CH_2-$; and
Z represents -CH(OH)- or -CO-.

(17) The compound or the pharmaceutically acceptable salt thereof according to (1) or (2), wherein
$Ar^1$ represents 4-hydroxyphenyl, 2,4-dihydroxyphenyl, 4-hydroxy-2-methylphenyl, 4-hydroxy-2-methoxyphenyl, or 2-chloro-4-hydroxyphenyl;
m represents 0;
$Ar^3$ represents pyridyl, pyrazyl, or pyrimidyl, which is bonded to 4-position of phenylene to which the $Ar^3$ is bonded and may be substituted with 1 to 4 same or different $R^3$;
$R^3$ represents a substituent independently selected from the group consisting of $C_1$-$C_6$ alkyl, $C_1$-$C_6$ alkyl substituted with one or more halogen, halogen, cyano, - $OR^7$, $-COR^7$, $-CONR^7R^{7a}$, and $-NR^7R^{7a}$;
$Ar^4$ represents phenyl substituted with one or more halogen;
X represents -S-;
Y represents $-CH_2-$; and
Z represents -CH(OH)-.

(18) The compound or the pharmaceutically acceptable salt thereof according to (1) or (2), wherein
$Ar^1$ represents 4-hydroxyphenyl, 2,4-dihydroxyphenyl, 4-hydroxy-2-methylphenyl, 4-hydroxy-2-methoxyphenyl, or 2-chloro-4-hydroxyphenyl;
m represents 0;
$Ar^3$ represents 3-pyridyl, 2-pyrazyl, or 5-pyrimidyl, which is bonded to 4-position of phenylene to which the $Ar^3$ is bonded and may be substituted with 1 to 4 same or different $R^3$;
$R^3$ represents a substituent independently selected from the group consisting of $C_1$-$C_6$ alkyl, $C_1$-$C_6$ alkyl substituted with one or more halogen, halogen, cyano,
$-OR^7$, $-COR^7$, $-CONR^7R^{7a}$, and $-NR^7R^{7a}$;
$Ar^4$ represents phenyl substituted with 1 to 5 same or different $R^4$;
$R^4$ represents a substituent independently selected from the group consisting of $C_1$-$C_6$ alkyl, $C_1$-$C_6$ alkyl substituted with one or more halogen, halogen, cyano, hydroxy, $C_1$-$C_4$ alkyleneoxy, and $C_1$-$C_3$ alkylenedioxy;
X represents -S-;
Y represents $-CH_2-$; and
Z represents -CH(OH)-.

(19) (3R,4R)-3-[(2R)-2-(4-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxyphenyl)-1-[4-(2-methoxypyridin-3-yl)phenyl] azetidin-2-one,
(3R,4R)-3-[(2R)-2-(4-fluorophenyl)-2-hydroxyethylthio]-4-(4-hydroxyphenyl)-1-[4-(pyrimidin-5-yl)phenyl]azetidin-2-one,
(3R,4R)-3-[(2R)-2-(4-fluorophenyl)-2-hydroxyethylthio]-4-(4-hydroxyphenyl)-1-[4-(4-methoxypyridin-3-yl)phenyl] azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbonitrile,
(3R,4R)-3-[(2R)-2-(4-fluorophenyl)-2-hydroxyethylthio]-4-(4-hydroxyphenyl)-1-[4-(6-methylpyridin-3-yl)phenyl]azetidin-2-one,
(3R,4R)-3-[(2R)-2-(4-fluorophenyl)-2-hydroxyethylthio]-1-[4-(6-dimethylaminopyridin-3-yl)phenyl]-4-(4-hydroxyphenyl)azetidin-2-one,
(3R,4R)-3-[(2R)-2-(4-fluorophenyl)-2-hydroxyethylthio]-4-(4-hydroxy-2-methoxyphenyl)-1-[4-(pyridin-3-yl)phenyl] azetidin-2-one,
(3R,4R)-3-[2-(4-fluorophenyl)-2-oxoethylthio]-4-(4-hydroxyphenyl)-1-[4-(pyridin-3-yl)phenyl]azetidin-2-one,
(3R,4R)-3-[(2R)-2-(3,4-dichlorophenyl)-2-hydroxyethylthio]-4-(4-hydroxyphenyl)-1-[4-(pyridin-3-yl)phenyl]azetidin-2-one,
(3R,4R)-3-[(2R)-2-(2,4-difluorophenyl)-2-hydroxyethylthio]-4-(4-hydroxyphenyl)-1-[4-(pyridin-3-yl)phenyl]azetidin-2-one,
(3R,4R)-3-((2R)-2-benzo[b]furan-5-yl-2-hydroxyethylthio)-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyridin-3-yl)phenyl]azetidin-2-one,
(3R,4R)-3-[(2R)-2-(2H-benzo[3,4-d]1,3-dioxolen-5-yl)-2-hydroxyethylthio]-4-(4-hydroxyphenyl)-1-[4-(pyridin-3-yl) phenyl]azetidin-2-one,
(3R,4R)-3-[(2R)-2-(4-fluorophenyl)-2-hydroxyethylthio]-4-(4-hydroxy-2-methylphenyl)-1-[4-(pyridin-3-yl)phenyl] azetidin-2-one,
(3R,4R)-3-[(2R)-2-hydroxy-2-(4-methylphenyl)ethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-1-[4-(5-acetylpyridin-3-yl)phenyl]-4-(4-hydroxyphenyl)azetidin-2-one,
N-ethyl-5-(4-{(3R,4R)-3-[(2R)-2-(4-fluorophenyl)-2-hydroxyethylthio]-4-(4-hydroxyphenyl)-2-oxoazetidinyl}phenyl) pyridine-3-carboxamide,
(3R,4R)-3-[(2R)-2-(4-fluorophenyl)-2-hydroxyethylthio]-4-(4-hydroxyphenyl)-1-[4-(5-methoxypyridin-3-yl)phenyl] azetidin-2-one,
(3R,4R)-3-[(2R)-2-(4-fluorophenyl)-2-hydroxyethylthio]-4-(4-hydroxyphenyl)-1-[4-(5-trifluoromethylpyridin-3-yl) phenyl]azetidin-2-one,
(3R,4R)-3-[(2R)-2-(4-fluorophenyl)-2-hydroxyethylthio]-4-(4-hydroxyphenyl)-1-[4-(pyrazin-2-yl)phenyl]azetidin-2-one,
(3R,4R)-3-[(2R)-2-(4-fluorophenyl)-2-hydroxyethylthio]-1-[4-(5-fluoropyridin-3-yl)phenyl]-4-(4-hydroxyphenyl)azetidin-2-one,
(3R,4R)-3-[(2R)-2-(4-fluorophenyl)-2-hydroxyethylthio]-1-[4-(5-chloropyridin-3-yl)phenyl]-4-(4-hydroxyphenyl)azetidin-2-one,
5-(4-{(3R,4R)-3-[(2R)-2-(4-chlorophenyl)-2-hydroxyethylthio]-4-(4-hydroxyphenyl)-2-oxoazetidinyl}phenyl)pyridine-3-carbonitrile,
(3R,4R)-3-[(2R)-2-(4-fluorophenyl)-2-hydroxyethylthio]-4-(2-chloro-4-hydroxyphenyl)-1-[4-(pyridin-3-yl)phenyl] azetidin-2-one,
5-(4-{(3R,4R)-3-[(2R)-2-(4-chlorophenyl)-2-hydroxyethylthio]-4-(4-hydroxyphenyl)-2-oxoazetidinyl}phenyl)pyridine-2-carbonitrile,
(3R,4R)-3-[(2R)-2-(4-chlorophenyl)-2-hydroxyethylthio]-4-(2,4-dihydroxyphenyl)-1-[4-(pyridin-3-yl)phenyl]azetidin-2-one,
5-(4-{(3R,4R)-3-[(2R)-2-(4-fluorophenyl)-2-hydroxyethylthio]-4-(2,4-dihydroxyphenyl)-2-oxoazetidinyl}phenyl)pyridine-3-carbonitrile,
(3R,4R)-3-[(2R)-2-(4-fluorophenyl)-2-hydroxyethylthio]-4-(2,4-dihydroxyphenyl)-1-[4-(4-methoxypyridin-3-yl)phenyl]azetidin-2-one,
5-(4-{(3R,4R)-3-[(2R)-2-(4-chlorophenyl)-2-hydroxyethylthio]-4-(2,4-dihydroxyphenyl)-2-oxoazetidinyl}phenyl)pyridine-3-carbonitrile,
(3R,4R)-3-[(2R)-2-(4-fluorophenyl)-2-hydroxyethylthio]-4-(4-hydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyrimidin-5-yl)phenyl]azetidin-2-one,
(3R,4R)-3-[(2R)-2-(4-chlorophenyl)-2-hydroxyethylthio]-1-[4-(5-fluoropyridin-3-yl)phenyl]-4-(4-hydroxyphenyl)azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbaldehyde,

(3R,4R)-3-[(2R)-2-(4-chlorophenyl)-2-hydroxyethylthio]-4-(2,4-dihydroxyphenyl)-1-[4-(5-fluoropyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-fluoropyridin-3-yl)phenyl]azetidin-2-one,
(3R,4R)-3-[(2R)-2-(4-fluorophenyl)-2-hydroxyethylthio]-1-[4-(5-fluoropyridin-3-yl)phenyl]-4-(4-hydroxy-2-methylphenyl)azetidin-2-one,
(3R,4R)-3-[(2R)-2-(4-chlorophenyl)-2-hydroxyethylthio]-4-(2,4-dihydroxyphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-trifluoromethylpyridin-3-yl)phenyl]azetidin-2-one,
5-(4-{(3R,4R)-3-[(2R)-2-(4-fluorophenyl)-2-hydroxyethylthio]-4-(4-hydroxy-2-methylphenyl)-2-oxoazetidinyl}phenyl)pyridine-3-carbonitrile,
(3R,4R)-3-[(2R)-2-(4-chloro-3-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-chloro-3-fluorophenyl)-2-hydroxyethylthio]-1-[4-(5-fluoropyridin-3-yl)phenyl]-4-(4-hydroxyphenyl)azetidin-2-one,
(3R,4R)-3-((2R)-2-benzo[b]thiophen-5-yl-2-hydroxyethylthio)-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-trifluoromethylpyridin-3-yl)phenyl]azetidin-2-one,
(3R,4R)-3-[(2R)-2-(4-chlorophenyl)-2-hydroxyethylthio]-1-[4-(5-chloropyridin-3-yl)phenyl]-4-(4-hydroxyphenyl)azetidin-2-one,
(3R,4R)-3-[(2R)-2-(4-chlorophenyl)-2-hydroxyethylthio]-1-[4-(6-dimethylaminopyridin-3-yl)phenyl]-4-(4-hydroxyphenyl)azetidin-2-one,
(3R,4R)-3-[(2R)-2-(4-chloro-2-hydroxyphenyl)-2-hydroxyethylthio]-4-(4-hydroxyphenyl)-1-[4-(pyridin-3-yl)phenyl]azetidin-2-one,
(3R,4R)-3-[(2R)-2-(2-chloro-4-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(6-methylpyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-chloropyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-methylpyridin-3-yl)phenyl]azetidin-2-one,
(3R,4R)-3-[(2R)-2-(4-chlorophenyl)-2-hydroxyethylthio]-1-[4-(6-chloropyridin-3-yl)phenyl]-4-(4-hydroxyphenyl)azetidin-2-one,
(3R,4R)-3-[(2R)-2-(4-fluorophenyl)-2-hydroxyethylthio]-4-(2,4-dihydroxyphenyl)-1-[4-(6-methylpyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(5-chloropyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(6-methylpyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(pyrimidin-5-yl)phenyl]azetidin-2-one,
(3R,4R)-3-[(2R)-2-(4-chloro-2-methoxyphenyl)-2-hydroxyethylthio]-4-(4-hydroxyphenyl)-1-[4-(pyridin-3-yl)phenyl]azetidin-2-one,
(3R,4R)-3-[(2R)-2-(4-chloro-2-methylphenyl)-2-hydroxyethylthio]-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyrazin-2-yl)phenyl]azetidin-2-one,
(3R,4R)-3-[(2R)-2-(4-chlorophenyl)-2-hydroxyethylthio]-1-[4-(5-acetylpyridin-3-yl)phenyl]-4-(4-hydroxyphenyl)azetidin-2-one, or a pharmaceutically acceptable salt thereof.

(20) A pharmaceutical composition comprising the compound or the pharmaceutically acceptable salt thereof according to any of (1) to (19) as an active ingredient.
(21) A cholesterol-lowering agent comprising the compound or the pharmaceutically acceptable salt thereof according to any of (1) to (19) as an active ingredient.
(22) A medicament for lowering a cholesterol concentration in a blood comprising the compound or the pharmaceutically acceptable salt thereof according to any of (1) to (19) as an active ingredient.
(23) A medicament for a treatment, a prevention of onset, or a prevention of progress of lipid metabolism disorder, hyperlipidemia, or atherosclerosis, comprising the compound or the pharmaceutically acceptable salt thereof according to any of (1) to (19) as an active ingredient.

[Effect of the Invention]

The present invention provides a novel compound having an excellent cholesterol-lowering effect.
The present invention further provides a drug for a treatment, prevention of onset, or prevention of progress of lipid metabolism disorder, hyperlipidemia, or atherosclerosis.

[Best Mode for Carrying Out the Invention]

Terms used singly or in combination in the present specification will be explained below. Unless otherwise specified, explanation of the respective substituents is common for various positions.
In the present invention, "$C_1$-$C_6$ alkylene" refers to a divalent group formed by removing 2 hydrogen atoms from 2 different carbon atoms of a saturated linear or branched aliphatic hydrocarbon group having 1 to 6 carbon atoms and includes, for example, methylene, ethylene, trimethylene, tetramethylene, pentamethylene, hexamethylene and the like.
In the present invention, "$C_1$-$C_6$ alkyl" refers to a saturated linear or branched aliphatic hydrocarbon group having 1 to 6 carbon atoms and includes, for example, methyl, ethyl, n-propyl, n-butyl, n-pentyl, n-hexyl, isopropyl, isobutyl, s-butyl, t-butyl, isopentyl, 2-methylbutyl, neopentyl, 1-ethylpropyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 1-ethylbutyl, 2-ethylbutyl, t-pentyl, isohexyl and the like.
In the present invention, "$C_2$-$C_6$ alkenyl" refers to as a linear or branched aliphatic hydrocarbon group having 2 to 6 carbon atoms and having a double bond, and include, for example, vinyl, allyl, 1-propenyl, 2-butenyl, 3-butenyl, 2-methyl-1-propenyl, 2-methyl-2-propenyl, 4-pentenyl, 5-hexenyl, 4-methyl-3-pentenyl and the like.
In the present invention, "$C_2$-$C_6$ alkynyl" refers to as a linear or branched aliphatic hydrocarbon group having 2 to 6 carbon atoms and having a triple bond, and include, for example, ethynyl, propargyl, 3-methylpropargyl, butynyl, 2-butyn-1-yl, pentynyl, and hexynyl and the like.
In the present invention, "halogen" refers to fluorine, chlorine, bromine, iodine and the like.
In the present invention, "$C_1$-$C_6$ alkoxy" refers to a group formed by the above-described "$C_1$-$C_6$ alkyl" and oxygen, and include, for example, methoxy, ethoxy, n-propoxy, isopropoxy, n-butoxy, s-butoxy, 2-methylpropoxy, n-pentyloxy, isopentyloxy, 2-methylbutoxy, 1-ethylpropoxy, 2,2-dimethylpropoxy, n-hexyloxy, 4-methylpentoxy, 3-methylpentoxy, 2-methylpentoxy, 3,3-dimethylbutoxy, 2,2-dimethylbutoxy, 1,1-dimethylbutoxy, t-butoxy and the like.
In the present invention, "$C_1$-$C_4$ alkyleneoxy" refers to a group formed by an alkylene group having 1 to 4 carbon atoms among the $C_1$-$C_6$ alkylene described above and one oxy group. Examples thereof include ethyleneoxy ($-CH_2CH_2O-$), trimethyleneoxy ($-CH_2CH_2CH_2O-$), tetramethyleneoxy ($-CH_2CH_2CH_2CH_2O-$), 1,1-dimethylethyleneoxy ($-CH_2C(CH_3)_2O-$) and the like.
In the present invention, "$C_1$-$C_3$ alkylenedioxy" refers to a group formed by an alkylene group having 1 to 3 carbon atoms among the $C_1$-$C_6$ alkylene described above and two oxy groups. Examples thereof include methylenedioxy ($-OCH_2O-$), ethylenedioxy ($-OCH_2CH_2O-$), trimethylenedioxy ($-OCH_2CH_2CH_2O-$), propylenedioxy ($-OCH_2CH(CH_3)O-$) and the like.
In the present invention, "aryl having 6 to 10 carbon atoms" refers to a group formed by withdrawal of one hydrogen atom that is attached to an aromatic hydrocarbon ring having 6 to 10 carbon atoms. Examples thereof include phenyl, naphthyl, indenyl, tetrahydronaphthyl, indanyl, azulenyl and the like.
In the present invention, "$C_7$-$C_{12}$ aralkyl" refers to as a group formed by removing a hydrogen atom from the side chain of an aromatic hydrocarbon having a side chain with 7 to 12 carbon atoms, and include, for example, benzyl and phenetyl and the like.
In the present invention, "monocyclic or bicyclic heteroaryl having 2 to 9 carbon atoms with 1 to 4 heteroatoms selected from the group consisting of oxygen, sulfur, and nitrogen" refers to a monovalent group formed by removing a hydrogen atom from a monocyclic or bicyclic heteroaryl group having 2 to 9 carbon atoms with 1 to 4 heteroatoms selected from the group consisting of oxygen, sulfur, and nitrogen. Further, for bicyclic heteroaryl, when one ring is an

aromatic ring or an aromatic heterocycle, the other ring may have a non-aromatic ring structure. The numbers of the respective heteroatoms and their combination in the heteroaryl are not particularly limited, as long as they can constitute a ring of a determined number of ring members and are chemically stable. Examples of such a heteroaryl group include pyridyl, pyrazyl, pyrimidyl, pyridazinyl, furanyl, thienyl, pyrrolidinyl, pyrazolyl, isoxazolyl, isothiazolyl, benzofuranyl, isobenzofuranyl, benzothienyl, indolyl, isoindolyl, benzothiazolyl, benzimidazolyl, benzoxazolyl, pyranyl, imidazolyl, oxazolyl, thiazolyl, triazinyl, triazolyl, furazanyl, thiadiazolyl, dihydrobenzofuranyl, dihydroisobenzofuranyl, dihydroquinolyl, dihydroisoquinolyl, dihydrobenzoxazolyl, dihydropteridinyl, benzoxazolyl, benzisoxazolyl, benzodioxazolyl, quinolyl, isoquinolyl, benzotriazolyl, pteridinyl, purinyl, quinoxalinyl, quinazolinyl, cinnolinyl and the like. "Monocyclic or bicyclic heteroaryl having 1 to 9 carbon atoms with 1 to 4 heteroatoms selected from the group consisting of oxygen, sulfur, and nitrogen" in the present specification further comprises tetrazolyl in addition to "monocyclic or bicyclic heteroaryl having 2 to 9 carbon atoms with 1 to 4 heteroatoms selected from the group consisting of oxygen, sulfur, and nitrogen".

In the present invention, "glucuronide" refers to a glucuronic acid derivative. In the above formula (I), a hydroxy group not involved in binding to $Ar^1$ may be modified by an appropriate substituent well known by the person skilled in the art and a carboxyl group at 5-position may be esterified with lower alcohol. A preferred substituent of hydroxy is acetoxy, and a preferred ester is methyl ester.

In the present invention, as a "$C_1$-$C_6$ alkyl substituted with one or more halogen", $C_1$-$C_6$ alkyl substituted with 1 to 3 halogen are particularly preferable.

In the present invention, as a "$C_1$-$C_6$ alkyl substituted with one or more -$OR^7$", $C_1$-$C_6$ alkyl substituted with 1 to 6 same or different -$OR^7$ are particularly preferable.

In the present invention, as a "$C_1$-$C_6$ alkyl substituted with one or more $C_1$-$C_6$ alkoxy", $C_1$-$C_6$ alkyl substituted with 1 to 6 same or different $C_1$-$C_6$ alkoxy are particularly preferable.

In the present invention, "aryl having 6 to 10 carbon atoms, which may be substituted with 1 to 5 same or different $R^1$" refers that aryl may be substituted with same $R^1$ and may be substituted with different $R^1$, when the aryl is substituted with 2 to 5 $R^1$. Also, the meanings of "which may be substituted with 1 to 5 same or different $R^3$" and so on are the same meaning.

In the above formula (I), X represents -S(O)r- or -O- and r represents 0, 1, or 2. A specific example of preferred X is -S- among them.

In the above formula (I), Y represents a bond or - $CR^5R^{5a}$-, and $R^5$ and $R^{5a}$ each independently represent a substituent selected from the group consisting of hydrogen, $C_1$-$C_6$ alkyl, and halogen. A specific example of preferred Y among them is -$CH_2$-.

In the above formula (I), Z represents -$CR^6R^{6a}$-, $R^6$ and $R^{6a}$ each independently represents a substituent selected from the group consisting of hydrogen, $C_1$-$C_6$ alkyl, $C_1$-$C_6$ alkyl substituted with one or more halogen, halogen, -$OR^7$, -$OCOR^7$, -$OCOOR^7$, -$OCONR^7R^{7a}$, -$NR^7R^{7a}$, - $NR^7COR^{7a}$, and -$NR^7CONR^7R^{7a}$.

$R^7$ and $R^{7a}$ each independently represents hydrogen, $C_1$-$C_6$ alkyl, $C_1$-$C_6$ alkyl substituted with one or more halogen, or $C_1$-$C_6$ alkyl substituted with one or more $C_1$-$C_6$ alkoxy, $C_1$-$C_6$ alkenyl, $C_1$-$C_6$ alkynyl, aryl having 6 to 10 carbon atoms, which may be substituted with 1 to 5 same or different $R^8$, or monocyclic or bicyclic heteroaryl having 1 to 9 carbon atoms with 1 to 4 heteroatoms selected from the group consisting of oxygen, sulfur, and nitrogen, which may be substituted with same or different 1 to 5 $R^8$, or $C_7$-$C_{12}$ aralkyl, which may be substituted with same or different 1 to 5 $R^8$. For $R^7$ and $R^{7a}$, although specific examples of the "aryl" and "monocyclic or bicyclic heteroaryl" are as defined above, examples of preferred "aryl" or "monocyclic or bicyclic heteroaryl" includes phenyl, pyridyl, pyrimidyl and pyridazinyl, and phenyl and pyridyl are particularly preferable. In addition, although specific examples of the "$C_7$-$C_{12}$ aralkyl" are as defined above, examples of preferred "$C_7$-$C_{12}$ aralkyl" include benzyl.

$R^8$ represents $C_1$-$C_6$ alkyl, $C_1$-$C_6$ alkyl substituted with one or more halogen, $C_1$-$C_6$ alkyl substituted with one or more $C_1$-$C_6$ alkoxy, $C_1$-$C_6$ alkoxy, or halogen.

Further, $R^6$ and $R^{6a}$ may together form oxo group.

Among them, specific examples of preferred Z are - CH(OH)-, -CO-, and -$CH(OCOR^7)$-, and especially -CH (OH)- is preferable.

In the above formula (I), $Ar^1$ represents aryl having 6 to 10 carbon atoms or monocyclic or bicyclic heteroaryl having 2 to 9 carbon atoms with 1 to 4 heteroatoms selected from the group consisting of oxygen, sulfur, and nitrogen, which may be substituted with same or different 1 to 5 $R^1$. Although specific examples of the "aryl" and "monocyclic or bicyclic heteroaryl" are as defined above, examples of preferred "aryl" or "monocyclic or bicyclic heteroaryl" of $Ar^1$ includes phenyl, pyridyl, pyrimidyl and pyridazinyl and particularly phenyl and pyridyl are preferable.

In the above formula (I), $Ar^1$ may be substituted with 1 to 5 same or different $R^1$. $R^1$ represents a substituent independently selected from the group consisting of $C_1$-$C_6$ alkyl, $C_1$-$C_6$ alkyl substituted with one or more halogen, $C_1$-$C_6$ alkyl substituted with one or more -$OR^7$, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, -$NR^7SO_2R^{7a}$, -$SO_2NR^7R^{7a}$, -$S(O)_{0-2}R^7$ and glucuronide. The definitions of $R^7$ and $R^{7a}$ in $R^1$ are the same as those of $R^7$ and $R^{7a}$ in the above $R^6$ and $R^{6a}$.

In addition, two independent $R^7$ or $R^7$ and $R^{7a}$ in the same $R^1$ may form, with a part of binding groups in $R^1$,

3 to 8-membered ring structure that may contain 3 or less oxygen or nitrogen. Examples of "3 to 8-membered ring structure that may contain 3 or less oxygen or nitrogen" as $R^1$ include 1-piperidinyl, 1-pyrrolidinyl, 4-morpholinyl, 1-piperazinyl, 4-alkylpiperazinyl, 4-acylpiperidinyl, and 2-oxomorpholin-1-yl.

Further, two adjacent independent $R^1$ can be bonded together to form 3 to 8-membered ring structure that may contain 3 or less oxygen or nitrogen. Specific examples thereof include $C_1$-$C_6$ alkylene, $C_1$-$C_4$ alkyleneoxy, and $C_1$-$C_3$ alkylenedioxy.

Among them, specific examples of preferred $R^1$ are $C_1$-$C_6$ alkyl, $C_1$-$C_6$ alkyl substituted with one or more halogen, halogen, -$OR^7$, -$COOR^7$, and glucuronide, and particularly hydroxy is preferable.

Further, suitable example of $Ar^1$ formed by $Ar^1$ and $R^1$ in combination include phenyl substituted with at least one hydroxy and pyridyl substituted with at least one hydroxy, and phenyl of which at least 4-position is substituted with hydroxy is preferable. In this case, phenyl and pyridyl may be further substituted with 1 to 4 same or different $R^1$. In particular, most suitable examples of $Ar^1$ formed by $Ar^1$ and $R^1$ in combination are 4-hydroxyphenyl, 2,4-dihydroxyphenyl, 4-hydroxy-2-methylphenyl, 4-hydroxy-2-methoxyphenyl, and 2-chloro-4-hydroxyphenyl.

In the above formula (I), $R^2$ represents a substituent independently selected from the group consisting of $C_1$-$C_6$ alkyl, $C_1$-$C_6$ alkyl substituted with one or more halogen, halogen, cyano, and -$OR^7$, and m represents 0, 1, 2, 3, or 4 and preferably 0. The definition of $R^7$ in $R^2$ is the same as that of $R^7$ for the above $R^6$.

Among them, specific examples of preferred $R^2$ are $C_1$-$C_6$ alkyl, $C_1$-$C_6$ alkyl substituted with one or more halogen, and halogen, and particularly halogen is preferable.

In the above formula (I), $Ar^3$ represents monocyclic or bicyclic heteroaryl having 2 to 9 carbon atoms with 1 to 4 heteroatoms selected from the group consisting of oxygen, sulfur, and nitrogen, which may be substituted with 1 to 5 same or different $R^3$. Although specific examples of "monocyclic or bicyclic heteroaryl" are as defined above, examples of preferred "monocyclic or bicyclic heteroaryl" for $Ar^3$ include pyridyl, pyrazyl, pyrimidyl, pyridazinyl, furanyl, thienyl, pyrrolidinyl, pyrazolyl, isoxazolyl, isothiazolyl, benzofuranyl, isobenzofuranyl, benzothienyl, indolyl, isoindolyl, benzothiazolyl, benzimidazolyl, or benzoxazolyl, and particularly pyridyl, pyrazyl, and pyrimidyl are preferable. In particular, 3-pyridyl, 2-pyrazyl, and 5-pyrimidyl are further preferable.

In addition, $Ar^3$ is preferably bonded to 4-position of phenylene to which the $Ar^3$ is bonded.

In the above formula (I), $Ar^3$ may be substituted with 1 to 5 same or different $R^3$. $R^3$ represents a substituent independently selected from the group consisting of $C_1$-$C_6$ alkyl, $C_1$-$C_6$ alkyl substituted with one or more halogen, $C_1$-$C_6$ alkyl substituted with one or more -$OR^7$, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, -$NR^7SO_2R^{7a}$, -$SO_2NR^7R^{7a}$, and -$S(O)_{0\text{-}2}R^7$. The definitions of $R^7$ and $R^{7a}$ in $R^3$ are the same as those of $R^7$ and $R^{7a}$ for the above $R^6$ and $R^{6a}$.

In addition, two independent $R^7$ or $R^7$ and $R^{7a}$ in the same $R^3$ may form, with a part of binding substituents in the $R^3$, 3 to 8-membered ring structure that may contain 3 or less oxygen or nitrogen. Examples of "3 to 8-membered ring structure that may contain 3 or less oxygen or nitrogen" as $R^3$ include 1-piperidinyl, 1-pyrrolidinyl, 4-morpholinyl, 1-piperazinyl, 4-alkylpiperazinyl, 4-acylpiperidinyl, and 2-oxomorpholin-1-yl.

Further, two adjacent independent $R^3$ can be bonded together to form 3 to 8-membered ring structure that may contain 3 or less oxygen or nitrogen. Specific examples thereof include $C_1$-$C_6$ alkylene, $C_1$-$C_4$ alkyleneoxy, and $C_1$-$C_3$ alkylenedioxy.

Among them, specific examples of preferred $R^3$ are $C_1$-$C_6$ alkyl, $C_1$-$C_6$ alkyl substituted with one or more halogen, halogen, cyano, -$OR^7$, -$COOR^7$, -$COR^7$, -$CONR^7R^{7a}$, and -$NR^7R^{7a}$, and particularly $C_1$-$C_6$ alkyl, $C_1$-$C_6$ alkyl substituted with one or more halogen, halogen, cyano, -$OR^7$, -$COR^7$, -$CONR^7R^{7a}$, and -$NR^7R^{7a}$ are preferable.

Further, suitable examples of $Ar^3$ formed by $Ar^3$ and $R^3$ in combination include pyridyl, pyrazyl, or pyrimidyl optionally substituted with 1 to 4 same or different substituent independently selected from the group consisting of $C_1$-$C_6$ alkyl, $C_1$-$C_6$ alkyl substituted with one or more halogen, halogen, cyano, -$OR^7$, -$COR^7$, -$CONR^7R^{7a}$, and -$NR^7R^{7a}$. In particular, unsubstituted pyridyl, unsubstituted pyrazyl, unsubstituted pyrimidyl, or pyridyl substituted at 4, 5, and/or 6-positions with the above preferred $R^3$ [$C_1$-$C_6$ alkyl, $C_1$-$C_6$ alkyl substituted with one or more halogen, halogen, cyano, -$OR^7$, -$COR^7$,- $CONR^7R^{7a}$, and -$NR^7R^{7a}$] and bonded at 3-position to phenylene is preferable. In more particular, unsubstituted 2-pyrazyl, unsubstituted 5-pyrimidyl, or pyridyl substituted at 5 and/or 6-positions with the above preferred $R^3$ [$C_1$-$C_6$ alkyl, $C_1$-$C_6$ alkyl substituted with one or more halogen, halogen, cyano, -$OR^7$, -$COR^7$, -$CONR^7R^{7a}$, and -$NR^7R^{7a}$] and bonded at 3-position to phenylene is preferable. In addition, $Ar^3$ is preferably bonded to 4-position of phenylene to which the $Ar^3$ is bonded.

In the above formula (I), $Ar^4$ represents aryl having 6 to 10 carbon atoms or monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^4$. Although specific examples of "aryl" and "monocyclic or bicyclic heteroaryl" are as defined above, examples of preferred "aryl" or "monocyclic or bicyclic heteroaryl" for $Ar^4$ include phenyl, pyridyl, furanyl, thienyl, thiazolyl, naphthyl, benzofuranyl, benzothienyl, and benzothiazolyl, and phenyl is particularly preferable.

In the above formula (I), $Ar^4$ may be substituted with 1 to 5 same or different $R^4$. $R^4$ represents a substituent

independently selected from the group consisting of $C_1$-$C_6$ alkyl, $C_1$-$C_6$ alkyl substituted with one or more halogen atoms, $C_1$-$C_6$ alkyl substituted with one or more -$OR^7$, $C_1$-$C_6$ alkylene, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, and -$NR^7SO_2R^{7a}$, -$SO_2NR^7R^{7a}$, -$S(O)_{0-2}R^7$, aryl having 6 to 10 carbon atoms, which may be substituted with 1 to 5 same or different $R^8$, and monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^8$. The definitions of $R^7$, $R^{7a}$ and $R^8$ for $R^4$ are the same as those of $R^7$, $R^{7a}$ and $R^8$ for the above $R^6$ and $R^{6a}$. In addition, although specific examples of "aryl" and "monocyclic or bicyclic heteroaryl" are as defined above, examples of preferred "aryl" or "monocyclic or bicyclic heteroaryl" for $R^4$ include phenyl, pyridyl, pyrimidyl, pyridazyl, and particularly phenyl and pyridyl are preferable.

In addition, two independent $R^7$s or $R^7$ and $R^{7a}$ in the same $R^4$ may form, with a part of binding groups in the $R^4$, 3 to 8-membered ring structure that may contain 3 or less oxygen or nitrogen. Examples of "3 to 8-membered ring structure that may contain 3 or less oxygen or nitrogen" as $R^4$ include 1-piperidinyl, 1-pyrrolidinyl, 4-morpholinyl, 1-piperazinyl, 4-alkylpiperazinyl, 4-acylpiperidinyl, and 2-oxomorpholin-1-yl.

Further, two adjacent independent $R^4$ can be bonded together to form 3 to 8-membered ring structure that may contain 3 or less oxygen or nitrogen. Specific examples thereof include $C_1$-$C_6$ alkylene, $C_1$-$C_4$ alkyleneoxy, and $C_1$-$C_3$ alkylenedioxy.

Among them, specific examples of preferred $R^4$ are $C_1$-$C_6$ alkyl, $C_1$-$C_6$ alkyl substituted with one or more halogen, halogen, cyano, nitro, -$OR^7$, -$CONR^7R^{7a}$, -$NR^7R^{7a}$, -$NR^7COR^{7a}$, $C_1$-$C_4$ alkyleneoxy, and $C_1$-$C_3$ alkylenedioxy, and particularly $C_1$-$C_6$ alkyl, $C_1$-$C_6$ alkyl substituted with one or more halogen, halogen, cyano, hydroxy, $C_1$-$C_4$ alkyleneoxy, and $C_1$-$C_3$ alkylenedioxy are preferable.

Further, suitable examples of $Ar^4$ formed by $Ar^4$ and $R^4$ in combination include phenyl substituted with 1 to 5 same or different substituent independently selected from the group consisting of $C_1$-$C_6$ alkyl, $C_1$-$C_6$ alkyl substituted with one or more halogen, halogen, cyano, hydroxy, $C_1$-$C_4$ alkyleneoxy, and $C_1$-$C_3$ alkylenedioxy, unsubstituted pyridyl, and unsubstituted benzofuranyl, and particularly phenyl substituted with 1 to 2 halogen is preferable.

In the above formula (I), a combination of X, Y, Z, $Ar^1$ $Ar^3$ $Ar^4$ $R^1$, $R^2$, $R^3$, $R^4$, $R^5$, $R^{5a}$, $R^6$, $R^{6a}$, $R^7$, and $R^{7a}$ is preferably a combination of the preferred substituents described above respectively, and more preferably a combination of the particularly preferred substituents.

Preferred compounds and the pharmaceutically acceptable salt thereof among compounds represented by the above formula (I) (referred to as "preferred compounds" hereinbelow) are listed below.

(3R,4R)-3-[(2R)-2-(4-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxyphenyl)-1-[4-(5-trifluoromethylpyridin-2-yl)phenyl]azetidin-2-one,
(3R,4R)-4-(4-hydroxyphenyl)-3-(2-phenylethylthio)-1-[4-(pyridin-3-yl)phenyl]azetidin-2-one,
(3R,4R)-3-[2-(4-fluorophenyl)ethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxyphenyl)-1-[4-(2-methoxypyridin-3-yl)phenyl]azetidin-2-one,
(3R,4R)-3-[(2R)-2-(4-fluorophenyl)-2-hydroxyethylthio]-1-[4-(2,6-dimethoxypyridin-3-yl)phenyl]-4-(4-hydroxyphenyl)azetidin-2-one,
(3R,4R)-3-[(2R)-2-(4-fluorophenyl)-2-hydroxyethylthio]-4-(4-hydroxyphenyl)-1-[4-(4-methylpyridin-3-yl)phenyl]azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyridine-2-carbonitrile,
(3R,4R)-3-[(2R)-2-(4-fluorophenyl)-2-hydroxyethylthio]-4-(4-hydroxyphenyl)-1-[4-(pyrimidin-5-yl)phenyl]azetidin-2-one,
(3R,4R)-3-[(2R)-2-(4-fluorophenyl)-2-hydroxyethylthio]-4-(4-hydroxyphenyl)-1-[4-(pyridin-4-yl)phenyl]azetidin-2-one,
(3R,4R)-3-[(2R)-2-(4-fluorophenyl)-2-hydroxyethylthio]-1-[4-(3-chloropyridin-4-yl)phenyl]-4-(4-hydroxyphenyl)azetidin-2-one,
(3R,4R)-3-[(2R)-2-(4-fluorophenyl)-2-hydroxyethylthio]-4-(4-hydroxyphenyl)-1-[4-(indol-4-yl)phenyl]azetidin-2-one
(3R,4R)-3-[(2R)-2-(4-fluorophenyl)-2-hydroxyethylthio]-4-(4-hydroxyphenyl)-1-[4-(6-methoxypyridin-3-yl)phenyl]azetidin-2-one,
(3R,4R)-3-[(2R)-2-(4-fluorophenyl)-2-hydroxyethylthio]-1-[4-(2-chloropyridin-3-yl)phenyl]-4-(4-hydroxyphenyl)azetidin-2-one,
(3R,4R)-3-[(2R)-2-(4-fluorophenyl)-2-hydroxyethylthio]-4-(4-hydroxyphenyl)-1-[4-(4-methoxypyridin-3-yl)phenyl]azetidin-2-one,
ethyl 5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carboxylate,
5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbonitrile,
(3R,4R)-4-(4-hydroxyphenyl)-3-[(4-methoxyphenyl)methylthio]-1-[4-(pyridin-3-yl)phenyl]azetidin-2-one
(3R,4R)-3-[(2R)-2-(4-fluorophenyl)-2-hydroxyethylthio]-4-(4-hydroxyphenyl)-1-[4-(6-methylpyridin-3-yl)phenyl]azetidin-2-one,
(3R,4R)-3-[(2R)-2-(4-fluorophenyl)-2-hydroxyethylthio]-1-[4-(2-fluoropyridin-3-yl)phenyl]-4-(4-hydroxyphenyl)azetidin-2-one,
(3R,4R)-3-[(2R)-2-(4-fluorophenyl)-2-hydroxyethylthio]-1-[4-(6-dimethylaminopyridin-3-yl)phenyl]-4-(4-hydroxyphenyl) azetidin-2-one,
(3R,4R)-3-[(2R)-2-(4-fluorophenyl)-2-hydroxyethylthio]-4-(4-hydroxyphenyl)-1-[4-(3-methoxypyridin-4-yl)phenyl]azetidin-2-one,
(3R,4R)-3-[(2R)-2-(4-fluorophenyl)-2-hydroxyethylthio]-4-(4-hydroxy-2-methoxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxy-3-methoxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxy-3-methylphenyl)-1-[4-(pyridin-3-yl)phenyl]azetidin-2-one,
(3R,4R)-3-[2-(4-fluorophenyl)-2-oxoethylthio]-4-(4-hydroxyphenyl)-1-[4-(pyridin-3-yl)phenyl]azetidin-2-one,
(3R,4R)-3-[(2R)-2-(3,4-dichlorophenyl)-2-hydroxyethylthio]-4-(4-hydroxyphenyl)-1-[4-(pyridin-3-yl)phenyl]azetidin-2-one,
(3R,4R)-3-[(2R)-2-(2H,3H-benzo[3,4-e]1,4-dioxin-6-yl)-2-hydroxyethylthio]-4-(4-hydroxyphenyl)-1-[4-(pyridin-3-yl)phenyl]azetidin-2-one,
(3R,4R)-3-[(2R)-2-(2,4-difluorophenyl)-2-hydroxyethylthio]-4-(4-hydroxyphenyl)-1-[4-(pyridin-3-yl)phenyl]azetidin-2-one,
(3R,4R)-3-((2R)-2-benzo[b]furan-5-yl-2-hydroxyethylthio)-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyridin-3-yl)phenyl]azetidin-2-one,
(3R,4R)-3-[(2R)-2-(2H-benzo[3,4-d]1,3-dioxolen-5-yl)-2-hydroxyethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-1-[4-(3,5-difluoropyridin-2-yl)phenyl]-4-(4-hydroxyphenyl)azetidin-2-one,
6-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbaldehyde,
(3R,4R)-3-[(2R)-2-(4-fluorophenyl)-2-hydroxyethylthio]-1-[3-fluoro-4-(pyridin-3-yl)phenyl]-4-(4-hydroxyphenyl)azetidin-2-one, (3R,4R)-3-[(2R)-2-(4-fluorophenyl)-2-hydroxyethylthio]-4-(3,4-dihydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(pyridin-3-yl)phenyl]azetidin-2-one,
(3R,4R)-3-[(2R)-2-(4-fluorophenyl)-2-hydroxyethylthio]-4-(3-chloro-4-hydroxyphenyl)-1-[4-(pyridin-3-yl)phenyl]azetidin-2-one,
3-[(2R)-2-(4-fluorophenyl)-2-hydroxyethylthio]-4-(6-hydroxypyridin-3-yl)-1-[4-(pyridin-3-yl)phenyl]azetidin-2-one,
methyl 5-{(2R,3R)-3-[(2R)-2-(4-fluorophenyl)-2-hydroxyethylthio]-4-oxo-1-[4-(pyridin-3-yl)phenyl]azetidin-2-yl}-2-hydroxybenzoate,
(3R,4R)-3-[(2R)-2-(4-fluorophenyl)-2-hydroxyethylthio]-4-(4-hydroxy-3-nitrophenyl)-1-[4-(pyridin-3-yl)phenyl]azetidin-2-one,
(3R,4R)-3-{(2R)-2-hydroxy-2-[4-(trifluoromethyl)phenyl]ethylthio}-4-(4-hydroxyphenyl)-1-[4-(pyridin-3-yl)phenyl]azetidin-2-one,
4-{(1R)-2-[(3R,4R)-4-(4-hydroxyphenyl)-2-oxo-1-[4-(pyridin-3-yl)phenyl]azetidin-3-ylthio]-1-hydroxyethyl}benzenecarbonitrile,
(3R,4R)-3-[(2R)-2-hydroxy-2-(4-methylphenyl)ethylthio]-4-(4-hydroxyphenyl)-1-[4-(pyridin-3-yl)phenyl]azetidin-2-one,
(3R,4R)-3-[(2S)-2-(5-chlorothiophen-2-yl)-2-hydroxyethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxyphenyl)-1-[3-methyl-4-(pyridin-3-yl)phenyl]azetidin-2-one,
(3R,4R)-3-[(2R)-2-(4-fluorophenyl)-2-hydroxyethylthio]-1-[4-(5-acetylpyridin-3-yl)phenyl]-4-(4-hydroxyphenyl)azetidin-2-one,
N-ethyl-5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carboxamide,
(3R,4R)-3-[(2R)-2-(4-fluorophenyl)-2-hydroxyethylthio]-4-(4-hydroxyphenyl)-1-[4-(5-methoxypyridin-3-yl)phenyl]azetidin-2-one,
(3R,4R)-3-[(2R)-2-(4-fluorophenyl)-2-hydroxyethylthio]-4-(4-hydroxyphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(4-hydroxyphenyl)-1-[3-methoxy-4-(pyridin-3-yl)phenyl]azetidin-2-one,
(3R,4R)-3-[(2R)-2-(4-fluorophenyl)-2-hydroxyethylthio]-4-(4-hydroxyphenyl)-1-[4-(pyrazin-2-yl)phenyl]azetidin-2-one,
(3R,4R)-3-[(2R)-2-(4-fluorophenyl)-2-hydroxyethylthio]-4-(4-hydroxyphenyl)-1-[4-(2-methylpyridin-3-yl)phenyl]azetidin-2-one,
(3R,4R)-3-[(2R)-2-(4-fluorophenyl)-2-hydroxyethylthio]-1-[4-(5-fluoropyridin-3-yl)phenyl]-4-(4-hydroxyphenyl)azetidin-2-one,
(3R,4R)-3-[(2R)-2-(4-fluorophenyl)-2-hydroxyethylthio]-1-[4-(5-chloropyridin-3-yl)phenyl]-4-(4-hydroxyphenyl)azetidin-2-one,
(3R,4R)-3-[(2R)-2-(4-chlorophenyl)-2-hydroxyethylthio]-4-(4-hydroxyphenyl)-1-[4-(2-methoxypyridin-3-yl)phenyl]azetidin-2-one,
5-(4-{(3R,4R)-3-[(2R)-2-(4-chlorophenyl)-2-hydroxyethylthio]-4-(4-hydroxyphenyl)-2-oxoazetidinyl}phenyl)pyridine-3-carbonitrile,
(3R,4R)-3-[(2R)-2-(4-fluorophenyl)-2-hydroxyethylthio]-4-(2-chloro-4-hydroxyphenyl)-1-[4-(pyridin-3-yl)phenyl]azetidin-2-one,
5-(4-{(3R,4R)-3-[(2R)-2-(4-chlorophenyl)-2-hydroxyethylthio]-4-(4-hydroxyphenyl)-2-oxoazetidinyl}phenyl)pyridine-2-carbonitrile,
(3R,4R)-3-[(2R)-2-(4-chlorophenyl)-2-hydroxyethylthio]-4-(4-hydroxyphenyl)-1-[4-(4-methoxypyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(pyridin-3-yl)phenyl]azetidin-2-one,
5-(4-{(3R,4R)-3-[(2R)-2-(4-fluorophenyl)-2-hydroxyethylthio]-4-(2,4-dihydroxyphenyl)-2-oxoazetidinyl}phenyl)pyridine-3-carbonitrile,
(3R,4R)-3-[(2R)-2-(4-fluorophenyl)-2-hydroxyethylthio]-4-(2,4-dihydroxyphenyl)-1-[4-(4-methoxypyridin-3-yl)phenyl]azetidin-2-one,
5-(4-{(3R,4R)-3-[(2R)-2-(4-chlorophenyl)-2-hydroxyethylthio]-4-(2,4-dihydroxyphenyl)-2-oxoazetidinyl}phenyl)pyridine-3-carbonitrile,
3-(4-{(3R,4R)-3-[(2R)-2-(4-fluorophenyl)-2-hydroxyethylthio]-4-(4-hydroxyphenyl)-2-oxoazetidinyl}phenyl)pyridine-4-carbonitrile,
4-(4-{(3R,4R)-3-[(2R)-2-(4-fluorophenyl)-2-hydroxyethylthio]-4-(4-hydroxyphenyl)-2-oxoazetidinyl}phenyl)thiophene-2-carbaldehyde,
(3R,4R)-3-[(2R)-2-(4-fluorophenyl)-2-hydroxyethylthio]-4-(4-hydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyrimidin-5-yl)phenyl]azetidin-2-one,
(3R,4R)-3-[(2R)-2-(4-chlorophenyl)-2-hydroxyethylthio]-1-[4-(5-fluoropyridin-3-yl)phenyl]-4-(4-hydroxyphenyl)azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbaldehyde,
(3R,4R)-3-[(2R)-2-(4-chlorophenyl)-2-hydroxyethylthio]-4-(2,4-dihydroxyphenyl)-1-[4-(5-fluoropyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-fluoropyridin-3-yl)phenyl]azetidin-2-one,
(3R,4R)-3-[(2R)-2-(4-fluorophenyl)-2-hydroxyethylthio]-1-[4-(5-fluoropyridin-3-yl)phenyl]-4-(4-hydroxy-2-methylphenyl)azetidin-2-one,
(3R,4R)-3-[(2R)-2-(4-chlorophenyl)-2-hydroxyethylthio]-4-(2,4-dihydroxyphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-trifluoromethylpyridin-3-yl)phenyl]azetidin-2-one,
(3R,4R)-3-[(2R)-2-(2-fluorophenyl)-2-hydroxyethylthio]-4-(4-hydroxyphenyl)-1-[4-(pyridin-3-yl)phenyl]azetidin-2-one,
(3R,4R)-3-[(2R)-2-(3-fluorophenyl)-2-hydroxyethylthio]-4-(4-hydroxyphenyl)-1-[4-(pyridin-3-yl)phenyl]azetidin-2-one,
(3R,4R)-3-[(2R)-2-(3-chlorophenyl)-2-hydroxyethylthio]-4-(4-hydroxyphenyl)-1-[4-(pyridin-3-yl)phenyl]azetidin-2-one,
5-(4-{(3R,4R)-3-[(2R)-2-(4-fluorophenyl)-2-hydroxyethylthio]-4-(4-hydroxy-2-methylphenyl)-2-oxoazetidinyl}phenyl)pyridine-3-carbonitrile,
(3R,4R)-3-[(2R)-2-(4-chloro-3-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-chloro-3-fluorophenyl)-2-hydroxyethylthio]-1-[4-(5-fluoropyridin-3-yl)phenyl]-4-(4-hydroxyphenyl)azetidin-2-one,
(3R,4R)-3-((2R)-2-benzo[b]thiophen-5-yl-2-hydroxyethylthio)-4-(4-hydroxyphenyl)-1-[4-(pyridin-3-yl)phenyl]azetidin-2-one,
(3R,4R)-3-[(2R)-2-hydroxy-2-(4-methoxyphenyl)ethylthio]-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-trifluoromethylpyridin-3-yl)phenyl]azetidin-2-one,
(3R,4R)-3-[(2R)-2-(4-chlorophenyl)-2-hydroxyethylthio]-1-[4-(5-chloropyridin-3-yl)phenyl]-4-(4-hydroxyphenyl)azetidin-2-one,
(3R,4R)-3-[(2R)-2-(4-chlorophenyl)-2-hydroxyethylthio]-1-[4-(6-dimethylaminopyridin-3-yl)phenyl]-4-(4-hydroxyphenyl)azetidin-2-one,
(3R,4R)-3-[(2R)-2-(4-chloro-2-hydroxyphenyl)-2-hydroxyethylthio]-4-(4-hydroxyphenyl)-1-[4-(pyridin-3-yl)phenyl]azetidin-2-one,
(3R,4R)-3-[(2R)-2-(2-chloro-4-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(6-methylpyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-chloropyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-methylpyridin-3-yl)phenyl]azetidin-2-one,
(3R,4R)-3-[(2R)-2-(4-chlorophenyl)-2-hydroxyethylthio]-1-[4-(6-chloropyridin-3-yl)phenyl]-4-(4-hydroxyphenyl)azetidin-2-one,
(3R,4R)-3-[(2R)-2-(4-fluorophenyl)-2-hydroxyethylthio]-4-(2,4-dihydroxyphenyl)-1-[4-(6-methylpyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(5-chloropyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(6-methylpyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(pyrimidin-5-yl)phenyl]azetidin-2-one,
(3R,4R)-3-[(2R)-2-(4-chloro-2-methoxyphenyl)-2-hydroxyethylthio]-4-(4-hydroxyphenyl)-1-[4-(pyridin-3-yl)phenyl]azetidin-2-one,
(3R,4R)-3-[(2R)-2-(4-chloro-2-methylphenyl)-2-hydroxyethylthio]-4-(4-hydroxyphenyl)-1-[4-(pyridin-3-yl)phenyl]azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyrimidine-2-carbonitrile,
(3R,4R)-3-[(2R)-2-(4-chlorophenyl)-2-hydroxyethylthio]-4-(4-hydroxyphenyl)-1-[4-(pyrazin-2-yl)phenyl]azetidin-2-one,
(3R,4R)-3-[(2R)-2-(4-chlorophenyl)-2-hydroxyethylthio]-1-[4-(5-acetylpyridin-3-yl)phenyl]-4-(4-hydroxyphenyl)azetidin-2-one,
N-ethyl-5-(4-{(3R,4R)-3-[(2R)-2-(4-chlorophenyl)-2-hydroxyethylthio]-4-(4-hydroxyphenyl)-2-oxoazetidinyl}phenyl)pyridine-3-carboxamide,
4-{(2R,3R)-3-[(2R)-2-acetyloxy-2-(4-fluorophenyl)ethylthio]-4-oxo-1-[4-(pyridin-3-yl)phenyl]azetidin-2-yl}phenyl acetate,
(1R)-2-[(3R,4R)-4-(4-hydroxyphenyl)-2-oxo-1-[4-(pyridin-3-yl)phenyl]azetidin-3-ylthio]-1-(4-fluorophenyl)ethyl acetate,
(2S,3R,4S,5R,6S)-6-(4-{(2R,3R)-3-[(2R)-2-acetyloxy-2-(4-fluorophenyl)ethylthio]-4-oxo-1-[4-(pyridin-3-yl)phenyl]azetidin-2-yl}phenoxy)-4,5-diacetyloxy-2-(methoxycarbonyl)-2H-3,4,5,6-tetrahydropyran-3-yl acetate,
(2S,3S,4S,5R,6S)-6-(4-{(2R,3R)-3-[(2R)-2-(4-fluorophenyl)-2-hydroxyethylthio]-4-oxo-1-[4-(pyridin-3-yl)phenyl]azetidin-2-yl}phenoxy)-3,4,5-trihydroxy-2H-3,4,5,6-tetrahydropyran-2-carboxylic acid,
(3R,4R)-3-{(2R)-2-[4-(4-fluorophenyl)phenyl]-2-hydroxyethylthio}-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxy-2-prop-2-enyloxyphenyl)-1-[4-(pyridin-3-yl)phenyl]azetidin-2-one,
(3R,4R)-3-[(2R)-2-(4-chlorophenyl)-2-hydroxyethylthio]-1-[4-(6-hydroxypyridin-3-yl)phenyl]-4-(4-hydroxyphenyl)azetidin-2-one,
(3R,4R)-3-[(2R)-2-amino-2-(4-chlorophenyl)ethylthio]-4-(4-hydroxyphenyl)-1-[4-(pyridin-3-yl)phenyl]azetidin-2-one,
N-{(1R)-2-[(3R,4R)-4-(4-hydroxyphenyl)-2-oxo-1-[4-(pyridin-3-yl)phenyl]azetidin-3-ylthio]-1-(4-chlorophenyl)ethyl} acetamide,
(3R,4R)-3-[(2R)-2-(4-chlorophenyl)-2-hydroxyethoxy]-4-(4-hydroxyphenyl)-1-[4-(pyridin-3-yl)phenyl]azetidin-2-one,
(3R,4R)-3-[(2R)-2-(4-fluorophenyl)-2-hydroxyethoxy]-4-(4-hydroxyphenyl)-1-[4-(pyridin-3-yl)phenyl]azetidin-2-one,
(3R,4R)-3-[(2R)-2-(4-fluorophenyl)-2-(phenylmethoxy)ethylthio]-4-(4-hydroxyphenyl)-1-[4-(pyridin-3-yl)phenyl]azetidin-2-one,
(1R)-2-[(3R,4R)-4-(4-hydroxyphenyl)-2-oxo-1-[4-(pyridin-3-yl)phenyl]azetidin-3-ylthio]-1-(4-chlorophenyl)ethyl benzoate,
(3R,4R)-3-{(2R)-2-hydroxy-2-[4-(methylsulfonyl)phenyl]ethylthio}-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-methylsulfonylpyridin-3-yl)phenyl] azetidin-2-one,
(3R,4R)-3-((2R)-2-azulen-2-yl-2-hydroxyethylthio)-4-(4-hydroxyphenyl)-1-[4-(pyridin-3-yl)phenyl]azetidin-2-one,
or a pharmaceutically acceptable salt thereof.

More preferred compounds and the pharmaceutically acceptable salt thereof among preferred compounds are listed below.

(3R,4R)-3-[(2R)-2-(4-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxyphenyl)-1-[4-(2-methoxypyridin-3-yl)phenyl]azetidin-2-one,
(3R,4R)-3-[(2R)-2-(4-fluorophenyl)-2-hydroxyethylthio]-4-(4-hydroxyphenyl)-1-[4-(pyrimidin-5-yl)phenyl]azetidin-2-one,
(3R,4R)-3-[(2R)-2-(4-fluorophenyl)-2-hydroxyethylthio]-4-(4-hydroxyphenyl)-1-[4-(4-methoxypyridin-3-yl)phenyl]azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbonitrile,
(3R,4R)-3-[(2R)-2-(4-fluorophenyl)-2-hydroxyethylthio]-4-(4-hydroxyphenyl)-1-[4-(6-methylpyridin-3-yl)phenyl]azetidin-2-one,
(3R,4R)-3-[(2R)-2-(4-fluorophenyl)-2-hydroxyethylthio]-1-[4-(6-dimethylaminopyridin-3-yl)phenyl]-4-(4-hydroxyphenyl) azetidin-2-one,
(3R,4R)-3-[(2R)-2-(4-fluorophenyl)-2-hydroxyethylthio]-4-(4-hydroxy-2-methoxyphenyl)-1-[4-(pyridin-3-yl)phenyl]azetidin-2-one,
(3R,4R)-3-[2-(4-fluorophenyl)-2-oxoethylthio]-4-(4-hydroxyphenyl)-1-[4-(pyridin-3-yl)phenyl]azetidin-2-one,
(3R,4R)-3-[(2R)-2-(3,4-dichlorophenyl)-2-hydroxyethylthio]-4-(4-hydroxyphenyl)-1-[4-(pyridin-3-yl)phenyl]azetidin-2-one,
(3R,4R)-3-[(2R)-2-(2,4-difluorophenyl)-2-hydroxyethylthio]-4-(4-hydroxyphenyl)-1-[4-(pyridin-3-yl)phenyl]azetidin-2-one,
(3R,4R)-3-((2R)-2-benzo[b]furan-5-yl-2-hydroxyethylthio)-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyridin-3-yl)phenyl]azetidin-2-one,
(3R,4R)-3-[(2R)-2-(2H-benzo[3,4-d]1,3-dioxolen-5-yl)-2-hydroxyethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(pyridin-3-yl)phenyl]azetidin-2-one,
(3R,4R)-3-[(2R)-2-hydroxy-2-(4-methylphenyl)ethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-1-[4-(5-acetylpyridin-3-yl)phenyl]-4-(4-hydroxyphenyl)azetidin-2-one,
N-ethyl-5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carboxamide,
(3R,4R)-3-[(2R)-2-(4-fluorophenyl)-2-hydroxyethylthio]-4-(4-hydroxyphenyl)-1-[4-(5-methoxypyridin-3-yl)phenyl]azetidin-2-one,
(3R,4R)-3-[(2R)-2-(4-fluorophenyl)-2-hydroxyethylthio]-4-(4-hydroxyphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(4-hydroxyphenyl)-1-[4-(pyrazin-2-yl)phenyl]azetidin-2-one,
(3R,4R)-3-[(2R)-2-(4-fluorophenyl)-2-hydroxyethylthio]-1-[4-(5-fluoropyridin-3-yl)phenyl]-4-(4-hydroxyphenyl)azetidin-2-one,
(3R,4R)-3-[(2R)-2-(4-fluorophenyl)-2-hydroxyethylthio]-1-[4-(5-chloropyridin-3-yl)phenyl]-4-(4-hydroxyphenyl)azetidin-2-one,
5-(4-{(3R,4R)-3-[(2R)-2-(4-chlorophenyl)-2-hydroxyethylthio]-4-(4-hydroxyphenyl)-2-oxoazetidinyl}phenyl)pyridine-3-carbonitrile,
(3R,4R)-3-[(2R)-2-(4-fluorophenyl)-2-hydroxyethylthio]-4-(2-chloro-4-hydroxyphenyl)-1-[4-(pyridin-3-yl)phenyl]azetidin-2-one,
5-(4-{(3R,4R)-3-[(2R)-2-(4-chlorophenyl)-2-hydroxyethylthio]-4-(4-hydroxyphenyl)-2-oxoazetidinyl}phenyl)pyridine-2-carbonitrile,
(3R,4R)-3-[(2R)-2-(4-chlorophenyl)-2-hydroxyethylthio]-4-(2,4-dihydroxyphenyl)-1-[4-(pyridin-3-yl)phenyl]azetidin-2-one,
5-(4-{(3R,4R)-3-[(2R)-2-(4-fluorophenyl)-2-hydroxyethylthio]-4-(2,4-dihydroxyphenyl)-2-oxoazetidinyl}phenyl)pyridine-3-carbonitrile,
(3R,4R)-3-[(2R)-2-(4-fluorophenyl)-2-hydroxyethylthio]-4-(2,4-dihydroxyphenyl)-1-[4-(4-methoxypyridin-3-yl)phenyl]azetidin-2-one,
5-(4-{(3R,4R)-3-[(2R)-2-(4-chlorophenyl)-2-hydroxyethylthio]-4-(2,4-dihydroxyphenyl)-2-oxoazetidinyl}phenyl)pyridine-3-carbonitrile,
(3R,4R)-3-[(2R)-2-(4-fluorophenyl)-2-hydroxyethylthio]-4-(4-hydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyrimidin-5-yl)phenyl]azetidin-2-one,
(3R,4R)-3-[(2R)-2-(4-chlorophenyl)-2-hydroxyethylthio]-1-[4-(5-fluoropyridin-3-yl)phenyl]-4-(4-hydroxyphenyl)azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbaldehyde,
(3R,4R)-3-[(2R)-2-(4-chlorophenyl)-2-hydroxyethylthio]-4-(2,4-dihydroxyphenyl)-1-[4-(5-fluoropyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-fluoropyridin-3-yl)phenyl]azetidin-2-one,
(3R,4R)-3-[(2R)-2-(4-fluorophenyl)-2-hydroxyethylthio]-1-[4-(5-fluoropyridin-3-yl)phenyl]-4-(4-hydroxy-2-methylphenyl)azetidin-2-one,
(3R,4R)-3-[(2R)-2-(4-chlorophenyl)-2-hydroxyethylthio]-4-(2,4-dihydroxyphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-trifluoromethylpyridin-3-yl)phenyl]azetidin-2-one,
5-(4-{(3R,4R)-3-[(2R)-2-(4-fluorophenyl)-2-hydroxyethylthio]-4-(4-hydroxy-2-methylphenyl)-2-oxoazetidinyl}phenyl)pyridine-3-carbonitrile,
(3R,4R)-3-[(2R)-2-(4-chloro-3-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-chloro-3-fluorophenyl)-2-hydroxyethylthio]-1-[4-(5-fluoropyridin-3-yl)phenyl]-4-(4-hydroxyphenyl)azetidin-2-one,
(3R,4R)-3-((2R)-2-benzo[b]thiophen-5-yl-2-hydroxyethylthio)-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-trifluoromethylpyridin-3-yl)phenyl]azetidin-2-one,
(3R,4R)-3-[(2R)-2-(4-chlorophenyl)-2-hydroxyethylthio]-1-[4-(5-chloropyridin-3-yl)phenyl]-4-(4-hydroxyphenyl)azetidin-2-one,
(3R,4R)-3-[(2R)-2-(4-chlorophenyl)-2-hydroxyethylthio]-1-[4-(6-dimethylaminopyridin-3-yl)phenyl]-4-(4-hydroxyphenyl)azetidin-2-one,

(3R,4R)-3-[(2R)-2-(4-chloro-2-hydroxyphenyl)-2-hydroxyethylthio]-4-(4-hydroxyphenyl)-1-[4-(pyridin-3-yl)phenyl]azetidin-2-one,
(3R,4R)-3-[(2R)-2-(2-chloro-4-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(6-methylpyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-chloropyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-methylpyridin-3-yl)phenyl]azetidin-2-one,
(3R,4R)-3-[(2R)-2-(4-chlorophenyl)-2-hydroxyethylthio]-1-[4-(6-chloropyridin-3-yl)phenyl]-4-(4-hydroxyphenyl)azetidin-2-one,
(3R,4R)-3-[(2R)-2-(4-fluorophenyl)-2-hydroxyethylthio]-4-(2,4-dihydroxyphenyl)-1-[4-(6-methylpyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(5-chloropyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(6-methylpyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(pyrimidin-5-yl)phenyl]azetidin-2-one,
(3R,4R)-3-[(2R)-2-(4-chloro-2-methoxyphenyl)-2-hydroxyethylthio]-4-(4-hydroxyphenyl)-1-[4-(pyridin-3-yl)phenyl]azetidin-2-one,
(3R,4R)-3-[(2R)-2-(4-chloro-2-methylphenyl)-2-hydroxyethylthio]-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyrazin-2-yl)phenyl]azetidin-2-one,
(3R,4R)-3-[(2R)-2-(4-chlorophenyl)-2-hydroxyethylthio]-1-[4-(5-acetylpyridin-3-yl)phenyl]-4-(4-hydroxyphenyl)azetidin-2-one, or a pharmaceutically acceptable salt thereof.

<General Method of Synthesis>

A compound of formula (I) and an intermediate can be synthesized by any a synthesis method as described below. For the above defined substituents $R^1$, $R^2$, $R^3$, $R^4$, $R^5$, $R^{5a}$, $R^6$, $R^{6a}$, $R^7$, $R^{7a}$ and $R^8$ in the compound of formula (I), when a compound contains reactive substituents such as hydroxy and amino under a use condition, these reactive substituents are protected by an appropriate protective group well known by the person skilled in the art. The protective group can be removed sequentially or simultaneously by the method well known by the person skilled in the art according to the property of the protective group.

(1) Synthesis Method 1

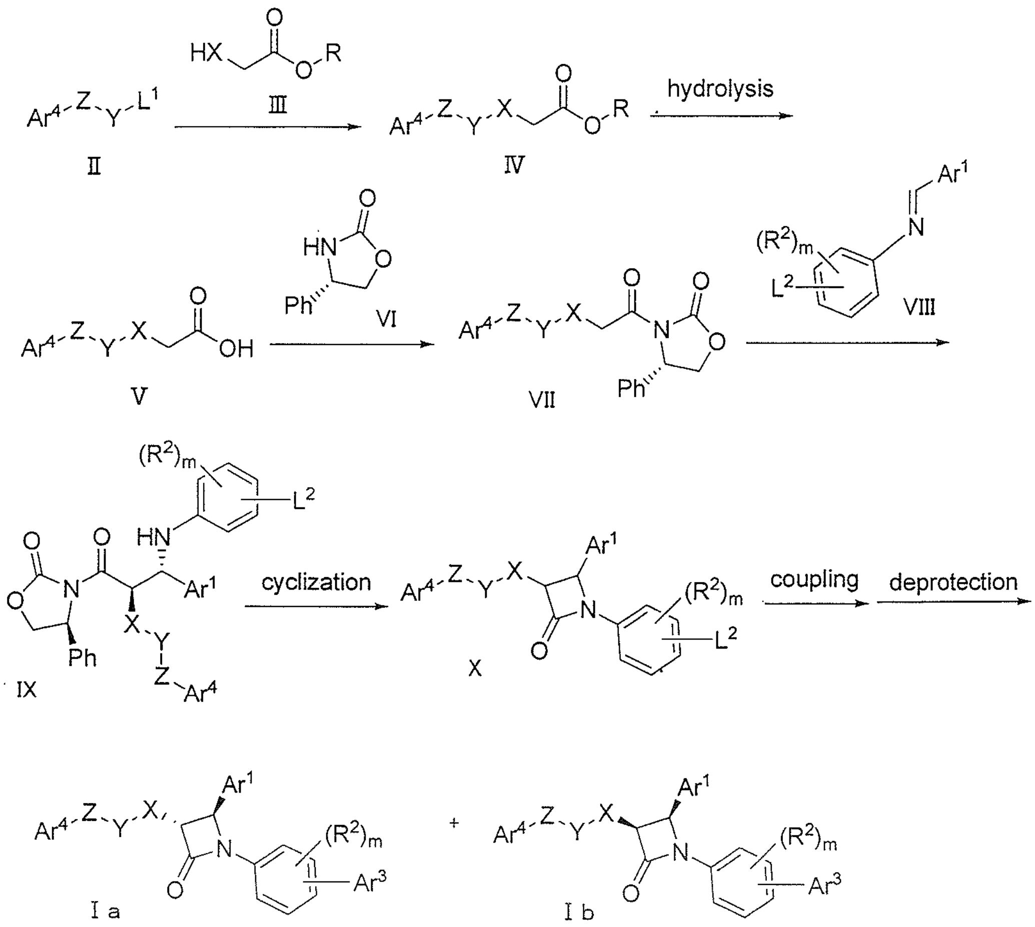


[In the formula, X, Y, Z, $Ar^1$, $Ar^3$, $Ar^4$, $R^2$, and m are the same as those defined for formula (I), respectively. $L^1$ and $L^2$ each represent halogen such as bromine and iodine, methanesulfonyloxy, p-toluenesulfonyloxy, or trifluoromethanesulfonyloxy. R represents $C_1$-$C_6$ alkyl and Ph represents phenyl.] A compound of formula (II) is reacted with a

compound of formula (III) under the presence of a base such as triethylamine and sodium hydride to synthesize a compound of formula (IV). The compound of formula (IV) thus obtained is ester-hydrolyzed with base such as sodium hydroxide to synthesize a compound of formula (V). A carboxyl group in formula (V) is converted into an active ester using, for example, pivaloyl chloride, followed by reaction with an optically active compound of formula (VI) to synthesize a compound of formula (VII). The compound of formula (VII) thus obtained is reacted with an imine of formula (VIII) under the presence of a base such as N-ethyldiisopropylamine and a Lewis acid such as titanium tetrachloride to obtain a compound of formula (IX). However, when hydroxy is present as a substituent in imine compound of formula (VIII), the substituent should be protected by a silyl protective group such as tert-butyldimethyl silyl in advance. The compound of formula (IX) thus obtained is cyclized by reaction with silylation reagent such as N,O-bis(trimethylsilyl)acetamide and fluoride catalyst such as tetrabutylammonium fluoride (TBAF) to obtain a compound of formula (X). The cyclization product, compound of formula (X), is reacted with, for example, boronic acid derivative ($Ar^3$-B($OR^a$)$_2$; wherein $R^a$ represents hydrogen or lower alkyl or pinacolyl formed from two $R^a$ together) under the presence of transition metal catalyst such as tetrakis(triphenylphosphine)palladium. When a reactive substituent such as hydroxy or amino is protected by an appropriate protective group well known by the person skilled in the art, the protective group is removed by the method well known by the person skilled in the art according to the property of the protective group to obtain a compound of formula (I). The compound can be resolved into a compound of formula (Ia) (trans-isomer) and a compound of formula (Ib) (cis-isomer) by a conventional purification technique such as silica gel column chromatography.

In addition, a compound of formula (I) wherein X is sulfoxide or sulfonyl can be synthesized by reacting a compound of formula (X), formula (Ia) or formula (Ib) wherein X is sulfur with oxidizing agent such as m-chloroperbenzoic acid and a mixture of potassium hydrogen persulfate, potassium hydrogen sulfate, and potassium sulfate (trade name "OXONE"). In this case, when a compound of formula (X), formula (Ia) or formula (Ib) has a reactive substituent such as hydroxy or amino, the reactive group is preferably protected with a protective group that can withstand a conversion

reaction.

(2) Synthesis Method 2

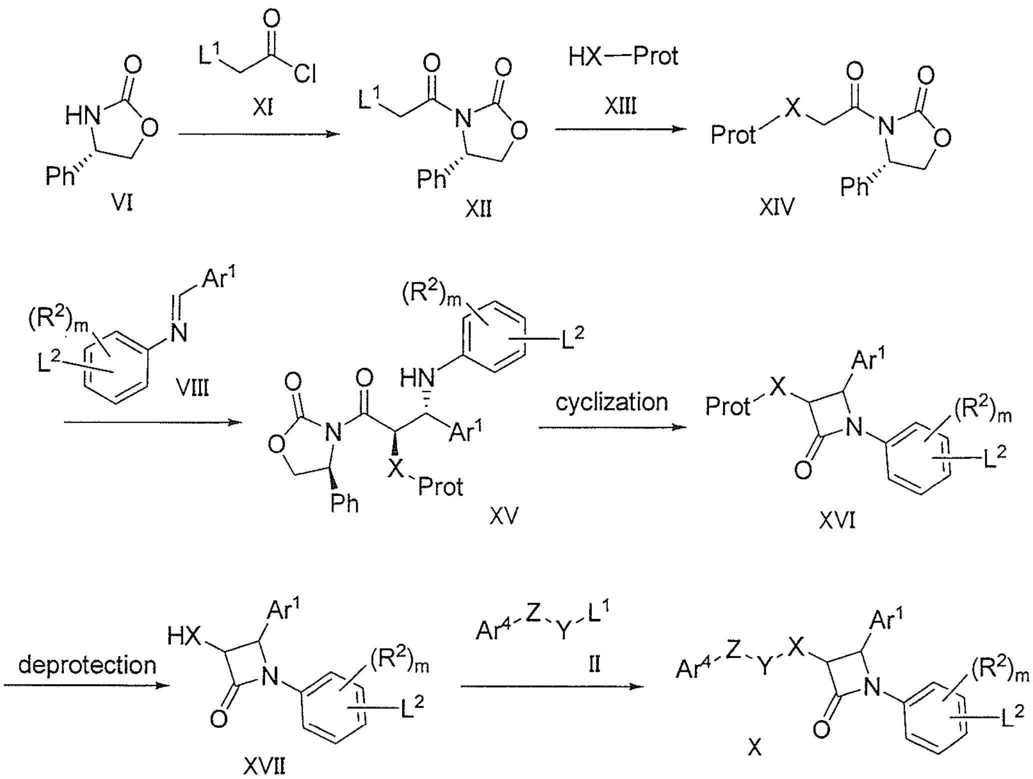


[In the formula, X, Y, Z, $Ar^1$, $Ar^4$, $R^2$, m, $L^1$, $L^2$, and Ph are the same as that defined above, respectively. Prot represents an appropriate protective group for hydroxy or thiol, such as p-methoxybenzyl]

A compound of Formula (X), an intermediate, is also synthesized as described below. A compound of Formula (VI) is reacted with acid chloride of Formula (XI) (where, $L^1$ is the same as that defined above) under the presence of base such as sodium hydride to synthesize a compound of Formula (XII). Further, the compound of Formula (XII) is reacted with a compound of Formula (XIII) under the presence of base such as triethylamine to synthesize a compound of Formula (XIV). The obtained compound of Formula (XIV) is reacted with imine of Formula (VIII) under the presence of base such as N-ethyldiisopropylamine and Lewis acid such as titanium tetrachloride to obtain a compound of Formula (XV). When hydroxy is present as a substituent in the imine compound of Formula (VIII), the hydroxy is protected in advance by silyl-based protective group such as tert-butyldimethylsilyl. The obtained compound of Formula (XV) is cyclized by a reaction with silylation agent such as N,O-bis(trimethylsilyl)acetamide and fluoride catalyst such as tetrabutylammonium fluoride (TBAF) to obtain a compound of Formula (XVI). The Prot group of Formula (XVI), a cyclization product, is removed by methods known by the person skilled in the art depending on the properties of the protective group to synthesize a compound of Formula (XVII). The obtained compound of Formula (XVII) is reacted with a compound of Formula (II) under the presence of base such as triethylamine, and thus the compound of Formula (X) is obtained.

(3) Synthesis Method 3

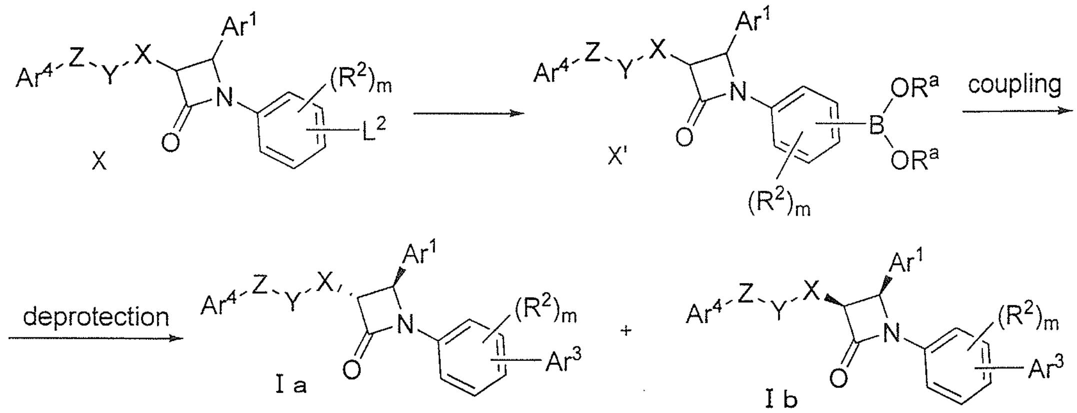


X X' I a I b

[In the formula, X, Y, Z, $Ar^1$, $Ar^3$, $Ar^4$, $R^2$, m, and $L^2$ are the same as that defined above, respectively. $R^a$ represents hydrogen or lower alkyl or pinacolyl formed by two $R^a$ bonding together.]

The compound of Formula (X) synthesized by a method such as Synthesis Method 1 or 2 described above is converted to a compound of Formula (X') by a reaction with boron reagent such as bis(pinacolato)diboron under the presence of transition metal catalyst such as tetrakis(triphenylphosphine)palladium. The compound of Formula (X') is then reacted with $Ar^3$-$L^1$ under the presence of transition metal catalyst such as [1,1'-bis(diphenylphosphino)ferrocene] dichloropalladium (II), and when a reactive substituent such as hydroxy or amino is protected with an appropriate protective group known by the person skilled in the art, the protective group is removed by methods known by the person skilled in the art depending on the properties of the protective group, to synthesize a compound of Formula (I). Further, the compound of Formula (I) is resolved into compounds of Formula (Ia) (trans isomer) and Formula (Ib) (cis isomer) by conventionally known purification techniques such as silica gel column chromatography.

The present invention also relates to a pharmaceutically acceptable salt of a 1-biarylazetidinone derivative represented by formula (I). Examples of such a salt are, for example, inorganic acid salts such as hydrochloride, hydrobromide, sulfate, nitrate, phosphate and carbonate; organic acid salts such as maleate, fumarate, citrate, malate, tartrate, lactate, succinate, benzoate, oxalate, methanesulfonate, benzenesulfonate, p-toluenesulfonate, acetate, trifluoroacetate, and formate; amino acid salts such as glycine salt, lysine salt, arginine salt, orthinine salt, glutamic acid salt, and aspartic acid salt; alkaline metal salts such as sodium salt, potassium salt, and lithium salt; alkaline earth metal salts such as calcium salt and magnesium salt; metal salts such as aluminum salt and iron salt; inorganic salts such as ammonium salt; and amine salts such as organic salts such as t-octylamine salt, dibenzylamine salt, morpholine salt, glucosamine salt, phenylglycyl alkyl ester salt, ethylene diamine salt, N-methylglucamine salt, guanidine salt, diethylamine salt, triethylamine salt, dicyclohexylamine salt, N,N'-dibenzylethylenediamine salt, chloroprocaine salt, procaine salt, diethanolamine salt, N-benzylphenyl amine salt, piperazine salt, tetramethylammonium salt, tris(hydroxymethyl) aminomethane salt.

The above described various pharmaceutically acceptable salts of a 1-biarylazetidinone derivative represented by formula (I) can be appropriately produced based on ordinary knowledge in the art.

In the present invention, stereoisomers, racemates, and all optically active substance of the compound represented by formula (I) can be used.

The 1-biarylazetidinone derivative represented by formula (I) and the pharmaceutically acceptable salt thereof according to the present invention have a cholesterol-lowering effect and/or TG-lowering effect. In the present specification, "cholesterol-lowering effect" refers to an effect of lowering a concentration of cholesterol in the blood (particularly a serum) and/or a concentration of cholesterol in the liver. As used in the present description, "cholesterol lowering effect" is a comprehensive concept including a total cholesterol (TC) lowering effect and an LDL-cholesterol lowering effect.

The 1-biarylazetidinone derivative represented by formula (I) and the pharmaceutically acceptable salt thereof according to the present invention can be used as a drug for the treatment, prevention of onset, and prevention of progress of lipid metabolism disorder, hyperlipidemia, or atherosclerosis, for which they are applicable clinically as a cholesterol-lowering agent.

The compound represented by formula (I) and a pharmaceutically acceptable salt thereof according to the present invention can be made into a pharmaceutical composition together with a pharmaceutically acceptable carrier and/or diluent. The pharmaceutical composition can be formed into various dosage forms and administered orally or parenterally. As parenteral administration, intravenous, subcutaneous, intramuscular, transdermal or rectal administration can be mentioned.

A pharmaceutical preparation containing one or two or more of the compound of the present invention or a salt thereof as active ingredients is prepared using a carrier, excipient, and other additives ordinary used for formulation. The carrier and excipient for formulation may be solid or liquid, and examples thereof include lactose, magnesium stearate, starch, talc, gelatin, agar, pectin, gum Arabic, olive oil, sesame oil, cacao butter, ethylene glycol and other conventionally used substances. Administration may be in the forms of both oral administration using tablets, pills, capsules, granules, powder, liquid formulation, and the like and parenteral administration using injections such as formulations for intravenous injection and muscular injection, suppositories, formulation for transdermal administration, and the like.

The 1-biarylazetidinone derivative represented by Formula (I) of the present invention and a pharmaceutically acceptable salt thereof have good properties as pharmaceuticals in terms of safety, stability, drug efficacy, efficacy sustainability, physical properties, pharmacodynamics, storage property, and manufacturability.

The 1-biarylazetidinone derivative represented by formula (I) and the pharmaceutically acceptable salt thereof according to the present invention can be administered at a dose usually in the range of 0.5 to 1000 mg, preferably 5 to 500 mg per dose daily for adults, although the dose varies depending on the type of disease, the administration route, the condition, age, sex, and body weight of a patient, and the like. However, since a dose varies depending on various conditions, a dose less than that described above is sufficient in some cases and a dose exceeding the above range may be required in some cases.

[Example]

The present invention will be described below referring to specific Examples. The present invention is not restricted by these Examples, however.

The structure of an isolated compound was confirmed by $^1$H-NMR. For $^1$H-NMR spectrum (400 MHz, DMSO-$d_6$, $CDCl_3$, or $CD_3OD$), its chemical shift (δ : ppm) and coupling constant (J : Hz) are shown. The following abbreviations denotes as follows: s = singlet, d = doublet, t = triplet, q = quartet, brs = broad singlet, and m = multiplet.

[Reference Example 1]

Synthesis of (1R)-2-bromo-1-(4-fluorophenyl)-1-(1,1,2,2-tetramethyl-1-silapropoxy)ethane

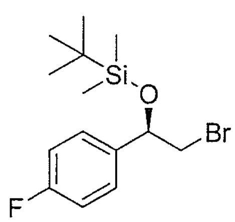

A solution of (R)-5,5-diphenyl-2-methyl-3,4-propano-1,3,2-oxazaborolidine (7.34 g) in dichloromethane (650 mL) was cooled to 0 ˚C and dimethylsulfide borane (50.3 mL) was added under nitrogen atmosphere. A solution of 2-bromo-1-(4-fluorophenyl)ethan-1-one (234 g) in dichloromethane (150 mL) was slowly added dropwise to the resulted solution and the mixture was stirred at 0 ˚C for 30 minutes. Water was added to the reaction solution to separate the organic layer, and then the aqueous layer was extracted with ethyl acetate. Combined organic layers were dried over sodium sulfate and filtered. The solvent was concentrated under reduced pressure, the resultant residue was dissolved in N,N-dimethylformamide, imidazole (294 g) and tert-butyldimethylsilyl chloride (326 g) were added at 0 ˚C, and the resulted mixture was stirred at room temperature overnight. After water was added to the reaction solution, the mixture was extracted with hexane. After the organic layer was washed with water and brine, it was dried over sodium sulfate and filtered. The filtrate was concentrated under reduced pressure and the obtained residue was purified by silica gel column chromatography (ethyl acetate/hexane = 0/100 → 5/95) to obtain the title compound (338 g, 94% yield) as a colorless oil. The NMR data of this compound is shown below.
$^1$H-NMR (400 MHz, $CDCl_3$) δ (ppm) = -0. 10 (s, 3H), 0.10 (s, 3H), 0.88 (s, 9H), 3.40 (dd, 1H, J = 10.4, 4.8 Hz), 3.45 (dd, 1H, J = 10.2, 7.6 Hz), 4.83 (dd, 1H, J = 7.6, 4.6 Hz), 7.03 (t, 2H, J = 8.7 Hz), 7.31 (dd, 2H, J = 8.8, 5.4 Hz) .

[Reference Example 2]

Synthesis of 2-[(2R)-2-(4-fluorophenyl)-2-(1,1,2,2-tetramethyl-1-silapropoxy)ethylthio]acetic acid

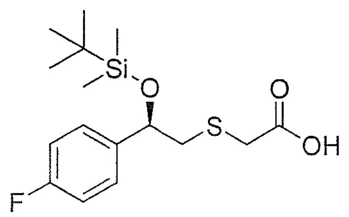

To a solution of (1R)-2-bromo-1-(4-fluorophenyl)-1-(1,1,2,2-tetramethyl-1-silapropoxy)ethane (172 g) in N,N-dimethylformamide (500 mL), methyl thioglycolate (69.2 mL) and triethylamine (216 mL) were added. The reaction solution was stirred at 40 ˚C overnight. After water was added to the reaction solution, the resulted mixture was extracted with hexane. The organic layer was washed with brine, then dried over sodium sulfate, and filtered. The solvent was concentrated under reduced pressure, and the resultant residue was purified by silica gel column chromatography (ethyl acetate/hexane = 1/19) to obtain a reaction product (153 g, yield 83%). A solution of this product in tetrahydrofuran (700 mL) was cooled to 0 ˚C, and 2 M aqueous sodium hydroxide solution (368 mL) and methanol (40 mL) were added, and the resulted mixture was stirred at 0 ˚C for 2 hours. After neutralized with addition of 2 M hydrochloric acid, the mixture was extracted with ethyl acetate. The organic layer was washed with brine, dried over sodium sulfate, and filtered. The filtrate was concentrated under reduced pressure and the residue obtained was purified by silica gel column chromatography (ethyl acetate/hexane = 1/5 → 1/4) to obtain the title compound (130 g, quantitative yield) as a colorless oil. The NMR data of this compound is shown below.
$^1$H-NMR (400 MHz, $CDCl_3$): δ (ppm) = -0.12 (s, 3H), 0.06 (s, 3H), 0.87 (s, 9H), 2.85 (dd, 1H, J = 13.5, 4.9 Hz), 2.94 (dd, 1H, J = 13.5, 7.4 Hz), 3.17 (d, 1H, J = 15.0 Hz), 3.23 (d, 1H, J = 15.0 Hz), 4.83 (dd, 1H, J = 7.4, 4.9 Hz), 7.01 (t, 2H, J = 8.7 Hz), 7.30 (dd, 2H, J = 8.7, 5.4 Hz).

[Reference Example 3]

Synthesis of (4S)-3-{2-[(2R)-2-(4-fluorophenyl)-2-(1,1,2,2-tetramethyl-1-silapropoxy)ethylthio]acetyl}-4-phenyl-1,3-oxazolidin-2-one

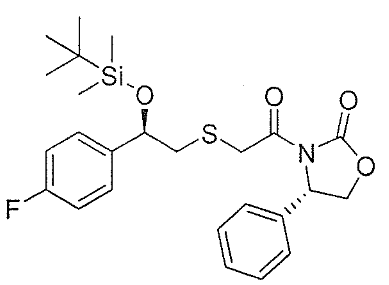

Under nitrogen atmosphere, a solution of 2-[(2R)-2-(4-fluorophenyl)-2-(1,1,2,2-tetramethyl-1-silapropoxy)ethylthio]acetic acid (130 g) in dichloromethane (1000 mL) was cooled to 0 ˚C, triethylamine (113 mL) and pivaroyl chloride (49.6 mL) were added, and the resulted mixture was stirred at 0 ˚C for about 15 minutes. After (S)-(+)-4-phenyl-2-oxazolidinone (60.0 g), N,N-dimethylformamide (300 mL), and 4-(dimethylamino)pyridine (8.98 g) were added to the reaction solution at 0 ˚C, the resulted mixture was stirred at 50 ˚C overnight. After water was added to the reaction solution, the mixture was extracted with diethyl ether. The organic layer was washed with brine, dried over sodium sulfate, and filtered. The filtrate was concentrated under reduced pressure and the obtained residue was purified by silica gel column chromatography (ethyl acetate/hexane = 1/9 → 2/8). The obtained solid was further recrystallized from

hexane to obtain the title compound (89.0 g, 50% yield) as a white solid. The NMR data of this compound is shown below. $^1$H-NMR (400 MHz, $CDCl_3$): δ (ppm) = -0.15 (s, 3H), 0.03 (s, 3H), 0.85 (s, 9H), 2.61 (dd, 1H, J = 13.4, 4.9 Hz), 2.72 (dd, 1H, J = 13.4, 7.7 Hz), 3.67 (d, 1H, J = 13.9 Hz), 3.80 (d, 1H, J = 13.9 Hz), 4.28 (dd, 1H, J = 8.9, 3.8 Hz), 4.68-4.72 (m, 2H), 5.39 (dd, 1H, J = 8.8, 3.7 Hz), 6. 97 (t, 2H, J = 8.7 Hz), 7.22 (dd, 2H, J = 8.4, 5.5 Hz), 7.31-7.37 (m, 5H).

[Reference Example 4]

Synthesis of 1-(4-[2-(4-iodophenyl)-2-azavinyl]phenoxy)-1,1,2,2-tetramethyl-1-silapropane

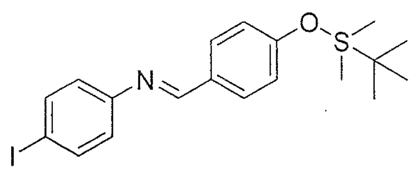

To a solution of 4-hydroxybenzaldehyde (28.1 g) in 2-propanol (460 mL) was added 4-iodoaniline (50.4 g), and the resulted mixture was stirred at 50 ˚C for 30 minutes, then the reaction solution was cooled to room temperature. Solid was filtered off and washed sequentially with 2-propanol and hexane to obtain a reaction product (65.7 g, yield 88%) as a yellow-green solid. This solid (2.58 g) was dissolved in N,N-dimethylformamide (8 mL), then imidazole (1.63 g) and tert-butyldimethylsilyl chloride (1.81 g) were added. The resulted mixture was stirred at room temperature for 1 hour. After water was added to the reaction solution, the resulted mixture was extracted with hexane, dried over sodium sulfate, and filtered. The filtrate was concentrated under reduced pressure to obtain the title compound as a crude product. The crude was used in the following reaction without purification. The NMR data of the crude product is shown below.
$^1$H-NMR (400 MHz, $CDCl_3$): δ (ppm) = 0.24 (s, 6H), 1.00 (s, 9H), 6.91-6.95 (m, 4H), 7.68 (d, 2H, J = 7.8 Hz), 7.78 (d, 2H, J = 8.3 Hz), 8.33 (s, 1H).

[Reference Example 5]

Synthesis of (4S)-3-{(2R,3R)-2-[(2R)-2-(4-fluorophenyl)-2-(1,1,2,2-tetramethyl-1-silapropoxy)ethylthio]-3-[(4-iodophenyl)amino]-3-[4-(1,1,2,2-tetramethyl-1-silapropoxy)phenyllpropanoyll-4-phenyl-1,3-oxazolidin-2-one

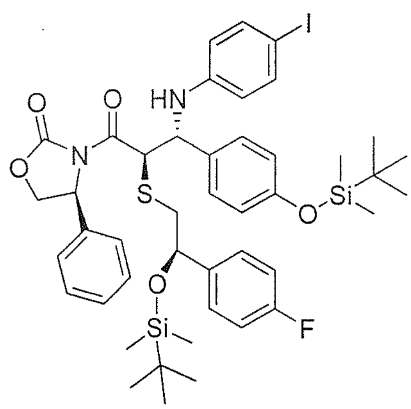

Under nitrogen atmosphere, a solution of (4S)-3-{2-[(2R)-2-(4-fluorophenyl)-2-(1,1,2,2-tetramethyl-1-silapropoxy)ethylthio]acetyl}-4-phenyl-1,3-oxazolidin-2-one (1.96 g) and 1-{4-[2-(4-iodophenyl)-2-azavinyl]phenoxy}-1,1,2,2-tetramethyl-1-silapropane (whole amount obtained in Reference Example 4) in dichloromethane (20 mL) was cooled to -5 ˚C, then N-ethyldiisopropylamine (3.42 mL) was added, followed by adding trimethylsilyl chloride (1.02 mL) dropwise

over 30 minutes. The reaction solution was further stirred at -5 ˚C for 30 minutes and then cooled to -35 ˚C. After titanium tetrachloride (0.483 mL) was added dropwise over 30 minutes, the resulted mixture was stirred at -35 ˚C to -25 ˚C for 4 hours. An aqueous solution (50 mL) of 5 equivalents of tartaric acid was slowly added at -10 ˚C or lower, and the reaction mixture was then filtered and washed with dichloromethane. The organic layer of the filtrate was separated, washed with brine, dried over sodium sulfate, and filtered. The filtrate was concentrated under reduced pressure and the obtained residue was purified by silica gel column chromatography (ethyl acetate/hexane = 5/95→10/90) to obtain the title compound (2.78 g, 75% yield) as an oily compound. The NMR data of this compound is shown below.
$^1$H-NMR (400 MHz, $CDCl_3$): δ (ppm) = -0.18 (s, 3H), -0.01 (s, 3H), 0.15 (s, 6H), 0.83 (s, 9H), 0.97 (s, 9H), 2.60 (dd, 1H, J = 13.0, 5.0 Hz), 2.69 (dd, 1H, J = 13.0, 7.4 Hz), 4.17 (dd, 1H, J = 8.8, 3.2 Hz), 4.58-4.65 (m, 3H), 5.08 (d, 1H, J = 9.0 Hz), 5.28-5.32 (m, 2H), 6.24 (d, 2H, J = 8.8 Hz), 6.67 (d, 2H, J = 8.5 Hz), 6.89-7.29 (m, 13H).

[Reference Example 6]

Synthesis of (3R,4R)-3-[(2R)-2-(4-fluorophenyl)-2-(1,1,2,2-tetramethyl-1-silapropoxy)ethylthio]-1-(4-iodophenyl)-4-[4-(1,1,2,2-tetramethyl-1-silapropoxy)phenyl]azetidin-2-one

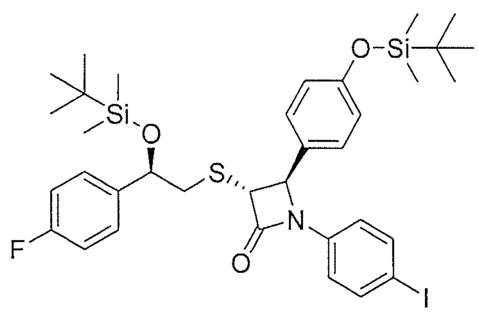

To a solution of (4S)-3-{2R,3R)-2-[(2R)-2-(4-fluorophenyl)-2-(1,1,2,2-tetramethyl-1-silapropoxy)ethylthio]-3-[(4-iodophenyl) amino]-3-[4-(1,1,2,2-tetramethyl-1-silapropoxy) phenyl] propanoyl}-4-phenyl-1,3-oxazolidin-2-one (1.39 g) in tert-butyl methyl ether (12 mL) was added N,O-bis(trimethylsilyl)acetamide (1.11 mL) at room temperature, and the resulted mixture was stirred for 30 minutes, then 1 M solution of tetrabutylammonium fluoride in tetrahydrofuran (0.150 mL) was added, and the resulted mixture was stirred at room temperature for 5 hours. After addition of saturated aqueous ammonium chloride, the mixture was extracted with ethyl acetate. The organic layer was washed with brine, dried over sodium sulfate, and filtered. The filtrate was concentrated under reduced pressure and the obtained residue was purified by silica gel column chromatography (ethyl acetate/hexane = 0/100 → 5/95) to obtain the title compound (0.505 g, 44% yield) as an oil. The NMR data of this compound is shown below.
$^1$H-NMR (400 MHz, $CDCl_3$): δ (ppm) = -0.17 (s, 3H), -0.03 (s, 3H), 0.20 (s, 6H), 0.81 (s, 9H), 0.97 (s, 9H), 2.84 (dd, 1H, J = 13.2, 4.9 Hz), 3.08 (dd, 1H, J = 13.2, 7.6 Hz), 3.88 (d, 1H, J = 2.0 Hz), 4.58 (d, 1H, J = 2.0 Hz), 4.81-4.84 (m, 1H), 6.82 (d, 2H, J = 8.0 Hz), 6.94-7.02 (m, 4H), 7.14 (d, 2H, J = 8.3 Hz), 7.26-7.30 (m, 2H), 7.53 (d, 2H, J = 8.3 Hz).

[Reference Example 7]

Synthesis of (3R, 4R)-3-[(2R)-2-(4-fluorophenyl)-2-(1,1,2,2-tetramethyl-1-silapropoxy)ethylthio]-1-[4-(pyridin-3-yl)phenyl]-4-[4-(1,1,2,2-tetramethyl-1-silapropoxy)phenyl]azetidin-2-one

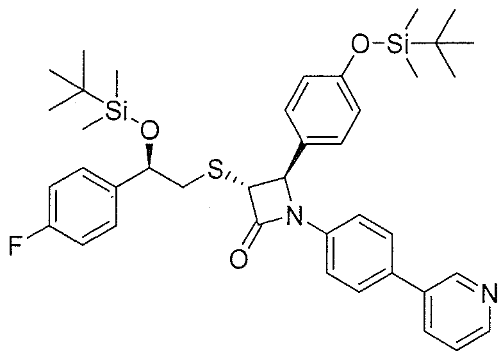

(3R, 4R)- 3-[(2R)- 2-(4- fluorophenyl)- 2-(1,1,2,2- tetramethyl- 1- silapropoxy) ethylthio]- 1-(4- iodophenyl)- 4-[4-(1,1,2,2-tetramethyl-1-silapropoxy)phenyl]azetidin-2-one (59.3 g), 3-pyridyl boronic acid (14.3 g), and tetrakis (triphenylphosphine)palladium (9.00 g) were dissolved in toluene (380 mL) and ethanol (150 mL), and 2 M aqueous potassium carbonate (80 mL) was added. After the reaction solution was degassed, the solution was stirred at 80 ˚C for 3 hours. The reaction solution was cooled to room temperature, washed with brine, then dried over sodium sulfate, and filtered. The filtrate was concentrated under reduced pressure, and the residue thus obtained was purified by silica gel column chromatography (ethyl acetate/hexane = 2/8→3/7) to obtain the title compound as a brownish-red solid (41.6 g, 75% yield). The NMR data of this compound is shown below.
1H-NMR (400 MHz, $CDCl_3$): δ (ppm) = -0.16 (s, 3H), -0.01 (s, 3H), 0.20 (s, 6H), 0.82 (s, 9H), 0.98 (s, 9H), 2.87 (dd, 1H, J = 13.4, 5.1 Hz), 3.11 (dd, 1H, J = 13.4, 7.6 Hz), 3.93 (d, 1H, J = 2.0 Hz), 4.66 (d, 1H, J = 2.0 Hz), 4.85 (dd, 1H, J = 7.6, 5.1 Hz), 6.85 (d, 2H, J = 8.3 Hz), 6.97 (t, 2H, J = 8.7 Hz), 7.19-7.47 (m, 9H), 7.79 (d, 1H, J = 7.8 Hz), 8.55 (d, 1H, J = 4.6 Hz), 8.76 (brs, 1H).

[Example 1]

Synthesis of (3R,4R)-3-[(2R)-2-(4-fluorophenyl)-2-hydroxyethylthio]-4-(4-hydroxyphenyl)-1[4-(pyridin-3-yl)phenyl]azetidin-2-one

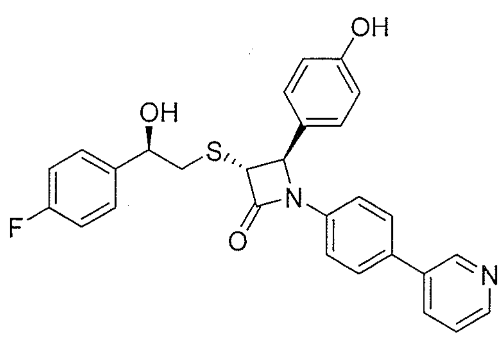

(3R,4R)-3-[(2R)-2-(4-fluorophenyl)-2-(1,1,2,2-tetramethyl-1-silapropoxy)ethylthio]-1-[4-(pyridin-3-yl)phenyl]-4-[4-(1,1,2,2-tetramethyl-1-silapropoxy)phenyl]azetidin-2-one (5.13 g) was dissolved in tetrahydrofuran (21 mL) and water (7 mL). The resulted mixture was then cooled to 0 ˚C. 4 N hydrogen chloride/dioxane solution (18 mL) was added dropwise, and the resulted mixture was stirred at 0 ˚C for 1 hour, then at room temperature for further 2 hours. Ethyl acetate and brine were added to the reaction solution and the resulted mixture was stirred for a while. The organic layer was separated and the aqueous layer was extracted with ethyl acetate. The combined organic fraction was sequentially washed with a saturated aqueous sodium hydrogen carbonate and brine, dried over sodium sulfate, and filtered. The filtrate was concentrated under reduced pressure, and the residue obtained was purified by silica gel column chromatography (ethyl acetate/hexane = 3/7) to obtain the title compound (2.86 g, 82% yield). The NMR data of this compound is shown below.
$^1$H-NMR (400 MHz, DMSO-$d_6$): δ (ppm) = 2.93 (d, 2H, J = 6.3 Hz), 4.27 (d, 1H, J = 2.4 Hz), 4.70-4.75 (m, 1H), 5.04 (d,

1H, J = 2.4 Hz), 5.64 (d, 1H, J = 4.6 Hz), 6.77 (t, 2H, J = 4.3 Hz), 7.08-7.13 (m, 2H), 7.27-7.44 (m, 7H), 7.67 (d, 2H, J = 8.8 Hz), 7.99 (d, 1H, J = 8.0 Hz), 8.50-8.52 (m, 1H), 8.81 (d, 1H, J = 2.2 Hz), 9.60 (s, 1H).

[Reference Example 8]

Synthesis of (4S)-3-{2-[(4-methoxyphenyl)methylthio]acetyl}-4-phenyl-1,3-oxazolidin-2-one

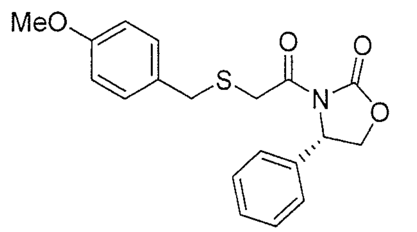

To a solution of sodium hydride (13.5 g) in tetrahydrofuran (920 mL) was added (4S)-4-phenyl-1,3-oxazolidin-2-one (50 g) portionwise at room temperature, and the resulted mixture was stirred for 1 hour. This reaction solution is referred to as Solution A. In a separate reaction vessel, a solution of chloroacetyl chloride (36.6 mL) in tetrahydrofuran (308 mL) was cooled to 0 ˚C, and the above Solution A was added dropwise in about 5 mL for each portion. After stirred at 0 ˚C for 1 hour, water (300 mL) was slowly added to the reaction solution. The resulted mixture was extracted with ethyl acetate. The organic layer was washed with water and brine, then dried over sodium sulfate, and filtered. The filtrate was concentrated under reduced pressure, the obtained residue was dissolved in dichloromethane (500 mL). To the resulted solution was added p-methoxybenzylthiol (47.2 g) at room temperature, followed by adding triethylamine (64 mL) dropwise using a dropping funnel over 1.5 hours. The resulted solution was then stirred at room temperature overnight. Water (300 mL) and dichloromethane (100 mL) were added to the reaction solution to separate the organic layer and the organic layer was then washed with brine. The organic layer was dried over sodium sulfate and filtered. The filtrate was concentrated under reduced pressure and the obtained residue was purified by silica gel column chromatography (ethyl acetate/hexane = 1/5 → 1/3 → 1/2) to obtain the title compound (90 g, 82% yield for 2 steps). The NMR data of this compound is shown below.
$^1$H-NMR (400 MHz, $CDCl_3$): δ (ppm) = 3.49-3.61 (m, 3H), 3.77-3.81 (m, 1H), 3.78 (s, 3H), 4.28 (dd, 1H, J = 8.9, 3.9 Hz), 4.62 (dd, 1H, J = 8.9 Hz), 5.43 (dd, 1H, J = 8.9, 3.9 Hz), 6.78-6.81 (m, 2H), 7.14-7.18 (m, 2H), 7.34-7.43 (m, 5H).

[Reference Example 9]

Synthesis of (4S)-3-{(2R,3R)-3-[(iodophenyl)amino]-2-[(4-methoxyphenyl)methylthio]-3-[4-(1,1,2,2-tetramethyl-1-silapropoxy)phenyl]propanoyl}-4-phenyl-1,3-oxazolidin-2-one

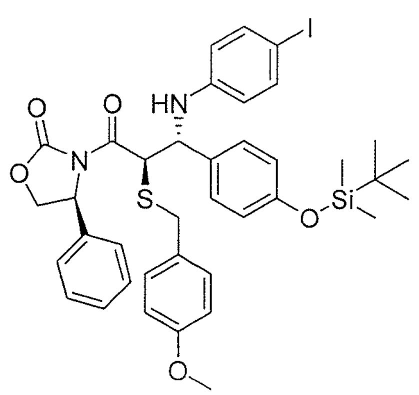

Under nitrogen atmosphere, a solution of (4S)-3-{2-[(4-methoxyphenyl)methylthio]acetyl}-4-phenyl-1,3-oxazolidin-2-one (32 g) and 1-{4-[(1E)-2-(4-iodophenyl)-2-azavinyl]phenoxyl-1,1,2,2-tetramethyl-1-silapropane (78 g) in dichloromethane (450 mL) was cooled to -5 ˚C. Then N-ethyldiisopropylamine (76 mL) was added, followed by adding trimethylsilyl chloride (36 mL) dropwise over 30 minutes. The reaction solution was stirred at -5 ˚C for 30 minutes and then cooled to -35 ˚C. Titanium chloride (10.8 mL) was added dropwise over 30 minutes and the resulted mixture was stirred at -35 ˚C to -25 ˚C for 3 hours. After added 4 equivalents of acetic acid (20 mL), an aqueous solution (500 mL) of 4 equivalents of aqueous tartaric acid were slowly added dropwise at -10 ˚C or lower. The resulted mixture was extracted with dichloromethane (300 mL). The organic layer of the filtrate was separated, washed with brine, dried over sodium sulfate, and filtered. The filtrate was concentrated under reduced pressure and the obtained residue was purified by silica gel column chromatography (ethyl acetate/hexane = 5/95 → 10/90) to obtain the title compound (64 g, 75% yield). The NMR data of this compound is shown below.
$^1$H-NMR (400 MHz, $CDCl_3$): δ (ppm) = 0.18 (s, 6H), 0.98 (s, 9H), 3.52-3.79 (m, 2H), 3.81 (s, 3H), 4.04 (dd, 1H, J = 8.5, 3.2 Hz), 4.39-4.48 (m, 2H), 5.10 (dd, 1H, J = 8.5, 3.2 Hz), 5.28-5.32 (m, 1H), 5.43 (d, 1H, J = 6.4 Hz), 6.17-6.18 (m, 2H), 6.60-6.66 (m, 2H), 6.71-6.87 (m, 4H), 6.97-7.02 (m, 2H), 7.06-7.31 (m, 7H).

[Reference Example 10]

Synthesis of (3R,4R)-1-(4-iodophenyl)-3-[(4-methoxyphenyl)methylthio]-4-[4-(1,1,2,2-tetramethyl-1-silapropoxy)phenyl]azetidin-2-one

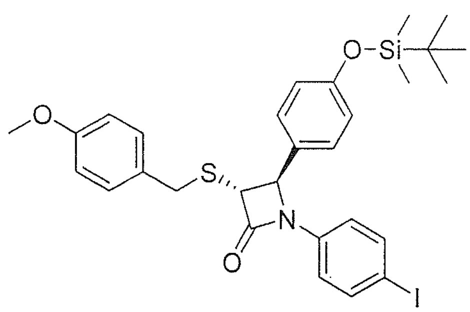

To a solution of (4S)-3-{(2R,3R)-3-[(iodophenyl)amino]-2-[(4-methoxyphenyl)methylthio]-3-[4-(1,1,2,2-tetramethyl-1-silapropoxy)phenyl]propanoyl}-4-phenyl-1,3-oxazolidin-2-one (7.5 g) in tert-butylmethyl ether (75 mL) was added N,O-bis(trimethylsilyl)acetamide (7.0 mL) at room temperature. The resulted mixture was stirred for 1 hour, then 1 M solution of tetrabutylammonium fluoride in tetrahydrofuran (0.94 mL) was added, and the resulted mixture was stirred at room temperature overnight. After addition of saturated aqueous ammonium chloride, the resulted mixture was extracted with ethyl acetate. The organic layer was washed with water and brine, dried over sodium sulfate, and filtered. The filtrate was concentrated under reduced pressure and the obtained residue was purified by silica gel column chromatography (ethyl acetate/hexane = 0/100 → 5/95) to obtain the title compound (2.5 g, 42% yield) as an oil. The NMR data of this compound is shown below.
$^1$H-NMR (400 MHz, $CDCl_3$): δ (ppm) = 0.19 (s, 6H), 0.97 (s, 9H), 3.75 (s, 3H), 3.89-3.90 (m, 2H), 3.90 (d, 1H, J = 2.2 Hz), 4.48 (d, 1H, J = 2.2 Hz), 6.69-6.72 (m, 2H), 6.77-6.79 (m, 2H), 6.95-7.00 (m, 2H), 7.04-7.05 (m, 2H), 7.14-7.15 (m, 2H), 7.50-7.53 (m, 2H).

[Reference Example 11]

Synthesis of (3R,4R)-1-(4-iodophenyl)-3-sulfanyl-4-[4-(1,1,2,2-tetramethyl-1-silapropoxy)phenyl]azetidin-2-one

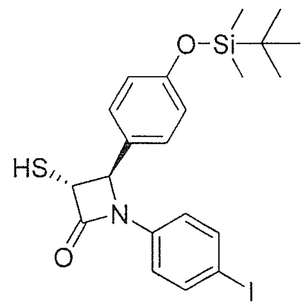

Under nitrogen atmosphere, (3R,4R)-1-(4-iodophenyl)-3-[(4-methoxyphenyl)methylthio]-4-[4-(1,1,2,2-tetramethyl-1-silapropoxy)phenyl]azetidin-2-one (293 mg) was dissolved in trifluoroacetic acid (2.3 mL), then mercury acetate (II) (148 mg) was added at 0 ˚C, and the resulted mixture was stirred at 0 ˚C for 15 minutes. Toluene (4.6 mL) and water (4.6 mL) were added, the resulted mixture was separated into two layers, and then the organic layer was extracted with toluene. Then the organic layer was washed with water, dried over sodium sulfate, and filtered. The filtrate was concentrated under reduced pressure and the obtained residue was purified by silica gel column chromatography (ethyl acetate/hexane = 1/9) to obtain the title compound (236 g, quantitative yield). The NMR data of this compound is shown below.
$^1$H-NMR (400 MHz, $CDCl_3$) δ (ppm) = 0.20 (s, 6H), 0.98 (s, 9H), 4.41 (d, 1H, J = 2.2 Hz), 4.83 (d, 1H, J = 2.0 Hz), 6.82-6.84 (m, 2H), 6.96-6.98 (m, 2H), 7.19-7.21 (m, 2H), 7.50-7.52 (m, 2H).

[Reference Example 12]

Synthesis of (3R,4R)-3-(2-benzo[b]thiophen-5-yl-2-oxoethylthio)-1-(4-iodophenyl)-4-[4-(1,1,2,2-tetramethyl-1-silapropoxy)phenyl]azetidin-2-one

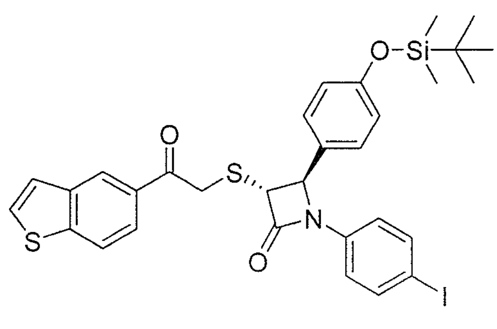

(3R,4R)-1-(4-iodophenyl)-3-sulfanyl-4-[4-(1,1,2,2-tetramethyl-1-silapropoxy)phenyl]azetidin-2-one (1.0 g) was dissolved in dichloromethane (10 mL), then triethylamine (1.7 mL) and 1-benzo[b]thiophen-5-yl-2-bromoethan-1-one (1.0 g) were added, and the resulted mixture was stirred at room temperature overnight. Water was added to the reaction solution and the resulted mixture was extracted with ethyl acetate. The organic layer was washed with brine, then dried over sodium sulfate, and filtered. The filtrate was concentrated under reduced pressure and the obtained residue was purified by silica gel column chromatography (ethyl acetate/hexane = 1/9) to obtain the title compound (850 mg, 63% yield). The NMR data of this compound is shown below.
$^1$H-NMR (400 MHz, $CDCl_3$): δ ppm) = 0.18-0.19 (m, 6H), 0.97 (s, 9H), 4.12 (d, 1H, J = 2.2 Hz), 4.27 (s, 2H), 4.81 (d, 1H, J = 2.4 Hz), 6.79-6.82 (m, 2H), 6.97-6.99 (m, 2H), 7.17-7.19 (m, 2H), 7.41-7.43 (m, 1H), 7.48-7.50 (m, 2H), 7.54-7.55 (m, 1H), 7.89-7.94 (m, 2H), 8.40-8.41 (m, 1H).

[Reference Example 13]

Synthesis of (3R,4R)-3-((2R)-2-benzo[b]thiophen-5-yl-2-hydroxyethylthio)-1-(4-iodophenyl)-4-[4-(1,1,2,2-tetramethyl-1-silapropoxy)phenyl]azetidin-2-one

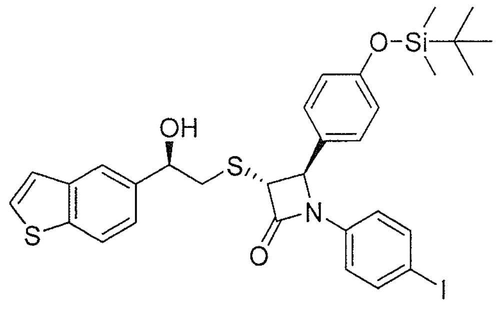

Under nitrogen atmosphere, a solution of (R)-5,5-diphenyl-2-methyl-3,4-propano-1,3,2-oxazaborolidine (129 mg) in dichloromethane (1 mL) was cooled to 0 °C and dimethyl sulfide borane (1.28 mL, 1 M dichloromethane solution) was added. To this solution, a solution of (3R,4R)-3-(2-benzo[b]thiophen-5-yl-2-oxoethylthio)-1-(4-iodophenyl)-4-[4-(1,1,2,2-tetramethyl-1-silapropoxy)phenyl]azetidin-2-one (800 mg) in dichloromethane (1 mL) was slowly added dropwise and the resulted mixture was stirred at 0 °C for 30 minutes. After water was added to the reaction solution to separate the organic layer, the aqueous layer was extracted with dichloromethane. The combined organic layers were dried over sodium sulfate and filtered. The filtrate was concentrated under reduced pressure and the obtained residue was purified by silica gel column chromatography (ethyl acetate/hexane = 1/9) to obtain the title compound (655 mg, 82% yield). The NMR data of this compound is shown below.
$^1$H-NMR (400 MHz, $CDCl_3$): δ (ppm) = 0.21-0.21 (m, 6H), 0.98 (s, 9H), 3.08-3.10 (m, 2H), 3.32 (d, 1H, J = 4.4 Hz), 4.01 (d, 1H, J = 2.4 Hz), 4.68 (d, 1H, J = 2.4 Hz), 5.02-5.06 (m, 1H), 6.83-6.85 (m, 2H), 7.02-7.04 (m, 2H), 7.17-7.19 (m, 2H), 7.30-7.33 (m, 2H), 7.45-7.46 (m, 1H), 7.54-7.56 (m, 2H), 7.82-7.84 (m, 2H).

[Reference Example 14]

Synthesis of (3R,4R)-3-[(2R)-2-benzo[b]thiophen-5-yl-2-(1,1,2,2-tetramethyl-1-silapropoxy)ethylthio]-1-(4-iodophenyl)-4-[4-(1,1,2,2-tetramethyl-1-silapropoxy)phenyl]azetidin-2-one

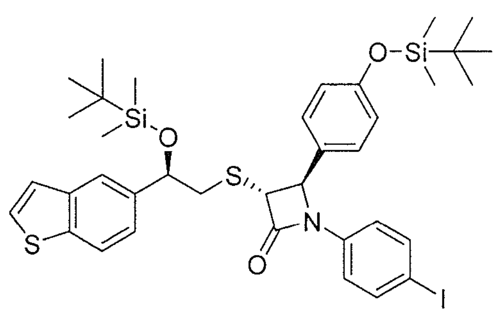

(3R,4R)-3-((2R)-2-benzo[b]thiophen-5-yl-2-hydroxyethylthio)-1-(4-iodophenyl)-4-[4-(1,1,2,2-tetramethyl-1-silapropoxy)phenyl]azetidin-2-one (655 mg) was dissolved in N,N-dimethylformamide (1 mL), then imidazole (195 mg) and tert-butyldimethylsilyl chloride (215 mg) were added, and the resulted mixture was stirred at room temperature for 3 hours. After water was added to the reaction solution, the resulted mixture was extracted with ethyl acetate. The organic layer was washed with water and brine, then dried over sodium sulfate, and filtered. The filtrate was concentrated

under reduced pressure and the obtained residue was washed with hexane to obtain the title compound (670 mg, 88% yield). The NMR data of this compound is shown below.
$^1$H-NMR (400 MHz, $CDCl_3$): δ (ppm) = -0.16 (s, 3H), 0.00-0.00 (m, 3H), 0.19-0.19 (m, 6H), 0.83 (s, 9H), 0.97 (s, 9H), 2.88-2.92 (m, 1H), 3.16 (dd, 1H, J = 13.4, 7.6 Hz), 3.90 (d, 1H, J = 2.4 Hz), 4.46 (d, 1H, J = 2.2 Hz), 4.94-4.98 (m, 1H), 6.77-6.80 (m, 2H), 6.97-6.99 (m, 2H), 7.05-7.07 (m, 2H), 7.29-7.34 (m, 2H), 7.44-7.46 (m, 1H), 7.51-7.53 (m, 2H), 7.75-7.79 (m, 2H).

[Reference Example 15]

Synthesis of (3R,4R)-3-[(2R)-2-(4-fluorophenyl)-2-(1,1,2,2-tetramethyl-1-silapropoxy)ethylthio]-1-[4-(4,4,5,5-tetramethyl(1,3,2-dioxaborolan-2-yl))phenyl]-4-[4-(1,1,2,2-tetramethyl-1-silapropoxy)phenyl]azetidin-2-one

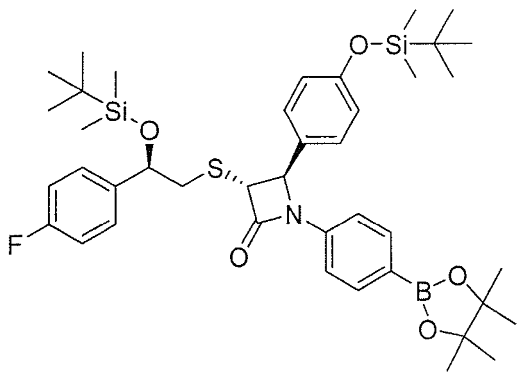

To a solution of (3R,4R)-3-[(2R)-2-(4-fluorophenyl)-2-(1,1,2,2-tetramethyl-1-silapropoxy)ethylthio]-1-(4-iodophenyl)-4-[4-(1,1,2,2-tetramethyl-1-silapropoxy)phenyl]azetidin-2-one (5.58 g) in 1,4-dioxane (130 mL) were added bis (pinacolato)diboron (3.71 g), potassium acetate (2.15 g) and [1,1'-bis(diphenylphosphino)ferrocene]dichloropalladium (II)dichloromethane (1:1) complex (597 mg) . The reaction solution was degassed and then stirred at 75 ˚C overnight. The reaction solution was cooled to room temperature, diluted with ethyl acetate, and then filtered through Celite. The filtrate was washed with brine, then dried over sodium sulfate, and filtered. The filtrate was concentrated under reduced pressure and the obtained residue was purified by silica gel column chromatography (ethyl acetate/hexane = 1/20 → 1/15 → 1/10) to obtain the title compound (2.95 g, 53% yield) as a colorless oil. The NMR data of this compound is shown below.
$^1$H-NMR (400 MHz, DMSO-$d_6$): δ (ppm) = -0.21 (s, 3H), -0.08 (s, 3H), 0.16 (m, 6H), 0.74 (s, 9H), 0.91 (s, 9H), 1.19-1.27 (m, 12H), 2.83-3.03 (m, 2H), 4.28 (d, 1H, J = 2.2 Hz), 4.89-4.91 (m, 1H), 5.08 (d, 1H, J = 2.4 Hz), 6.82-6.88 (m, 2H), 7.09-7.16 (m, 2H), 7.18-7.23 (m, 2H), 7.27-7.33 (m, 2H), 7.33-7.40 (m, 2H), 7.53-7.59 (m, 2H).

[Example 2]

Synthesis of (3R,4R)-3-[(2R)-2-(4-fluorophenyl)-2-hydroxyethylthio]-4-(2,4-dihydroxyphenyl)-1-[4-(pyridin-3-yl)phenyl] azetidin-2-one

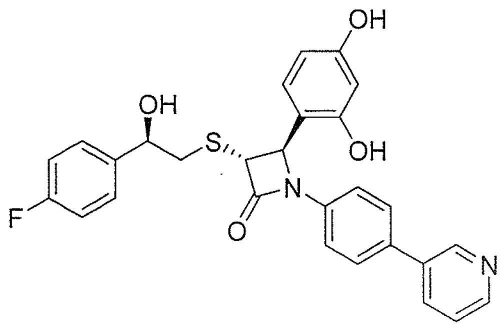

(3R,4R)-3-[(2R)-2-(4-fluorophenyl)-2-(1,1,2,2-tetramethyl-1-silapropoxy)ethylthio]-4-[2,4-bis(1,1,2,2-tetramethyl-1-silapropoxy)phenyl]-1-(4-iodophenyl)azetidin-2-one (2.79 g), 3-pyridylboronic acid (575 mg), and tetrakis(triphenylphosphine)palladium (361 mg) were dissolved in toluene (30 mL) and ethanol (12 mL). Then 2 M aqueous potassium carbonate (3.12 mL) was added to the resulted mixture. The reaction solution was degassed and then stirred at 80 ˚C for 5 hours. The reaction solution was cooled to room temperature, washed with brine, then dried over sodium sulfate, and filtered. The filtrate was concentrated under reduced pressure and the obtained residue was purified by silica gel column chromatography (ethyl acetate/hexane = 2/8) to obtain the reaction product (1.1 g). A solution of the obtained product in dichloromethane (10.4 mL) was added to a mixed solution of pyridine (7.81 mL) and hydrogen fluoride-pyridine (2.60 mL) at 0 ˚C. The resulted mixture was stirred at 0 ˚C for 30 minutes and then at room temperature overnight. The organic layer was washed with water, then dried over sodium sulfate, and filtered. The filtrate was concentrated under reduced pressure and the obtained residue was purified by silica gel column chromatography (ethyl acetate/hexane = 8/2) to obtain the title compound (470 mg, 32% yield for 2 steps). The NMR data of this compound is shown below.
$^1$H-NMR (400 MHz, DMSO-$d_6$): δ (ppm) = 2.90-2.98 (m, 2H), 4.37 (d, 1H, J = 2.2 Hz), 4.70 (q, 1H, J = 5.8 Hz), 5.07 (d, 1H, J = 2.2 Hz), 5.65 (d, 1H, J = 4.6 Hz), 6.22 (dd, 1H, J = 8.4, 2.3 Hz), 6.31 (d, 1H, J = 2.2 Hz), 7.06-7.11 (m, 3H), 7.29-7.44 (m, 5H), 7.67 (d, 2H, J = 8.8 Hz), 7.99 (d, 1H, J = 8.0 Hz), 8.51 (dd, 1H, J = 4.6, 1.5 Hz), 8.82 (d, 1H, J = 2.2 Hz), 9.45 (s, 1H), 9.83 (s, 1H).

[Example 3]

Synthesis of (3R,4R)-3-[(2R)-2-(4-fluorophenyl)-2-hydroxyethylthio]-4-(4-hydroxyphenyl)-1-[4-(5-trifluoromethylpyridin-2-yl)phenyl]azetidin-2-one

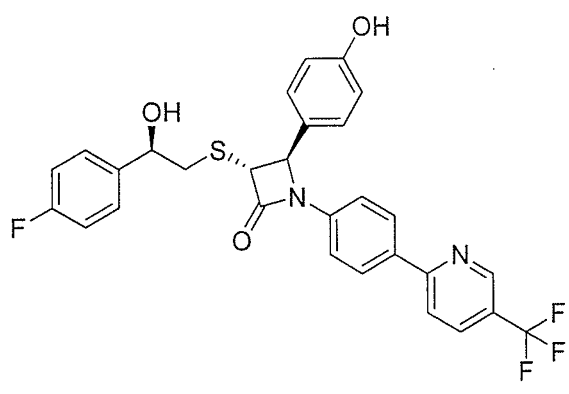

(3R,4R)-3-[(2R)-2-(4-fluorophenyl)-2-(1,1,2,2-tetramethyl-1-silapropoxy)ethylthio]-1-[4-(4,4,5,5-tetramethyl(1,3,2-dioxaborolan-2-yl))phenyl]-4-[4-(1,1,2,2-tetramethyl-1-silapropoxy)phenyl]azetidin-2-one (350 mg), 2-bromo-5-(trifluoromethyl)pyridine (207 mg), and tetrakis(triphenylphosphine)palladium (catalytic amount) were dissolved in a mixed solution of toluene/ethanol = 5/2 (8 mL). To this solution was added 2 M aqueous potassium carbonate (0.46 mL). The reaction solution was degassed and then stirred at 80 ˚C for 2 hours. The reaction solution was cooled to room temperature and diluted with ethyl acetate. The resulted solution was washed sequentially with water and brine, then

dried over sodium sulfate, and filtered. The filtrate was concentrated under reduced pressure and the obtained residue was purified by silica gel column chromatography (ethyl acetate/hexane = 1/8). After the obtained reaction product was dissolved in tetrahydrofuran (5 mL) and water (1 mL), 4 N hydrogen chloride/dioxane solution (1 mL) was added dropwise and stirred at room temperature overnight. The reaction solution was diluted with ethyl acetate, and washed sequentially with water, saturated aqueous sodium hydrogen carbonate, and brine, dried over sodium sulfate, and filtered. The filtrate was concentrated under reduced pressure and the obtained residue was purified by silica gel column chromatography (ethyl acetate/hexane = 1/1) to obtain the title compound (149 mg, 59% yield for 2 steps). The NMR data of this compound is shown below. $^1$H-NMR (400 MHz, DMSO-$d_6$) : δ (ppm) = 2.92-2.94 (m, 2H), 4.29 (d, 1H, J = 2.4 Hz), 4.71-4.74 (m, 1H), 5.08 (d, 1H, J = 2.4 Hz), 5.65-5.66 (m, 1H), 6.75-6.78 (m, 2H), 7.09-7.12 (m, 2H), 7.27-7.29 (m, 2H), 7.33-7.36 (m, 4H), 8.09-8.11 (m, 3H), 8.22 (dd, 1H, J = 8.7, 2.3 Hz), 8.97-8.97 (m, 1H), 9.60 (s, 1H).

[Examples 4 to 106]

Compounds of Example Nos. 4 to 106 shown below were synthesized using the respective corresponding starting materials and reagents in accordance with the synthesis method described in Example 1 above. The Example No., structure, and $^1$H-NMR spectrum (400 MHz) of the respective compounds are summarized in Tables 1 to 21. The type of deuterated solvent used in the measurement is shown at the beginning of the $^1$H-NMR spectrum date for the respective compounds.

[Table 1]

| Example No. | Structure | NMR data δ(ppm) |
|---|---|---|
| 4 | 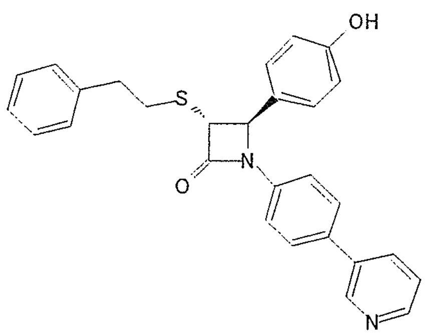 | (CDCl3) δ: 2.91-3.02 (m, 4H), 3.97 (d, 1H, J = 2.4 Hz), 4.73 (d, 1H, J = 2.2 Hz), 6.84 (d, 2H, J = 8.5 Hz), 7.16-7.29 (m, 7H), 7.36-7.46 (m, 5H), 7.87 (d, 1H, J = 8.0 Hz), 8.30 (s, 1H), 8.55 (d, 1H, J = 4.9 Hz), 8.71 (d, 1H, J = 2.2 Hz). |
| 5 | 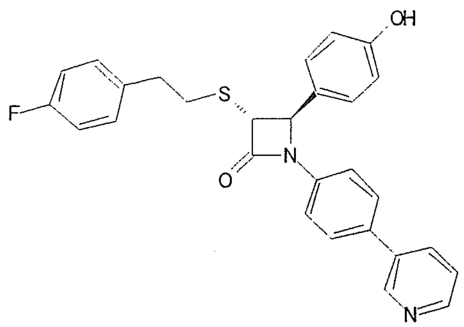 | (CDCl3) δ: 2.89-2.99 (m, 4H), 3.95 (d, 1H, J = 2.2 Hz), 4.73 (d, 1H, J = 2.2 Hz), 6.85 (d, 2H, J = 8.5 Hz), 6.95 (t, 2H, J = 8.5 Hz), 7.11-7.19 (m, 4H), 7.36-7.46 (m, 5H), 7.88 (d, 1H, J = 8.0 Hz), 8.55 (d, 1H, J = 4.9 Hz), 8.70 (d, 1H, J = 2.4 Hz), 8.94 (s, 1H). |
| 6 | 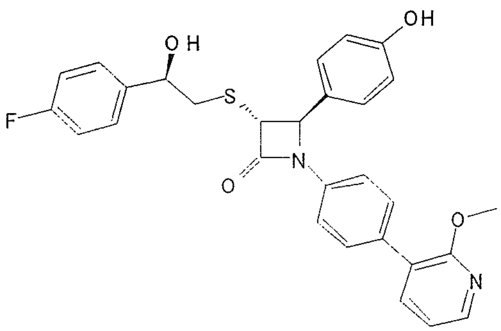 | (DMSO-d6) δ: 2.92-2.93 (m, 2H), 3.82 (s, 3H), 4.26 (d, 1H, J = 2.2 Hz), 4.71-4.74 (m, 1H), 5.03 (d, 1H, J = 2.2 Hz), 5.68 (d, 1H, J = 4.6 Hz), 6.78 (d, 2H, J = 8.5 Hz), 7.04 (dd, 1H, J = 7.3, 4.9 Hz), 7.09-7.12 (m, 2H), 7.26-7.28 (m, 4H), 7.33-7.36 (m, 2H), 7.48 (d, 2H, J = 8.5 Hz), 7.67 (dd, 1H, J = 7.3, 1.7 Hz), 8.12 (dd, 1H, J = 4.9, 1.7 Hz), 9.62 (s, 1H). |

(continued)

| Example No. | Structure | NMR data δ(ppm) |
|---|---|---|
| 7 | 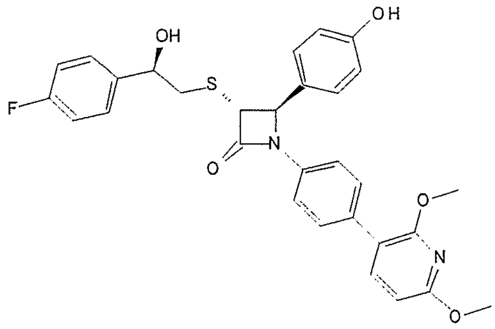 | (DMSO-d6) δ: 2.91-2.93 (m, 2H), 3.85 (s, 3H), 3.87 (s, 3H), 4.24 (d, 1H, J = 2.2 Hz), 4.71-4.73 (m, 1H), 5.01 (d, 1H, J = 2.4 Hz), 5.67 (d, 1H, J = 4.6 Hz), 6.43 (d, 1H, J = 8.0 Hz), 6.77 (d, 2H, J = 8.5 Hz), 7.09-7.12 (m, 2H), 7.22-7.28 (m, 4H), 7.34-7.36 (m, 2H), 7.43 (d, 2H, J = 8.5 Hz), 7.63 (d, 1H, J = 8.0 Hz), 9.61 (s, 1H). |
| 8 | 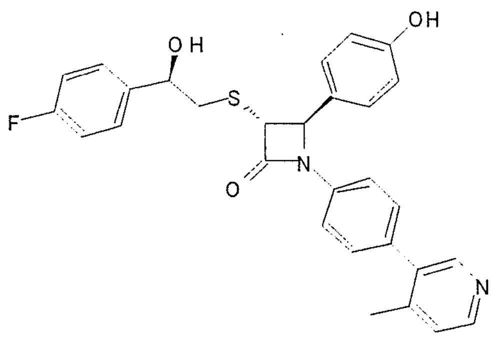 | (DMSO-d6) δ: 2.43 (s, 3H), 2.93-2.94 (m, 2H), 4.32 (d, 1H, J = 2.0 Hz), 4.72-4.73 (m, 1H), 5.08 (d, 1H, J = 2.2 Hz), 6.79 (d, 2H, J = 8.3 Hz), 7.11 (t, 2H, J = 8.8 Hz), 7.29-7.37 (m, 6H), 7.46 (d, 2H, J = 8.3 Hz), 7.91 (d, 1H, J = 5.9 Hz), 8.67 (s, 1H), 8.70 (d, 1H, J = 5.9 Hz), 9.69 (br s, 1H). |

[Table 2]

| Example No. | Structure | NMR data δ(ppm) |
|---|---|---|
| 9 | 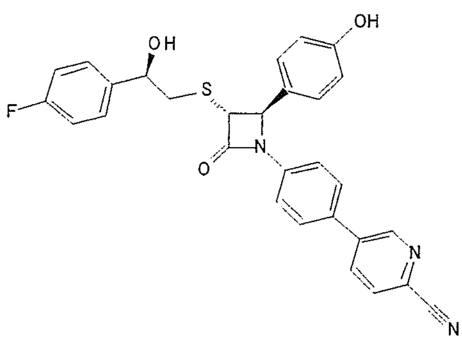 | (DMSO-d6) δ: 2.92-2.94 (m, 2H), 4.30-4.30 (m, 1H), 4.71-4.73 (m, 1H), 5.08 (br s, 1H), 5.67 (d, 1H, J = 4.4 Hz), 6.77-6.78 (m, 2H), 7.09-7.12 (m, 2H), 7.27-7.28 (m, 2H), 7.34-7.36 (m, 4H), 7.79-7.80 (m, 2H), 8.07 (d, 1H, J = 8.0 Hz), 8.25-8.27 (m, 1H), 9.02-9.04 (m, 1H), 9.62 (s, 1H). |
| 10 | 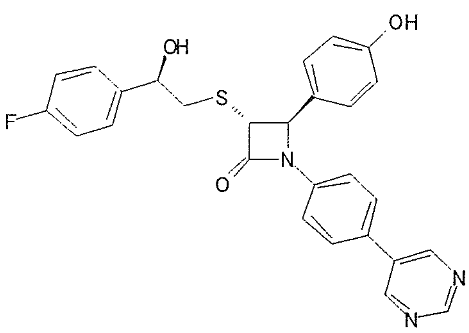 | (DMSO-d6) δ: 2.92-2.94 (m, 2H), 4.30 (d, 1H, J = 2.0 Hz), 4.70-4.76 (m, 1H), 5.07 (d, 1H, J = 2.2 Hz), 5.67 (d, 1H, J = 4.6 Hz), 6.77 (d, 2H, J = 8.3 Hz), 7.10-7.12 (m, 2H), 7.28 (d, 2H, J = 8.3 Hz), 7.34-7.36 (m, 4H), 7.76 (d, 2H, J = 8.5 Hz), 9.06 (s, 2H), 9.13 (s, 1H), 9.62 (s, 1H). |

(continued)

| Example No. | Structure | NMR data δ(ppm) |
|---|---|---|
| 11 | 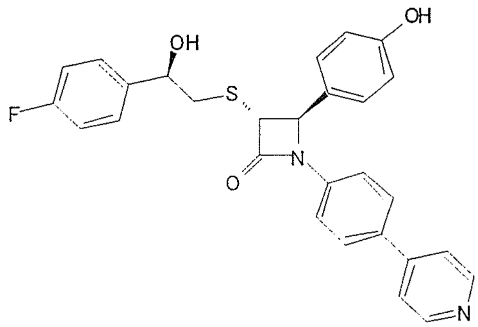 | (DMSO-d6) δ: 2.92-2.94 (m, 2H), 4.29 (d, 1H, J = 2.4 Hz), 4.71-4.74 (m, 1H), 5.07 (d, 1H, J = 2.2 Hz), 5.68 (d, 1H, J = 4.9 Hz), 6.77 (d, 2H, J = 8.5 Hz), 7.10-7.12 (m, 2H), 7.28 (d, 2H, J = 8.5 Hz), 7.32-7.37 (m, 4H), 7.62-7.63 (m, 2H), 7.77 (d, 2H, J = 8.5 Hz), 8.56-8.58 (m, 2H), 9.62 (s, 1H). |
| 12 | 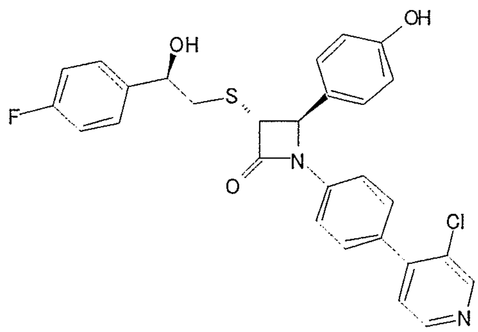 | (DMSO-d6) δ: 2.93-2.94 (m, 2H), 4.30-4.30 (m, 1H), 4.71-4.74 (m, 1H), 5.07 (d, 1H, J = 2.2 Hz), 5.67-5.68 (m, 1H), 6.78 (d, 2H, J = 8.0 Hz), 7.10-7.12 (m, 2H), 7.29-7.37 (m, 6H), 7.42 (d, 1H, J = 5.1 Hz), 7.49 (d, 2H, J = 8.0 Hz), 8.52-8.53 (m, 1H), 8.68 (s, 1H), 9.62 (br s, 1H). |
| 13 | 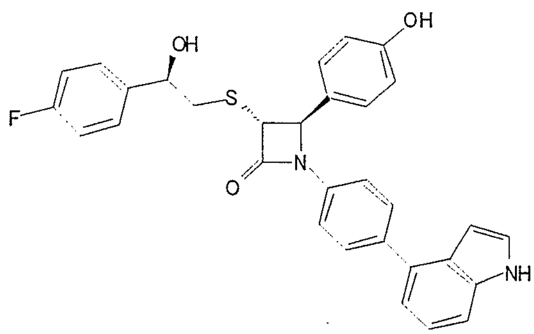 | (DMSO-d6) δ: 2.94-2.95 (m, 2H), 4.26 (d, 1H, J = 2.2 Hz), 4.72-4.75 (m, 1H), 5.04 (d, 1H, J = 2.4 Hz), 5.68 (d, 1H, J = 4.6 Hz), 6.48-6.48 (m, 1H), 6.78-6.79 (m, 2H), 6.99 (d, 1H, J = 7.3 Hz), 7.09-7.14 (m, 3H), 7.32-7.35 (m, 8H), 7.59 (d, 2H, J = 8.3 Hz), 9.61 (s, 1H), 11.24 (s, 1H). |

[Table 3]

| Example No. | Structure | NMR data δ(ppm) |
|---|---|---|
| 14 | 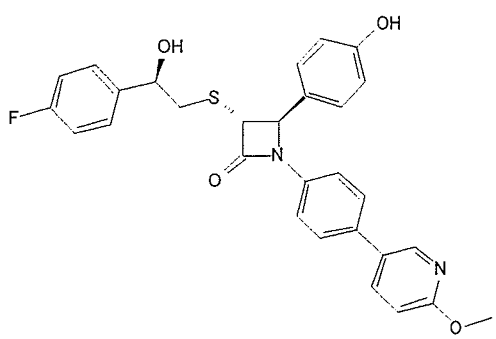 | (DMSO-d6) δ: 2.92-2.93 (m, 2H), 3.85 (s, 3H), 4.26 (d, 1H, J = 2.4 Hz), 4.71-4.74 (m, 1H), 5.03 (d, 1H, J = 2.4 Hz), 5.66 (d, 1H, J = 4.6 Hz), 6.77 (d, 2H, J = 8.5 Hz), 6.86 (d, 1H, J = 8.8 Hz), 7.10-7.12 (m, 2H), 7.27-7.29 (m, 4H), 7.33-7.37 (m, 2H), 7.59 (d, 2H, J = 8.5 Hz), 7.92 (dd, 1H, J = 8.7, 2.6 Hz), 8.40 (d, 1H, J = 2.7 Hz), 9.60 (s, 1H). |

(continued)

| Example No. | Structure | NMR data δ(ppm) |
|---|---|---|
| 15 | 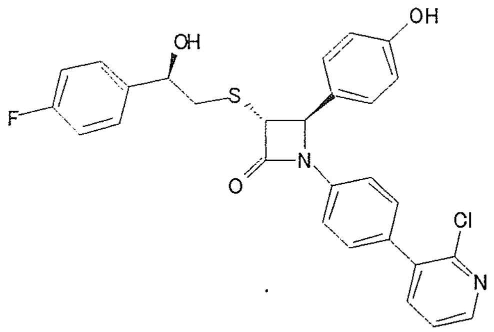 | (DMSO-d6) δ: 2.93-2.94 (m, 2H), 4.29 (d, 1H, J = 2.4 Hz), 4.71-4.74 (m, 1H), 5.06 (d, 1H, J = 2.2 Hz), 5.67 (d, 1H, J = 4.6 Hz), 6.77-6.79 (m, 2H), 7.10-7.12 (m, 2H), 7.31-7.35 (m, 6H), 7.42-7.49 (m, 3H), 7.81 (dd, 1H, J = 7.6, 2.0 Hz), 8.38-8.39 (m, 1H), 9.62 (s, 1H). |
| 16 | 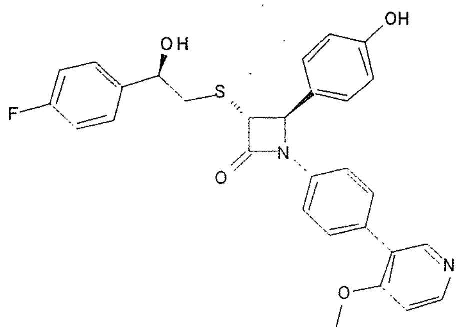 | (DMSO-d6) δ: 2.92-2.93 (m, 2H), 3.80 (s, 3H), 4.26 (d, 1H, J = 2.2 Hz), 4.71-4.74 (m, 1H), 5.03 (d, 1H, J = 2.2 Hz), 5.67 (d, 1H, J = 4.6 Hz), 6.77-6.79 (m, 2H), 7.09-7.13 (m, 3H), 7.26-7.29 (m, 4H), 7.34-7.36 (m, 2H), 7.43-7.45 (m, 2H), 8.29 (s, 1H), 8.40 (d, 1H, J = 5.6 Hz), 9.61 (s, 1H). |
| 17 | 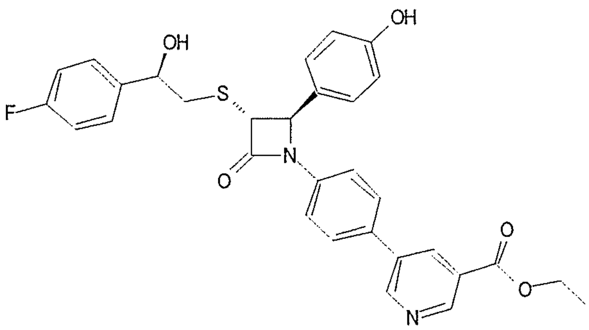 | (DMSO-d6) δ: 1.33 (t, 3H, J = 7.1 Hz), 2.93-2.94 (m, 2H), 4.29 (d, 1H, J = 2.4 Hz), 4.35-4.37 (m, 2H), 4.71-4.74 (m, 1H), 5.07 (d, 1H, J = 2.4 Hz), 5.67-5.68 (m, 1H), 6.77-6.79 (m, 2H), 7.10-7.13 (m, 2H), 7.28 (d, 2H, J = 8.5 Hz), 7.34-7.38 (m, 4H), 7.75 (d, 2H, J = 8.5 Hz), 8.39-8.40 (m, 1H), 9.03-9.05 (m, 2H), 9.61 (s, 1H). |
| 18 | 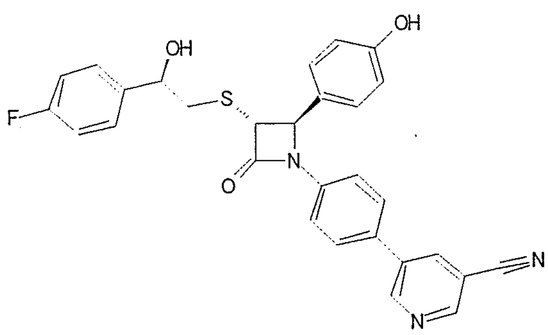 | (DMSO-d6) δ: 2.92-2.94 (m, 2H), 4.30 (d, 1H, J = 2.4 Hz), 4.71-4.74 (m, 1H), 5.08 (d, 1H, J = 2.2 Hz), 5.67-5.68 (m, 1H), 6.77 (d, 2H, J = 8.5 Hz), 7.11 (t, 2H, J = 8.8 Hz), 7.28 (d, 2H, J = 8.5 Hz), 7.33-7.37 (m, 4H), 7.78 (d, 2H, J = 8.5 Hz), 8.56-8.57 (m, 1H), 8.95-8.95 (m, 1H), 9.12-9.12 (m, 1H), 9.61 (s, 1H). |

[Table 4]

| Example No. | Structure | NMR data δ(ppm) |
|---|---|---|
| 19 | 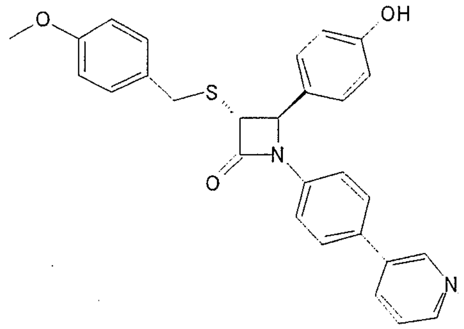 | (DMSO-d6) δ: 3.69 (s, 3H), 3.90 (s, 2H), 4.17 (d, 1H, J = 2.2 Hz), 5.00 (d, 1H, J = 2.2 Hz), 6.73-6.78 (m, 4H), 7.15-7.22 (m, 4H), 7.29 (d, 2H, J = 8.5 Hz), 7.41-7.45 (m, 1H), 7.68 (d, 2H, J = 8.5 Hz), 7.98-8.01 (m, 1H), 8.50-8.52 (m, 1H), 8.81-8.82 (m, 1H), 9.60 (s, 1H). |
| 20 | 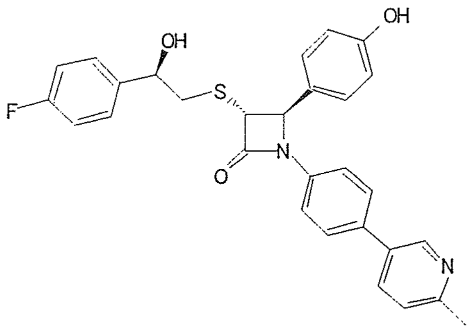 | (DMSO-d6) δ: 2.46 (s, 3H), 2.92-2.93 (m, 2H), 4.27 (d, 1H, J = 2.2 Hz), 4.71-4.73 (m, 1H), 5.03-5.05 (m, 1H), 5.66-5.67 (m, 1H), 6.77 (d, 2H, J = 8.5 Hz), 7.11 (t, 2H, J = 8.9 Hz), 7.28-7.35 (m, 7H), 7.64 (d, 2H, J = 8.5 Hz), 7.87 (dd, 1H, J = 8.0, 2.4 Hz), 8.67 (d, 1H, J = 2.4 Hz), 9.60 (s, 1H). |
| 21 | 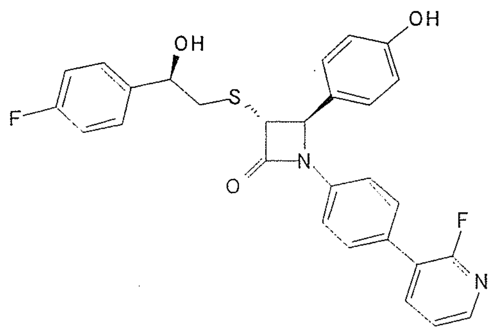 | (DMSO-d6) δ: 2.92-2.94 (m, 2H), 4.28 (d, 1H, J = 2.2 Hz), 4.71-4.74 (m, 1H), 5.06 (d, 1H, J = 2.4 Hz), 5.67-5.68 (m, 1H), 6.78 (d, 2H, J = 8.5 Hz), 7.11 (t, 2H, J = 8.9 Hz), 7.27-7.37 (m, 6H), 7.40-7.44 (m, 1H), 7.54-7.56 (m, 2H), 8.01-8.06 (m, 1H), 8.18-8.19 (m, 1H), 9.61 (s, 1H). |
| 22 | 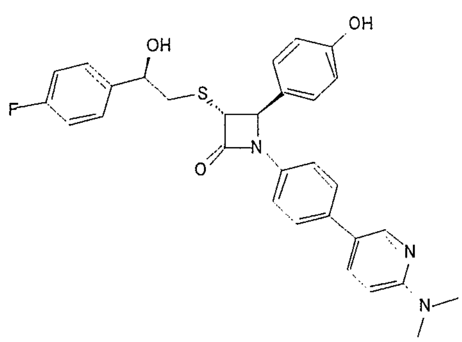 | (DMSO-d6) δ: 2.91-2.93 (m, 2H), 3.02-3.02 (m, 6H), 4.22-4.25 (m, 1H), 4.71-4.72 (m, 1H), 4.99-5.02 (m, 1H), 5.66-5.67 (m, 1H), 6.65-6.67 (m, 1H), 6.76-6.77 (m, 2H), 7.09-7.12 (m, 2H), 7.24-7.26 (m, 4H), 7.34-7.36 (m, 2H), 7.51-7.53 (m, 2H), 7.72-7.74 (m, 1H), 8.34 (brs, 1H), 9.59-9.60 (m, 1H). |

(continued)

| Example No. | Structure | NMR data δ(ppm) |
| --- | --- | --- |
| 23 | 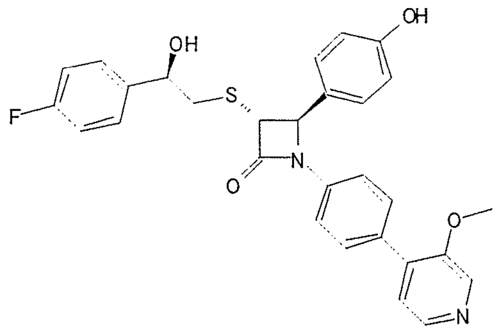 | (DMSO-d6) δ: 2.92-2.93 (m, 2H), 3.84 (br s, 3H), 4.27 (d, 1H, J = 2.2 Hz), 4.71-4.74 (m, 1H), 5.04 (d, 1H, J = 2.2 Hz), 5.67-5.68 (m, 1H), 6.78 (d, 2H, J = 8.3 Hz), 7.11 (t, 2H, J = 8.8 Hz), 7.28-7.29 (m, 5H), 7.34-7.36 (m, 2H), 7.52 (d, 2H, J = 8.5 Hz), 8.22 (d, 1H, J = 4.9 Hz), 8.41 (s, 1H), 9.62 (s, 1H). |

[Table 5]

| Example No. | Structure | NMR data δ(ppm) |
| --- | --- | --- |
| 24 | 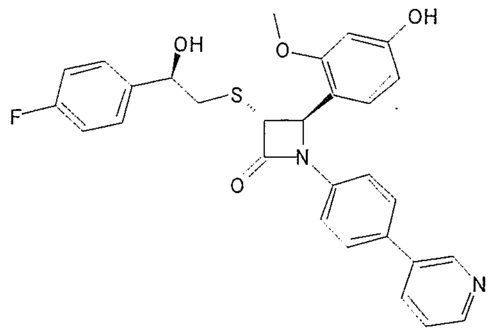 | (DMSO-d6) δ: 2.89-2.99 (m, 2H), 3.73 (s, 3H), 4.28 (d, 1H, J = 2.0 Hz), 4.68-4.73 (m, 1H), 5.10 (d, 1H, J = 2.0 Hz), 5.65 (d, 1H, J = 4.6 Hz), 6.35 (d, 1H, J = 8.3 Hz), 6.45 (s, 1H), 7.07-7.11 (m, 3H), 7.28-7.44 (m, 5H), 7.67 (d, 2H, J = 8.3 Hz), 7.98-8.01 (m, 1H), 8.50-8.51 (m, 1H), 8.81-8.82 (m, 1H), 9.68 (s, 1H). |
| 25 | 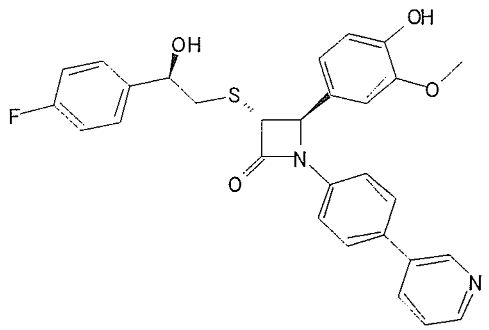 | (DMSO-d6) δ: 2.92-2.94 (m, 2H), 3.74 (s, 3H), 4.34 (d, 1H, J = 2.2 Hz), 4.70-4.74 (m, 1H), 5.02 (d, 1H, J = 2.0 Hz), 5.68 (d, 1H, J = 4.6 Hz), 6.77 (d, 1H, J = 8.0 Hz), 6.89 (d, 1H, J = 8.0 Hz), 7.03-7.13 (m, 3H), 7.33-7.44 (m, 5H), 7.68 (d, 2H, J = 8.5 Hz), 7.98-8.01 (m, 1H), 8.50-8.52 (m, 1H), 8.82 (s, 1H), 9.18 (s, 1H). |
| 26 | 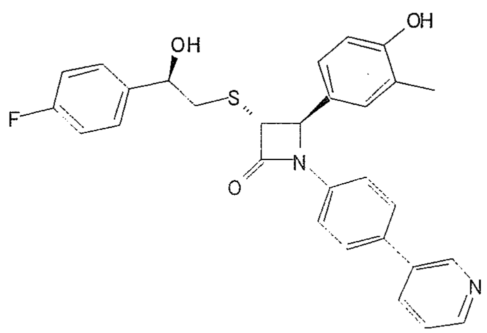 | (DMSO-d6) δ6: 2.09 (s, 3H), 2.91-2.93 (m, 2H), 4.25 (d, 1H, J = 2.0 Hz), 4.69-4.73 (m, 1H), 4.99 (d, 1H, J = 2.2 Hz), 5.67 (d, 1H, J = 4.6 Hz), 6.77 (d, 1H, J = 8.3 Hz), 7.08-7.16 (m, 4H), 7.31-7.44 (m, 5H), 7.68 (d, 2H, J = 8.5 Hz), 7.98-8.00 (m, 1H), 8.50-8.51 (m, 1H), 8.81-8.82 (m, 1H), 9.52 (s, 1H). |

(continued)

| Example No. | Structure | NMR data δ(ppm) |
|---|---|---|
| 27 | 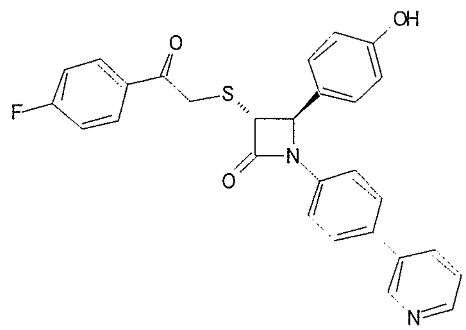 | (DMSO-d6) δ: 4.33 (d, 1H, J = 2.4 Hz), 4.38 (d, 2H, J = 5.6 Hz), 5.18 (d, 1H, J = 2.4 Hz), 6.74-6.77 (m, 2H), 7.26-7.40 (m, 6H), 7.45-7.47 (m, 1H), 7.68-7.70 (m, 2H), 8.02-8.06 (m, 3H), 8.53 (d, 1H, J = 4.8 Hz), 8.84-8.84 (m, 1H), 9.64 (s, 1H). |
| 28 | 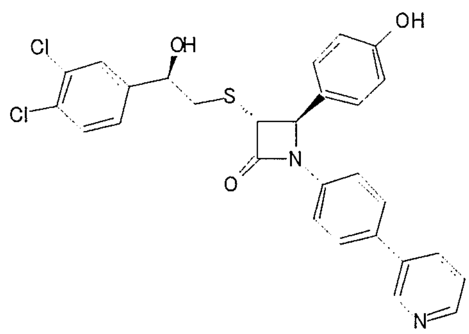 | (DMSO-d6) δ: 2.93-3.03 (m, 2H), 4.34 (d, 1H, J = 2.4 Hz), 4.78-4.79 (m, 1H), 5.07 (d, 1H, J = 2.4 Hz), 5.92 (d, 1H, J = 4.9 Hz), 6.68-6.83 (m, 2H), 7.25-7.33 (m, 5H), 7.43-7.45 (m, 1H), 7.56-7.59 (m, 2H), 7.66-7.70 (m, 2H), 8.01-8.05 (m, 1H), 8.52-8.55 (m, 1H), 8.83-8.91 (m, 1H), 9.62 (s, 1H). |

[Table 6]

| Example No. | Structure | NMR data δ(ppm) |
|---|---|---|
| 29 | 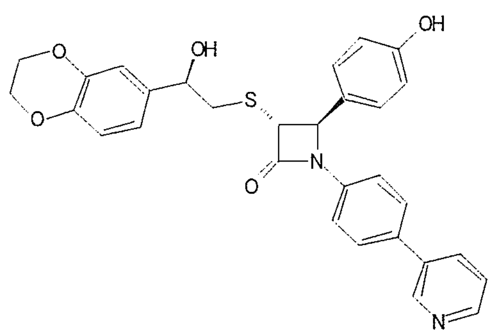 | (DMSO-d6) δ: 2.87-2.89 (m, 2H), 4.19 (s, 4H), 4.28 (d, 1H, J = 2.2 Hz), 4.54-4.61 (m, 1H), 5.03 (d, 1H, J = 2.4 Hz), 5.50 (d, 1H, J = 4.6 Hz), 6.76-6.80 (m, 5H), 7.28-7.32 (m, 4H), 7.42-7.44 (m, 1H), 7.67 (d, 2H, J = 8.5 Hz), 7.98-8.00 (m, 1H), 8.50-8.51 (m, 1H), 8.81 (d, 1H, J = 2.4 Hz), 9.59 (s, 1H). |
| 30 | 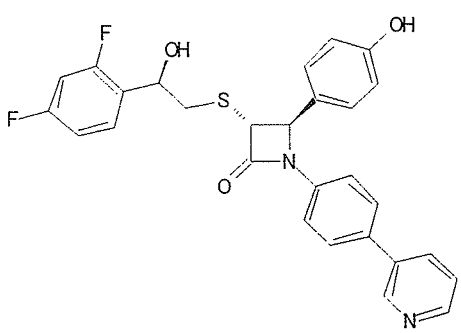 | (DMSO-d6) δ: 2.99-3.01 (m, 2H), 4.29 (d, 1H, J = 2.4 Hz), 4.95-5.04 (m, 2H), 5.82 (d, 1H, J = 4.9 Hz), 6.78-6.81 (m, 2H), 7.01-7.22 (m, 2H), 7.29-7.33 (m, 4H), 7.42-7.58 (m, 2H), 7.68-7.75 (m, 2H), 7.99-8.01 (m, 1H), 8.52-8.53 (m, 1H), 8.82-8.83 (m, 1H), 9.68 (s, 1H). |

(continued)

| Example No. | Structure | NMR data δ(ppm) |
|---|---|---|
| 31 | 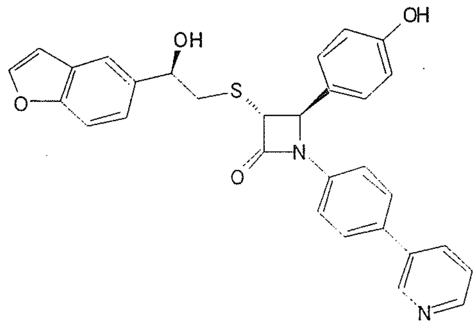 | (DMSO-d6) δ: 2.98-3.02 (m, 2H), 4.33 (d, 1H, J = 2.0 Hz), 4.82-4.85 (m, 1H), 4.99 (d, 1H, J = 2.0 Hz), 5.50-5.68 (m, 1H), 6.73-6.81 (m, 2H), 6.90-6.94 (m, 1H), 7.22-7.36 (m, 5H), 7.43-7.45 (m, 1H), 7.50-7.52 (m, 1H), 7.58-7.59 (m, 1H), 7.69-7.77 (m, 2H), 7.87-8.04 (m, 2H), 8.52-8.53 (m, 1H), 8.83 (d, 1H, J = 2.0 Hz), 9.63 (s, 1H). |
| 32 | 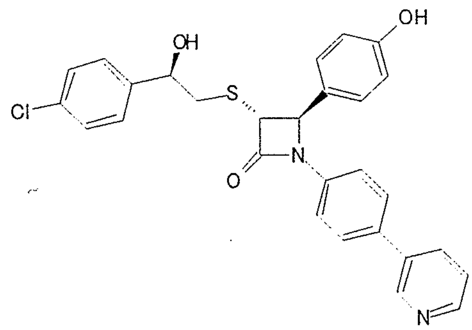 | (DMSO-d6) δ: 2.94 (d, 2H, J = 6.4 Hz), 4.29 (d, 1H, J = 2.4 Hz), 4.71-4.77 (m, 1H), 5.05 (d, 1H, J = 2.4 Hz), 5.72 (d, 1H, J = 4.8 Hz), 6.79 (d, 2H, J = 8.4 Hz), 7.26-7.35 (m, 4H), 7.35-7.37 (m, 4H), 7.41-7.47 (m, 1H), 7.66-7.71 (m, 2H), 7.98-8.02 (m, 1H), 8.52 (dd, 1H, J = 4.8, 1.7 Hz), 8.83 (dd, 1H, J = 2.3, 1.3 Hz), 9.62 (br s, 1H). |
| 33 | 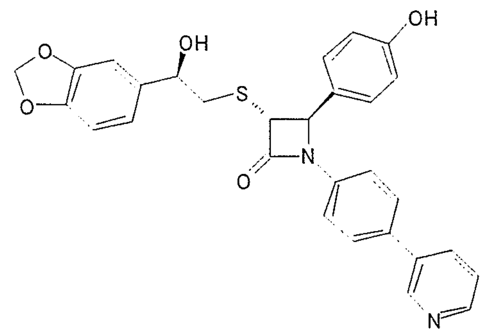 | (DMSO-d6) δ6: 2.88-2.90 (m, 2H), 4.27 (d, 1H, J = 2.4 Hz), 4.62 (dd, 1H, J = 11.5, 6.3 Hz), 5.04 (d, 1H, J = 2.2 Hz), 5.56 (d, 1H, J = 4.6 Hz), 5.95-5.97 (m, 2H), 6.76-6.87 (m, 5H), 7.26-7.33 (m, 4H), 7.43 (dd, 1H, J = 8.0, 4.9 Hz), 7.68 (d, 2H, J = 8.5 Hz), 7.99 (dt, 1H, J = 8.0, 2.0 Hz), 8.51 (dd, 1H, J = 4.8, 1.6 Hz), 8.81 (d, 1H, J = 2.4 Hz), 9.59 (s, 1H). |

[Table 7]

| Example No. | Structure | NMR data δ(ppm) |
|---|---|---|
| 34 | 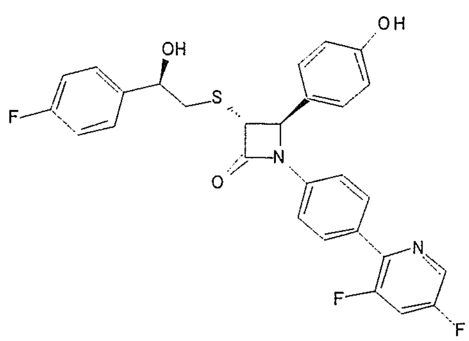 | (DMSO-d6) δ: 2.92-2.94 (m, 2H), 4.28 (d, 1H, J = 2.4 Hz), 4.71-4.74 (m, 1H), 5.06 (d, 1H, J= 2.4 Hz), 5.65-5.66 (m, 1H), 6.74-6.79 (m, 2H), 7.09-7.12 (m, 2H), 7.26-7.29 (m, 2H), 7.33-7.36 (m, 4H), 7.79-7.81 (m, 2H), 7.99-8.04 (m, 1H), 8.58 (d, 1H, J = 2.4 Hz), 9.60 (s, 1H). |

(continued)

| Example No. | Structure | NMR data δ(ppm) |
|---|---|---|
| 35 | 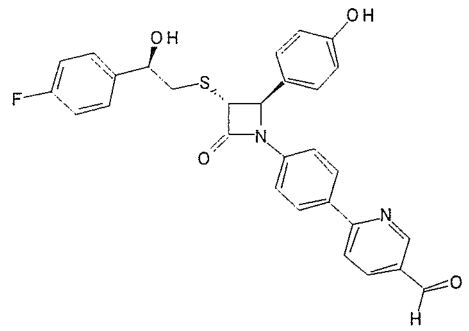 | (DMSO-d6) δ: 2.93-2.95 (m, 2H), 4.30-4.30 (m, 1H), 4.72-4.74 (m, 1H), 5.08 (d, 1H, J = 2.2 Hz), 5.66-5.67 (m, 1H), 6.78 (d, 2H, J = 8.5 Hz), 7.11 (t, 2H, J = 8.7 Hz), 7.28 (d, 2H, J = 8.5 Hz), 7.35-7.36 (m, 4H), 8.10-8.15 (m, 3H), 8.24-8.26 (m, 1H), 9.09-9.09 (m, 1H), 9.60 (s, 1H), 10.09 (s, 1H). |
| 36 | 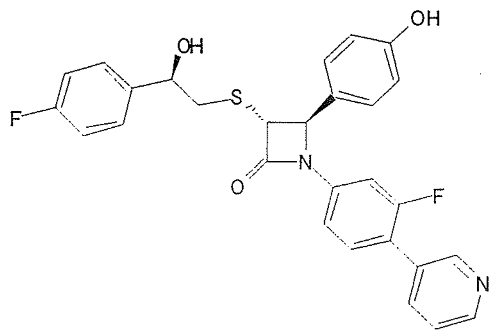 | (DMSO-d6) δ: 2.91-2.93 (m, 2H), 4.33 (d, 1H, J = 2.7 Hz), 4.71-4.74 (m, 1H), 5.09 (d, 1H, J = 2.4 Hz), 5.67-5.68 (m, 1H), 6.78-6.80 (m, 2H), 7.11-7.15 (m, 4H), 7.29-7.38 (m, 4H), 7.45-7.47 (m, 1H), 7.53-7.59 (m, 1H), 7.88-7.91 (m, 1H), 8.54-8.56 (m, 1H), 8.67 (s, 1H), 9.63 (s, 1H). |
| 37 | 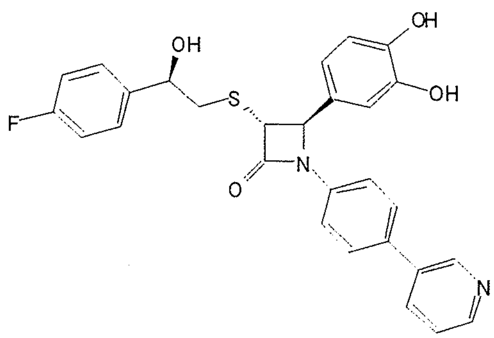 | (DMSO-d6) δ: 2.92-2.94 (m, 2H), 4.24 (d, 1H, J = 2.0 Hz), 4.70-4.74 (m, 1H), 4.98 (d, 1H, J = 2.0 Hz), 5.67 (d, 1H, J = 4.6 Hz), 6.74-6.77 (m, 3H), 7.09-7.13 (m, 2H), 7.32-7.45 (m, 5H), 7.69 (d, 2H, J = 8.5 Hz), 7.99-8.01 (m, 1H), 8.50-8.52 (m, 1H), 8.81-8.83 (m, 1H), 9.07 (s, 1H), 9.10 (s, 1H). |
| 38 | 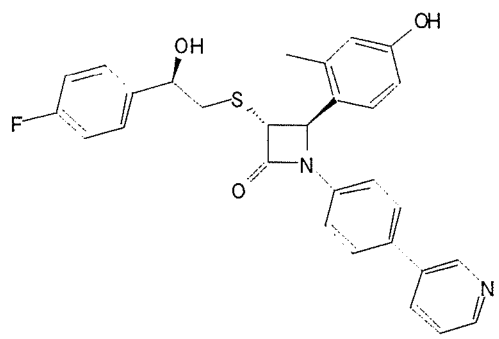 | (DMSO-d6) δ: 2.37 (s, 3H), 2.92-3.00 (m, 2H), 4.16 (d, 1H, J = 2.0 Hz), 4.77 (dd, 1H, J = 11.3, 6.0 Hz), 5.26 (d, 1H, J = 2.0 Hz), 5.67 (d, 1H, J = 4.6 Hz), 6.53 (d, 1H, J = 8.5 Hz), 6.64 (s, 1H), 6.91 (d, 1H, J = 8.3 Hz), 7.11 (t, 2H, J = 8.8 Hz), 7.31-7.46 (m, 5H), 7.71 (d, 2H, J = 8.3 Hz), 8.00-8.02 (m, 1H), 8.51-8.52 (m, 1H), 8.83 (s, 1H), 9.44 (s, 1H). |

[Table 8]

| Example No. | Structure | NMR data δ(ppm) |
|---|---|---|
| 39 | 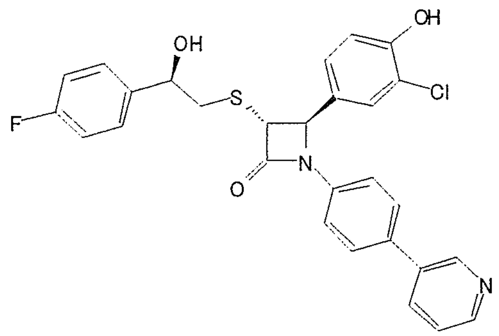 | (DMSO-d6) δ: 2.89-3.06 (m, 2H), 4.34 (d, 1H, J = 2.4 Hz), 4.70-4.73 (m, 1H), 5.10 (d, 1H, J = 2.2 Hz), 5.67 (d, 1H, J = 4.6 Hz), 5.75 (s, 1H), 6.96-7.71 (m, 11H), 7.98-8.01 (m, 1H), 8.51-8.52 (m, 1H), 8.82-8.82 (m, 1H), 10.40 (s, 1H). |
| 40 | 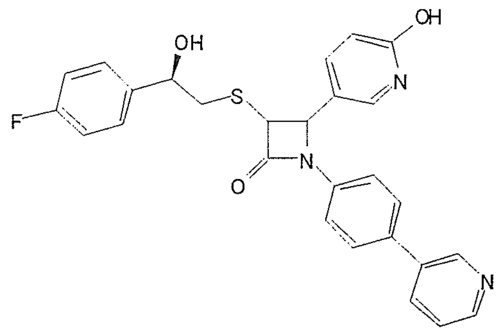 | (DMSO-d6) δ: 2.85-2.96 (m, 2H), 4.42-4.45 (m, 1H), 4.72-4.78 (m, 1H), 5.02-5.04 (m, 1H), 5.67-5.69 (m, 1H), 6.33 (d, 1H, J = 9.5 Hz), 7.09-7.14 (m, 2H), 7.34-7.49 (m, 6H), 7.71-7.73 (m, 3H), 7.99-8.02 (m, 1H), 8.51-8.53 (m, 1H), 8.83-8.84 (m, 1H), 11.75 (s, 1H). |
| 41 | 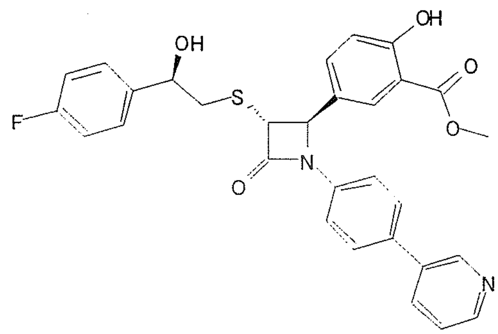 | (DMSO-d6) δ: 2.91-2.94 (m, 2H), 3.89 (s, 3H), 4.33 (d, 1H, J = 2.4 Hz), 4.71-4.76 (m, 1H), 5.21 (d, 1H, J = 2.2 Hz), 5.66 (d, 1H, J = 4.6 Hz), 7.01-7.12 (m, 3H), 7.32-7.44 (m, 5H), 7.59 (dd, 1H, J = 8.5, 2.4 Hz), 7.69 (d, 2H, J = 8.8 Hz), 7.91 (d, 1H, J = 2.4 Hz), 7.99 (dt, 1H, J = 8.2, 2.0 Hz), 8.51 (dd, 1H, J = 4.8, 1.6 Hz), 8.81 (d, 1H, J = 1.7 Hz), 10.56 (s, 1H). |
| 42 | 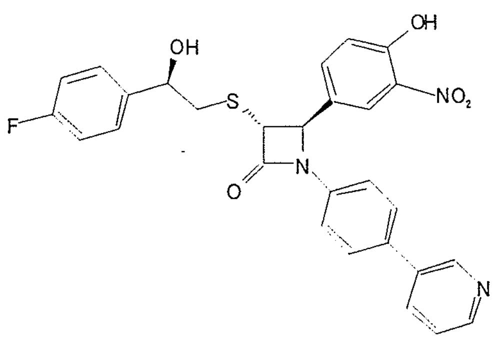 | (DMSO-d6) δ: 2.89-2.95 (m, 2H), 4.39 (d, 1H, J = 2.2 Hz), 4.72-4.76 (m, 1H), 5.26 (d, 1H, J = 2.4 Hz), 5.67 (d, 1H, J = 4.6 Hz), 7.08-7.16 (m, 3H), 7.33-7.45 (m, 5H), 7.61-7.63 (m, 1H), 7.70 (d, 2H, J = 8.5 Hz), 7.99-8.01 (m, 1H), 8.09-8.09 (m, 1H), 8.51-8.52 (m, 1H), 8.82-8.82 (m, 1H), 11.21 (s, 1H). |

(continued)

| Example No. | Structure | NMR data δ(ppm) |
|---|---|---|
| 43 | 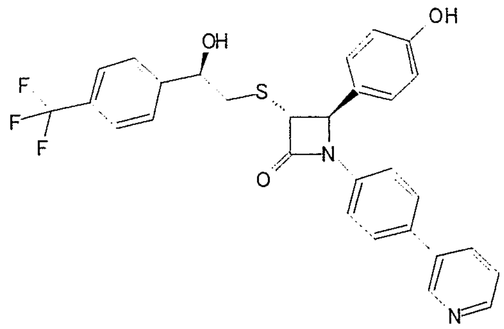 | (DMSO-d6) δ: 2.95-3.00 (m, 2H), 4.30 (d, 1H, J = 2.2 Hz), 4.84-4.85 (m, 1H), 5.07 (d, 1H, J = 2.2 Hz), 5.85 (d, 1H, J = 4.6 Hz), 6.78-6.80 (m, 2H), 7.29-7.34 (m, 4H), 7.43-7.45 (m, 1H), 7.58-7.67 (m, 6H), 7.99-8.01 (m, 1H), 8.52 (dd, 1H, J = 4.6, 1.5 Hz), 8.83 (d, 1H, J = 1.5 Hz), 9.61 (s, 1H). |

[Table 9]

| Example No. | Structure | NMR data δ(ppm) |
|---|---|---|
| 44 | 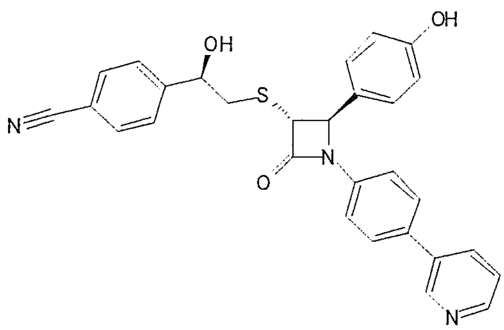 | (DMSO-d6) δ: 2.96-2.97 (m, 2H), 4.29 (d, 1H, J = 2.4 Hz), 4.83 (s, 1H), 5.06 (d, 1H, J = 2.2 Hz), 5.88 (s, 1H), 6.77-6.79 (m, 2H), 7.28-7.32 (m, 4H), 7.42-7.44 (m, 1H), 7.52-7.54 (m, 2H), 7.67-7.77 (m, 4H), 7.98-8.00 (m, 1H), 8.51-8.52 (m, 1H), 8.81 (d, 1H, J = 1.5 Hz), 9.62 (s, 1H). |
| 45 | 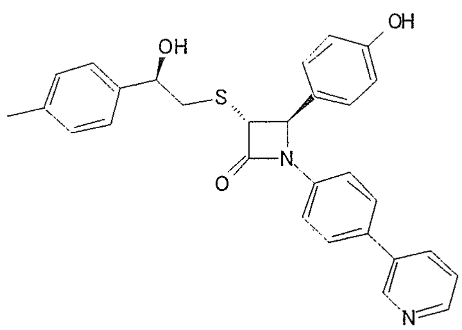 | (DMSO-d6) δ: 2.25 (s, 3H), 2.90 (d, 2H, J = 6.6 Hz), 4.24 (d, 1H, J = 2.0 Hz), 4.64-4.67 (m, 1H), 5.01 (d, 1H, J = 2.0 Hz), 5.54 (d, 1H, J = 4.6 Hz), 6.77 (d, 2H, J = 8.2 Hz), 7.08 (d, 2H, J = 7.8 Hz), 7.15-7.36 (m, 6H), 7.42-7.44 (m, 1H), 7.66-7.69 (m, 2H), 7.99 (d, 1H, J = 8.2 Hz), 8.51 (d, 1H, J = 4.7 Hz), 8.81 (d, 1H, J = 2.0 Hz), 9.60 (s, 1H). |
| 46 | 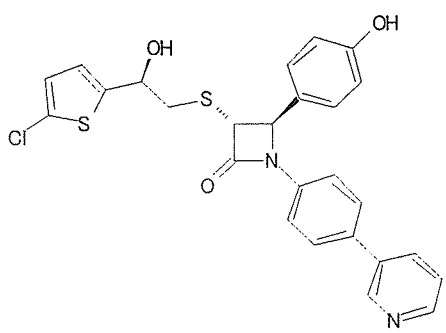 | (DMSO-d6) δ: 2.99 (d, 2H, J = 6.3 Hz), 4.33 (d, 1H, J = 2.3 Hz), 4.89 (q, 1H, J = 5.8 Hz), 5.07 (d, 1H, J = 2.3 Hz), 6.19 (d, 1H, J = 4.9 Hz), 6.76-6.78 (m, 2H), 6.84-6.85 (m, 1H), 6.95 (d, 1H, J = 3.9 Hz), 7.29-7.33 (m, 4H), 7.42-7.44 (m, 1H), 7.67-7.69 (m, 2H), 7.98-8.00 (m, 1H), 8.50-8.52 (m, 1H), 8.82 (d, 1H, J = 2.0 Hz), 9.60 (s, 1H). |

(continued)

| Example No. | Structure | NMR data δ(ppm) |
|---|---|---|
| 47 | 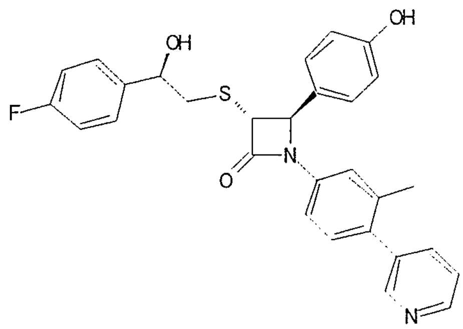 | (DMSO-d6) δ: 2.14 (s, 3H), 2.86-2.96 (m, 2H), 4.25 (d, 1H, J = 2.4 Hz), 4.71-4.74 (m, 1H), 5.03 (d, 1H, J = 2.4 Hz), 5.65-5.71 (m, 1H), 6.78 (d, 2H, J = 8.5 Hz), 7.04-7.06 (m, 1H), 7.09-7.18 (m, 3H), 7.27-7.29 (m, 3H), 7.33-7.38 (m, 2H), 7.41-7.43 (m, 1H), 7.71-7.73 (m, 1H), 8.48-8.49 (m, 1H), 8.52-8.53 (m, 1H), 9.61 (s, 1H). |
| 48 | 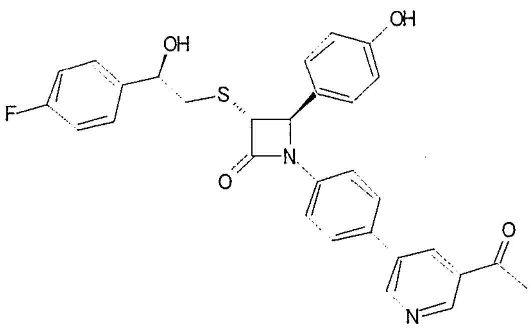 | (DMSO-d6) δ: 2.66 (s, 3H), 2.93-2.94 (m, 2H), 4.29 (d, 1H, J = 2.2 Hz), 4.71-4.74 (m, 1H), 5.07 (d, 1H, J = 2.2 Hz), 5.66-5.67 (m, 1H), 6.77-6.79 (m, 2H), 7.10-7.12 (m, 2H), 7.29-7.35 (m, 6H), 7.76-7.78 (m, 2H), 8.41-8.41 (m, 1H), 9.03-9.04 (m, 2H), 9.60 (s, 1H). |

[Table 10]

| Example No. | Structure | NMR data δ(ppm) |
|---|---|---|
| 49 | 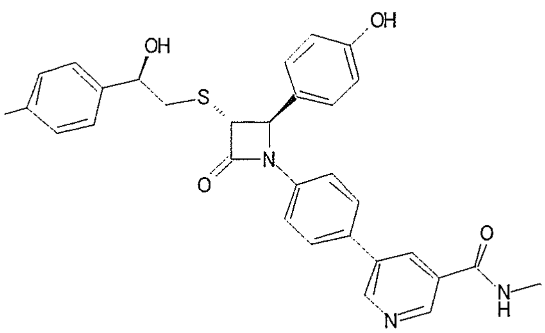 | (DMSO-d6) δ: 1.14-1.16 (m, 3H), 2.93-2.95 (m, 2H), 3.27-3.32 (m, 2H), 4.29 (d, 1H, J = 2.2 Hz), 4.72-4.74 (m, 1H), 5.06 (d, 1H, J = 2.2 Hz), 5.66 (d, 1H, J = 4.6 Hz), 6.78 (d, 2H, J = 8.3 Hz), 7.10-7.12 (m, 2H), 7.28 (d, 2H, J = 8.5 Hz), 7.34-7.37 (m, 4H), 7.74 (d, 2H, J = 8.5 Hz), 8.34-8.35 (m, 1H), 8.67-8.69 (m, 1H), 8.90-8.93 (m, 2H), 9.60 (s, 1H). |
| 50 | 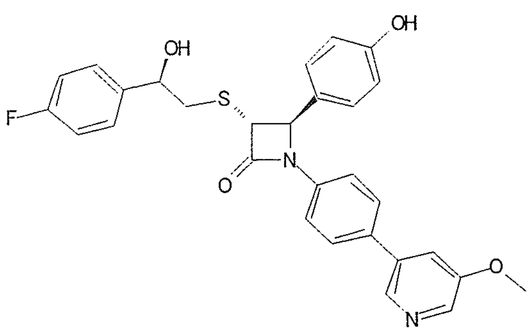 | (DMSO-d6) δ: 2.92-2.94 (m, 2H), 3.86 (s, 3H), 4.28-4.29 (m, 1H), 4.71-4.74 (m, 1H), 5.05-5.07 (m, 1H), 5.67-5.68 (m, 1H), 6.77 (d, 2H, J = 8.1 Hz), 7.11 (t, 2H, J = 8.5 Hz), 7.29-7.35 (m, 6H), 7.54-7.55 (m, 1H), 7.69-7.71 (m, 2H), 8.22-8.23 (m, 1H), 8.40-8.42 (m, 1H), 9.62 (s, 1H). |

(continued)

| Example No. | Structure | NMR data δ(ppm) |
|---|---|---|
| 51 | 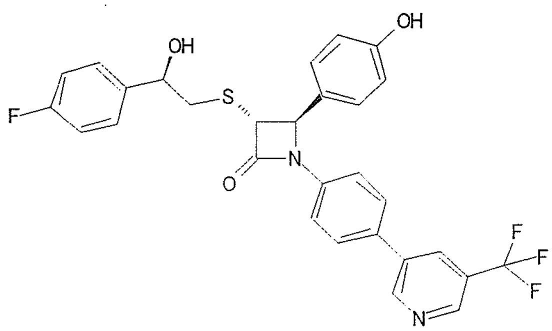 | (DMSO-d6) δ: 2.93-2.94 (m, 2H), 4.30 (d, 1H, J = 2.4 Hz), 4.71-4.74 (m, 1H), 5.08 (d, 1H, J = 2.4 Hz), 5.68 (d, 1H, J = 4.6 Hz), 6.77-6.79 (m, 2H), 7.10-7.12 (m, 2H), 7.28-7.29 (m, 2H), 7.33-7.37 (m, 4H), 7.79-7.82 (m, 2H), 8.37-8.40 (m, 1H), 8.90-8.90 (m, 1H), 9.13-9.14 (m, 1H), 9.62 (s, 1H). |
| 52 | 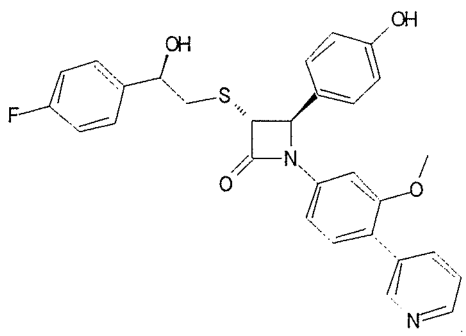 | (DMSO-d6)δ: 2.93-2.94 (m, 2H), 3.66 (s, 3H), 4.31 (d, 1H, J = 2.2 Hz), 4.73 (dd, 1H, J = 11.2, 6.1 Hz), 5.05 (d, 1H, J = 2.2 Hz), 5.68 (d, 1H, J = 4.6 Hz), 6.77-6.79 (m, 3H), 7.09-7.14 (m, 3H), 7.26-7.39 (m, 6H), 7.79-7.81 (m, 1H), 8.45-8.47 (m, 1H), 8.58-8.59 (m, 1H), 9.62 (s, 1H). |
| 53 | 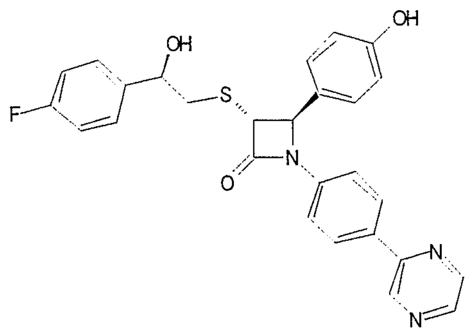 | (DMSO-d6) δ: 2.93-2.95 (m, 2H), 4.29 (d, 1H, J = 2.2 Hz), 4.71-4.74 (m, 1H), 5.08 (d, 1H, J = 2.2 Hz), 5.67 (d, 1H, J = 4.6 Hz), 6.76-6.79 (m, 2H), 7.10-7.12 (m, 2H), 7.27-7.29 (m, 2H), 7.34-7.36 (m, 4H), 8.07-8.09 (m, 2H), 8.54-8.55 (m, 1H), 8.65-8.65 (m, 1H), 9.15-9.17 (m, 1H), 9.60 (s, 1H). |

[Table 11]

| Example No. | Structure | NMR data δ(ppm) |
|---|---|---|
| 54 | 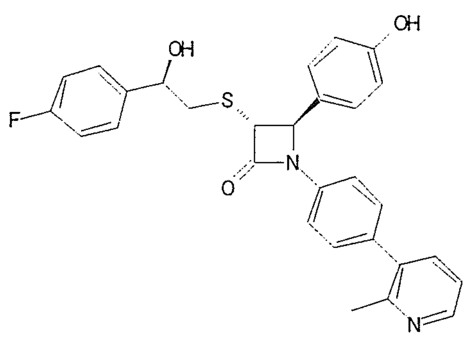 | (DMSO-d6) δ: 2.37 (s, 3H), 2.93-2.94 (m, 2H), 4.28 (d, 1H, J = 2.4 Hz), 4.71-4.74 (m, 1H), 5.04 (d, 1H, J = 2.4 Hz), 5.67 (d, 1H, J = 4.6 Hz), 6.77-6.79 (m, 2H), 7.10-7.12 (m, 2H), 7.23-7.25 (m, 1H), 7.31-7.35 (m, 8H), 7.52 (dd, 1H, J = 7.7, 1.6 Hz), 8.41 (dd, 1H, J = 4.9, 1.7 Hz), 9.61 (s, 1H). |

(continued)

| Example No. | Structure | NMR data δ(ppm) |
|---|---|---|
| 55 | 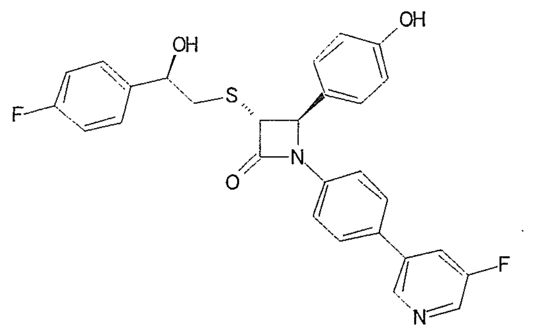 | (DMSO-d6) δ: 2.92-2.94 (m, 2H), 4.29 (d, 1H, J = 2.2 Hz), 4.71-4.74 (m, 1H), 5.06 (d, 1H, J = 2.2 Hz), 5.67 (d, 1H, J = 4.6 Hz), 6.76-6.78 (m, 2H), 7.10-7.12 (m, 2H), 7.27-7.37 (m, 6H), 7.73-7.75 (m, 2H), 7.97-8.00 (m, 1H), 8.51 (d, 1H, J = 2.7 Hz), 8.72-8.74 (m, 1H), 9.61 (s, 1H). |
| 56 | 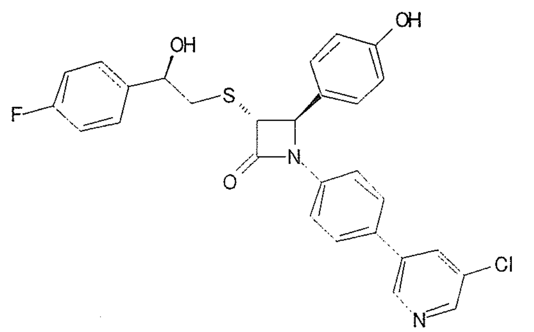 | (DMSO-d6) δ: 2.93-2.94 (m, 2H), 4.28 (d, 1H, J = 2.4 Hz), 4.71-4.74 (m, 1H), 5.06 (d, 1H, J = 2.2 Hz), 5.66 (d, 1H, J = 4.6 Hz), 6.76-6.78 (m, 2H), 7.10-7.12 (m, 2H), 7.27-7.37 (m, 6H), 7.73-7.75 (m, 2H), 8.16-8.16 (m, 1H), 8.55-8.56 (m, 1H), 8.79-8.79 (m, 1H), 9.60 (s, 1H). |
| 57 | 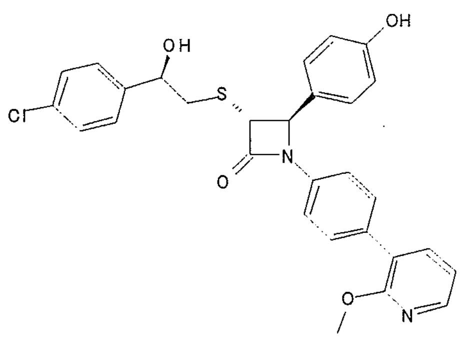 | (DMSO-d6) δ: 2.94 (d, 2H, J = 6.6 Hz), 3.84 (s, 3H), 4.27 (d, 1H, J = 2.5 Hz), 4.70-4.78 (m, 1H), 5.02 (d, 1H, J = 2.5 Hz), 5.72 (d, 1H, J = 4.8 Hz), 6.76-6.81 (m, 2H), 7.03-7.08 (m, 1H), 7.25-7.31 (m, 4H), 7.34-7.37 (m, 4H), 7.47-7.52 (m, 2H), 7.66-7.71 (m, 1H), 8.12-8.15 (m, 1H), 9.61 (br s, 1H). |
| 58 | 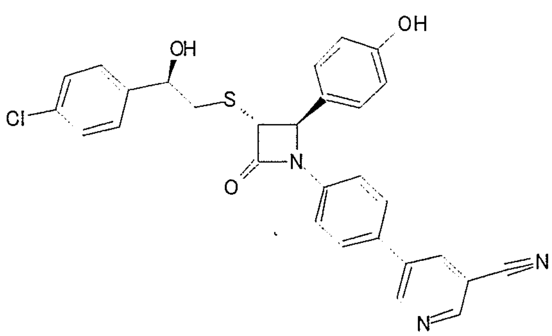 | (DMSO-d6) δ: 2.94 (d, 2H, J = 6.4 Hz), 4.31 (d, 1H, J = 2.4 Hz), 4.71-4.77 (m, 1H), 5.07 (d, 1H, J = 2.5 Hz), 5.72 (d, 1H, J = 4.8 Hz), 6.76-6.81 (m, 2H), 7.26-7.30 (m, 2H), 7.33-7.37 (m, 6H), 7.77-7.81 (m, 2H), 8.56-8.59 (m, 1H), 8.96 (d, 1H, J = 2.0 Hz), 9.13 (d, 1H, J = 2.0 Hz), 9.62 (br s, 1H). |

[Table 12]

| Example No. | Structure | NMR data δ(ppm) |
|---|---|---|
| 59 | 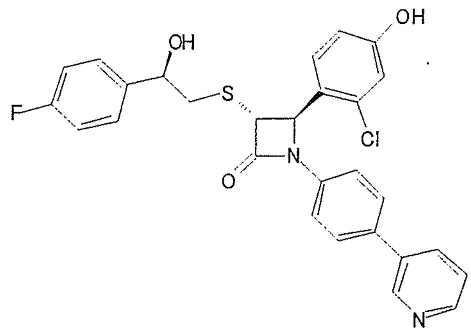 | (DMSO-d6) δ: 2.99-3.09 (m, 2H), 4.41 (d, 1H, J = 2.2 Hz), 4.79-4.83 (m, 1H), 5.38 (d, 1H, J = 2.2 Hz), 5.72 (d, 1H, J = 4.6 Hz), 6.82 (dd, 1H, J = 8.5, 2.4 Hz), 6.96 (d, 1H, J = 2.4 Hz), 7.16 (t, 2H, J = 8.9 Hz), 7.27-7.52 (m, 6H), 7.75-7.81 (m, 2H), 8.05-8.08 (m, 1H), 8.58-8.59 (m, 1H), 8.90 (s, 1H), 10.19 (s, 1H). |
| 60 | 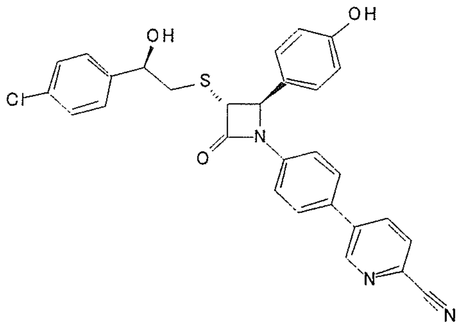 | (DMSO-d6) δ: 2.93 (d, 2H, J = 6.5 Hz), 4.30 (d, 1H, J = 2.6 Hz), 4.70-4.76 (m, 1H), 5.07 (d, 1H, J = 2.6 Hz), 5.71 (d, 1H, J = 4.1 Hz), 6.74-6.80 (m, 2H), 7.25-7.30 (m, 2H), 7.32-7.38 (m, 6H), 7.78-7.82 (m, 2H), 8.06-8.08 (m, 1H), 8.24-8.29 (m, 1H), 9.03-9.03 (m, 1H), 9.61 (br s, 1H). |
| 61 | 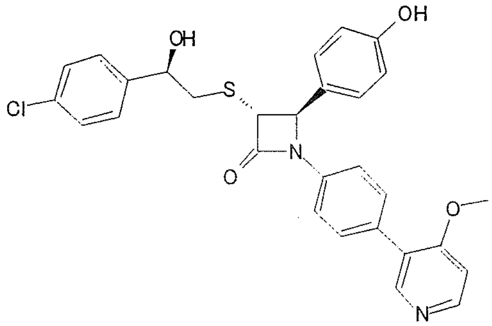 | (DMSO-d6) δ: 2.93 (d, 2H, J = 6.4 Hz), 3.80 (s, 3H), 4.26 (d, 1H, J = 2.4 Hz), 4.70-4.76 (m, 1H), 5.02 (d, 1H, J = 2.4 Hz), 5.71 (d, 1H, J = 4.8 Hz), 6.78 (d, 2H, J = 8.4 Hz), 7.11 (d, 1H, J = 5.6 Hz), 7.25-7.29 (m, 4H), 7.33-7.35 (m, 4H), 7.44 (d, 2H, J = 8.8 Hz), 8.29 (s, 1H), 8.40 (d, 1H, J = 6.0 Hz), 9.60 (br s, 1H). |
| 62 | 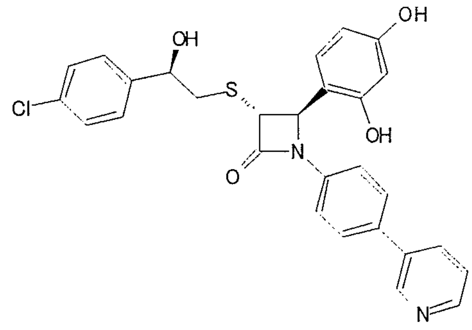 | (DMSO-d6) δ: 2.93-2.95 (m, 2H), 4.38 (d, 1H, J = 2.2 Hz), 4.71 (dd, 1H, J = 11.1, 6.5 Hz), 5.06 (d, 1H, J = 2.2 Hz), 5.69 (d, 1H, J = 4.6 Hz), 6.22 (dd, 1H, J = 8.4, 2.3 Hz), 6.31 (d, 1H, J = 2.2 Hz), 7.06 (d, 1H, J = 8.5 Hz), 7.31-7.44 (m, 7H), 7.67 (d, 2H, J = 8.5 Hz), 7.98-8.00 (m, 1H), 8.50-8.51 (m, 1H), 8.81-8.82 (m, 1H), 9.44 (s, 1H), 9.82 (s, 1H). |

(continued)

| Example No. | Structure | NMR data δ(ppm) |
|---|---|---|
| 63 | 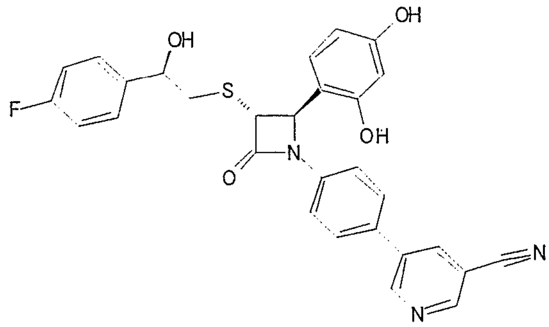 | (DMSO-d6) δ: 2.90-2.99 (m, 2H), 4.39 (d, 1H, J = 2.4 Hz), 4.70 (dd, 1H, J = 11.1, 6.2 Hz), 5.08 (d, 1H, J = 2.2 Hz), 5.65 (d, 1H, J = 4.6 Hz), 6.22 (dd, 1 H, J = 8.3, 2.2 Hz), 6.30 (d, 1H, J = 2.2 Hz), 7.07-7.11 (m, 3H), 7.32-7.36 (m, 4H), 7.77 (d, 2H, J = 8.8 Hz), 8.57 (t, 1H, J = 2.1 Hz), 8.94 (d, 1H, J = 2.0 Hz), 9.12 (d, 1H, J = 2.2 Hz), 9.44 (s, 1H), 9.83 (s, 1H). |

[Table 13]

| Example No. | Structure | NMR data δ(ppm) |
|---|---|---|
| 64 | 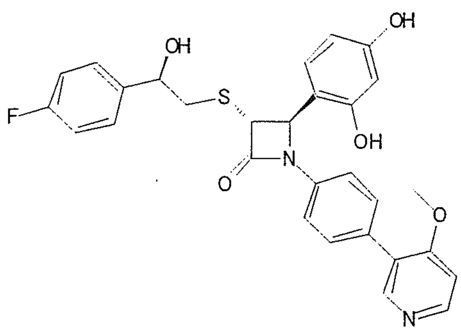 | (DMSO-d6) δ: 2.89-2.98 (m, 2H), 3.81 (s, 3H), 4.35 (d, 1H, J = 2.2 Hz), 4.70 (dd, 1H, J = 11.2, 6.3 Hz), 5.05 (d, 1H, J = 2.4 Hz), 5.65 (d, 1H, J = 4.6 Hz), 6.22 (dd, 1H, J = 8.3, 2.4 Hz), 6.31 (d, 1H, J = 2.2 Hz), 7.06-7.12 (m, 4H), 7.27 (d, 2H, J = 8.8 Hz), 7.34 (dd, 2H, J = 8.7, 5.7 Hz), 7.43 (d, 2H, J = 8.8 Hz), 8.29 (s, 1H), 8.40 (d, 1H, J = 5.6 Hz), 9.44 (s, 1H), 9.82 (s, 1H). |
| 65 | 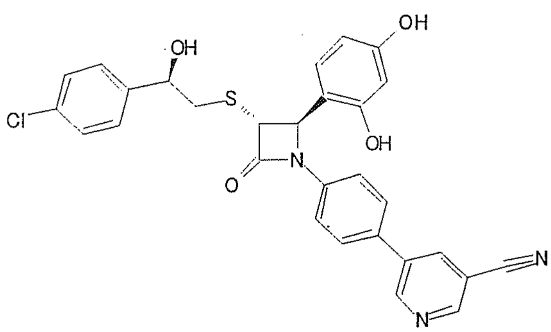 | (DMSO-d6) δ: 2.93-2.95 (m, 2H), 4.41 (d, 1H, J = 2.2 Hz), 4.71 (dd, 1H, J = 11.2, 6.3 Hz), 5.07 (d, 1H, J = 2.2 Hz), 5.69 (d, 1H, J = 4.6 Hz), 6.22 (dd, 1H, J = 8.3, 2.4 Hz), 6.31 (d, 1H, J = 2.2 Hz), 7.07 (d, 1H, J = 8.5 Hz), 7.32-7.34 (m, 6H), 7.77 (d, 2H, J = 8.8 Hz), 8.57 (t, 1H, J = 2.2 Hz), 8.94 (d, 1H, J = 2.0 Hz), 9.12 (d, 1H, J = 2.2 Hz), 9.44 (s, 1H), 9.82 (s, 1H). |
| 66 | 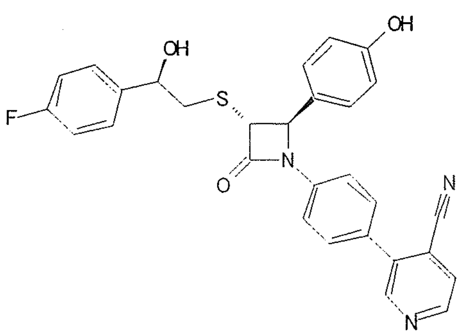 | (DMSO-d6) δ: 2.93-2.95 (m, 2H), 4.31 (d, 1H, J = 2.4 Hz), 4.72-4.74 (m, 1H), 5.09 (d, 1H, J = 2.4 Hz), 5.67 (d, 1H, J = 4.6 Hz), 6.77-6.80 (m, 2H), 7.10-7.12 (m, 2H), 7.30-7.40 (m, 6H), 7.61-7.63 (m, 2H), 7.93 (d, 1H, J = 5.1 Hz), 8.77 (d, 1H, J = 4.9 Hz), 8.84 (s, 1H), 9.61 (s, 1H). |

(continued)

| Example No. | Structure | NMR data δ(ppm) |
|---|---|---|
| 67 | 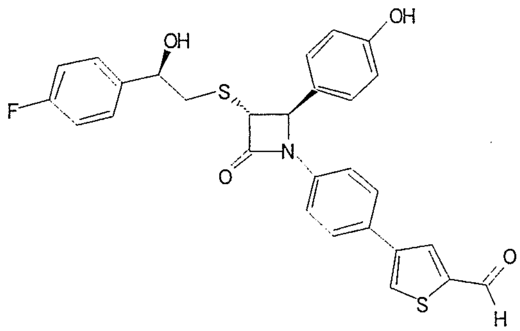 | (DMSO-d6) δ: 2.92-2.94 (m, 2H), 4.27 (d, 1H, J = 2.2 Hz), 4.71-4.74 (m, 1H), 5.04 (d, 1H, J = 2.4 Hz), 5.66 (d, 1H, J = 4.4 Hz), 6.76-6.78 (m, 2H), 7.08-7.14 (m, 2H), 7.26-7.29 (m, 4H), 7.33-7.37 (m, 2H), 7.69-7.71 (m, 2H), 8.34-8.34 (m, 1H), 8.38-8.39 (m, 1H), 9.60 (s, 1H), 9.93-9.93 (m, 1H). |
| 68 | 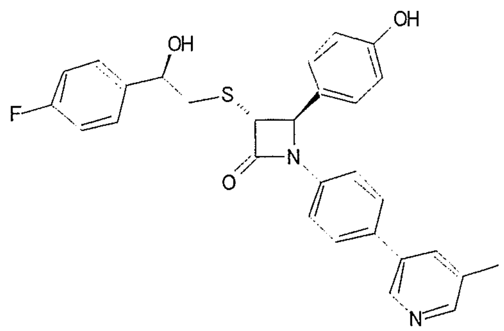 | (DMSO-d6) δ: 2.32 (s, 3H), 2.92-2.94 (m, 2H), 4.27 (d, 1H, J = 2.2 Hz), 4.71-4.74 (m, 1H), 5.05 (d, 1H, J = 2.4 Hz), 5.66 (d, 1H, J = 4.6 Hz), 6.76-6.78 (m, 2H), 7.09-7.12 (m, 2H), 7.26-7.37 (m, 6H), 7.64-7.67 (m, 2H), 7.80-7.82 (m, 1H), 8.35-8.35 (m, 1H), 8.60-8.61 (m, 1H), 9.60 (s, 1H). |

[Table 14]

| Example No. | Structure | NMR data δ(ppm) |
|---|---|---|
| 69 | 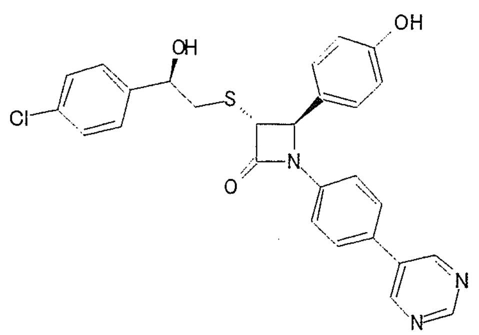 | (DMSO-d6) δ: 2.93 (d, 2H, J = 6.1 Hz), 4.30 (d, 1H, J = 2.4 Hz), 4.70-4.76 (m, 1H), 5.06 (d, 1H, J = 2.4 Hz), 5.71 (d, 1H, J = 4.8 Hz), 6.77 (d, 2H, J = 8.4 Hz), 7.27 (d, 2H, J = 8.4 Hz), 7.32-7.37 (m, 6H), 7.77 (d, 2H, J = 8.5 Hz), 9.06 (s, 2H), 9.13 (s, 1H), 9.60 (br s, 1H). |
| 70 | 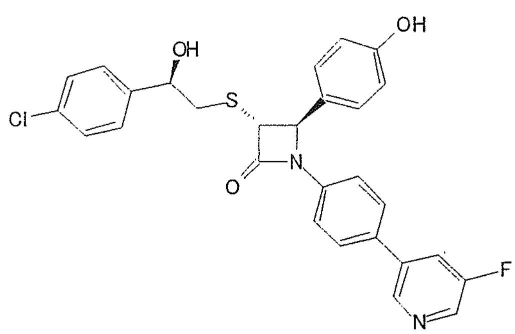 | δ: 2.93 (d, 2H, J = 6.4 Hz), 4.29 (d, 1H, J = 2.4 Hz), 4.70-4.75 (m, 1H), 5.06 (d, 1H, J = 2.4 Hz), 5.72 (d, 1H, J = 4.4 Hz), 6.77 (d, 2H, J = 8.4 Hz), 7.25-7.37 (m, 8H), 7.74 (d, 2H, J = 8.7 Hz), 7.96-8.02 (m, 1H), 8.51 (d, 1H, J = 2.4 Hz), 8.73 (s, 1H), 9.61 (br s, 1H). |

(continued)

| Example No. | Structure | NMR data δ(ppm) |
|---|---|---|
| 71 | 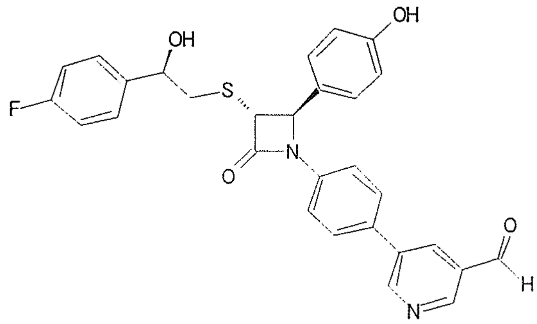 | (DMSO-d6) δ: 2.93-2.95 (m, 2H), 4.29 (d, 1H, J = 2.4 Hz), 4.71-4.74 (m, 1H), 5.07 (d, 1H, J = 2.4 Hz), 5.67 (d, 1H, J = 4.6 Hz), 6.77-6.79 (m, 2H), 7.08-7.13 (m, 2H), 7.27-7.29 (m, 2H), 7.34-7.37 (m, 4H), 7.77-7.79 (m, 2H), 8.42-8.43 (m, 1H), 9.00-9.01 (m, 1H), 9.10-9.11 (m, 1H), 9.61 (s, 1H), 10.14 (s, 1H). |
| 72 | 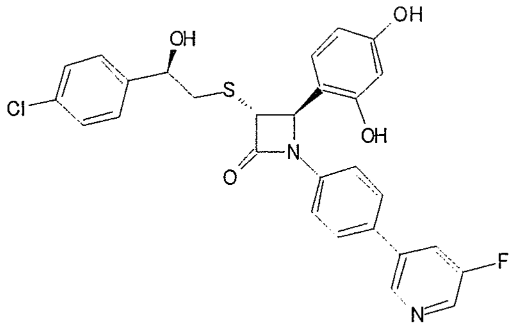 | (DMSO-d6) δ: 2.93-2.95 (m, 2H), 4.40 (d, 1H, J = 2.4 Hz), 4.68-4.73 (m, 1H), 5.07 (d, 1H, J = 2.4 Hz), 5.70 (d, 1H, J = 4.6 Hz), 6.22 (dd, 1H, J = 8.3, 2.2 Hz), 6.31 (d, 1H, J = 2.2 Hz), 7.07 (d, 1H, J = 8.5 Hz), 7.30-7.33 (m, 6H), 7.73 (d, 2H, J = 8.8 Hz), 7.97-8.01 (m, 1H), 8.50-8.51 (m, 1H), 8.73-8.74 (m, 1H), 9.44 (s, 1H), 9.82 (s, 1H). |
| 73 | 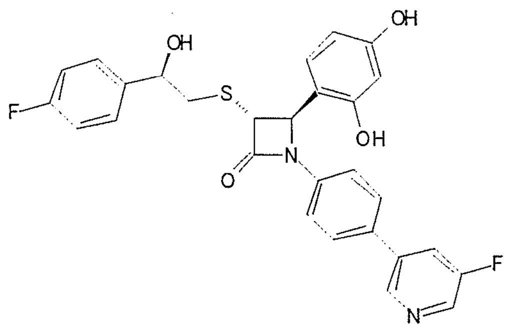 | (DMSO-d6) δ: 2.90-2.99 (m, 2H), 4.38 (d, 1H, J = 2.2 Hz), 4.68-4.72 (m, 1H), 5.07 (d, 1H, J = 2.4 Hz), 5.64 (d, 1H, J = 4.6 Hz), 6.22 (dd, 1H, J = 8.3, 2.4 Hz), 6.31 (d, 1H, J = 2.2 Hz), 7.06-7.12 (m, 3H), 7.31-7.36 (m, 4H), 7.73 (d, 2H, J = 8.8 Hz), 7.97-8.00 (m, 1H), 8.50-8.51 (m, 1H), 8.73-8.74 (m, 1H), 9.44 (s, 1H), 9.82 (s, 1H). |

[Table 15]

| Example No. | Structure | NMR data δ(ppm) |
|---|---|---|
| 74 | 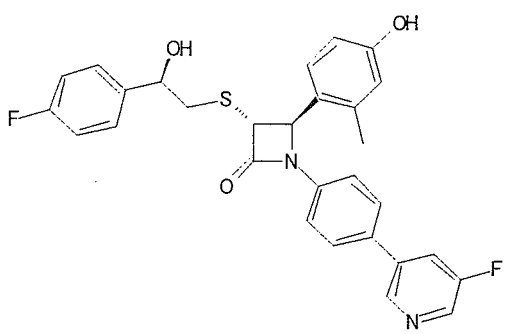 | (DMSO-d6) δ: 2.37 (s, 3H), 2.92-3.01 (m, 2H), 4.17 (d,1H,J= 2.4 Hz), 4.75-4.79 (m, 1H),5.26 (d, 1H, J = 2.4 Hz), 5.65 (d, 1H, J =4.4 Hz), 6.53 (dd, 1H, J = 8.3, 2.4 Hz), 6.65 (d, 1H, J = 2.4 Hz), 6.91 (d, 1H, J = 8.5 Hz), 7.09-7.13 (m, 2H), 7.31-7.39 (m, 4H), 7.77 (d, 2H, J = 8.8 Hz), 7.98-8.02 (m, 1H), 8.52-8.52 (m, 1H), 8.74-8.75 (m, 1H), 9.43 (s, 1H). |

(continued)

| Example No. | Structure | NMR data δ(ppm) |
|---|---|---|
| 75 | 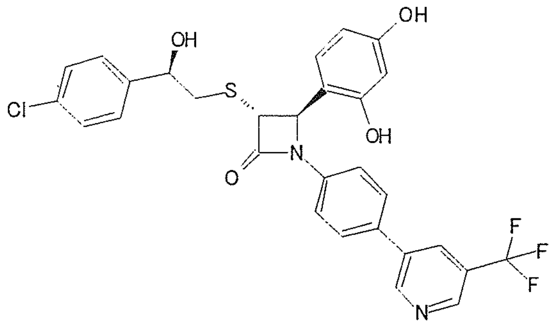 | (DMSO-d6) δ: 2.91-2.98 (m, 2H), 4.41 (d, 1H, J = 2.4 Hz), 4.69-4.73 (m, 1H), 5.08 (d, 1H, J = 2.2 Hz), 5.69 (d, 1H, J = 4.6 Hz), 6.22 (dd, 1H, J = 8.4, 2.3 Hz), 6.31 (d, 1H, J = 2.2 Hz), 7.07 (d, 1H, J = 8.5 Hz), 7.31-7.35 (m, 6H), 7.80 (d, 2H, J = 8.8 Hz), 8.37-8.40 (m, 1H), 8.89-8.91 (m, 1H), 9.13-9.14 (m, 1H), 9.44 (s, 1H), 9.82 (s, 1H). |
| 76 | 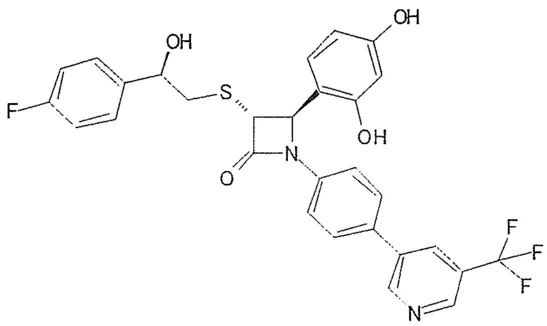 | (DMSO-d6) δ: 2.90-2.99 (m, 2H), 4.39 (d, 1H, J = 2.4 Hz), 4.68-4.73 (m, 1H), 5.08 (d, 1H, J = 2.2 Hz), 5.65 (d, 1H, J = 4.6 Hz), 6.22 (dd, 1H, J = 8.3, 2.2 Hz), 6.31 (d, 1H, J = 2.2 Hz), 7.07-7.11 (m, 3H), 7.33-7.36 (m, 4H), 7.80 (d, 2H, J = 8.5 Hz), 8.38-8.39 (m, 1H), 8.90-8.90 (m, 1H), 9.13-9.14 (m, 1H), 9.44 (s, 1H), 9.83 (s, 1H). |
| 77 | 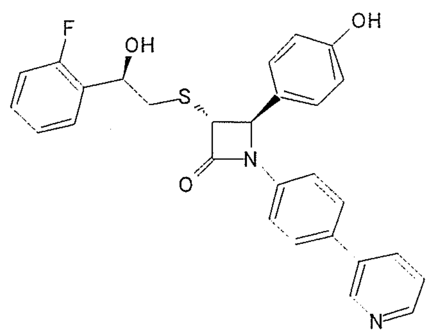 | (DMSO-d6) δ: 2.94-3.03 (m, 2H), 4.28 (d, 1H, J = 2.2 Hz), 5.00-5.02 (m, 2H), 5.76 (d, 1H, J = 4.9 Hz), 6.77-6.80 (m, 2H), 7.09-7.20 (m, 2H), 7.26-7.31 (m, 5H), 7.44-7.48 (m, 2H), 7.66-7.69 (m, 2H), 7.98-8.00 (m, 1H), 8.50-8.54 (m, 1H), 8.81-8.82 (m, 1H), 9.60 (s, 1H). |
| 78 | 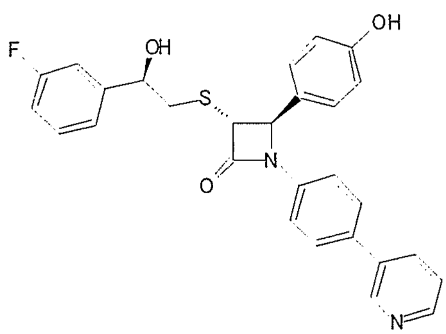 | (DMSO-d6) δ: 2.93-2.97 (m, 2H), 4.32 (d, 1H, J = 2.4 Hz), 4.73-4.80 (m, 1H), 5.07 (d, 1H, J = 2.4 Hz), 5.75-5.76 (m, 1H), 6.66-6.82 (m, 2H), 7.01-7.17 (m, 3H), 7.22-7.37 (m, 5H), 7.41-7.46 (m, 1H), 7.67-7.75 (m, 2H), 7.98-8.00 (m, 1H), 8.51-8.53 (m, 1H), 8.81-8.82 (m, 1H), 9.61 (s, 1H). |

[Table 16]

| Example No. | Structure | NMR data δ(ppm) |
|---|---|---|
| 79 | 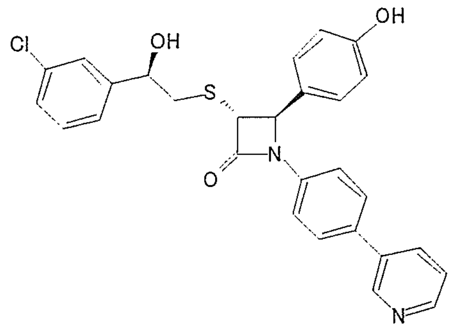 | (DMSO-d6) δ: 2.90-3.00 (m, 2H), 4.33 (d, 1H, J = 2.4 Hz), 4.70-4.79 (m, 1H), 5.06 (d, 1H, J = 2.4 Hz), 5.75-5.77 (m, 1H), 6.73-6.82 (m, 2H), 7.10-7.38 (m, 7H), 7.41-7.48 (m, 1H), 7.66-7.75 (m, 3H), 7.98-8.00 (m, 1H), 8.51-8.53 (m, 1H), 8.81-8.82 (m, 1H),9.60(s, 1H). |
| 80 | 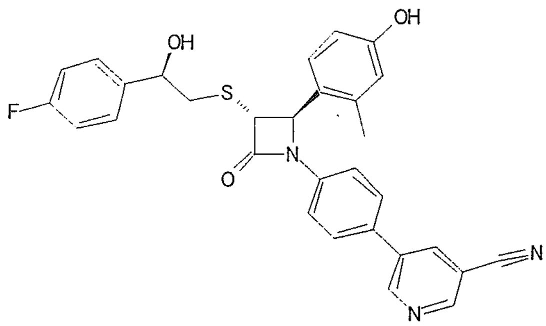 | (DMSO-d6) δ: 2.37 (s, 3H), 2.92-3.01 (m, 2H), 4.18 (d, 1H, J = 2.2 Hz), 4.75-4.79 (m, 1H), 5.27 (d, 1H, J = 2.4 Hz), 5.66 (d, 1H, J = 4.6 Hz), 6.52-6.54 (m, 1H),6.65(d, 1H, J = 2.2 Hz), 6.90 (d, 1H, J = 8.5 Hz), 7.09-7.13 (m, 2H), 7.32-7.39 (m, 4H), 7.81 (d, 2H, J = 8.8 Hz), 8.58-8.59 (m, 1H), 8.95-8.96 (m, 1H), 9.13-9.14 (m, 1H), 9.43 (s, 1H). |
| 81 | 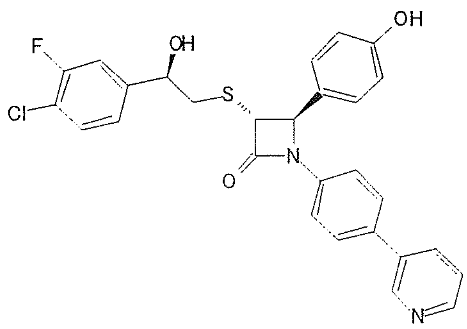 | (DMSO-d6) δ: 2.91-3.01 (m, 2H), 4.31 (d, 1H, J = 2.4 Hz), 4.77-4.78 (m, 1H), 5.07 (d, 1H, J = 2.4 Hz), 5.84 (d, 1H, J = 4.6 Hz), 6.75-6.81 (m, 2H), 7.19-7.21 (m, 1H), 7.26-7.36 (m, 5H), 7.42-7.44 (m, 1H),7.51 (t, 1H, J =7.9 Hz), 7.67-7.69 (m, 2H), 7.98-8.00 (m, 1H), 8.50-8.52 (m, 1H), 8.81-8.82 (m, 1H), 9.61 (s, 1H). |
| 82 | 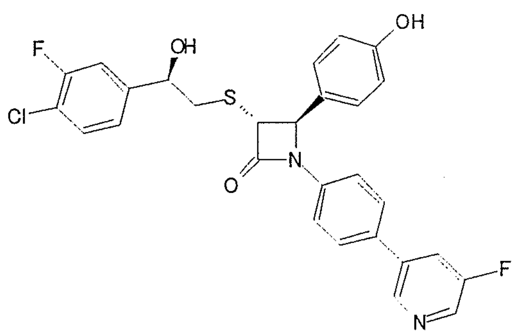 | (DMSO-d6) δ: 2.93-2.99 (m, 2H), 4.32 (d, 1H, J = 2.4 Hz), 4.76-4.78 (m, 1H), 5.08 (d, 1H, J = 2.2 Hz), 5.85 (br s, 1H), 6.75-6.80 (m, 2H), 7.19-7.21 (m, 1H), 7.25-7.38 (m, 5H), 7.51 (t, 1H, J = 8.0 Hz), 7.73-7.75 (m, 2H), 7.97-8.00 (m, 1H), 8.51 (d, 1H, J =2.7 Hz), 8.73-8.73 (m, 1H), 9.61 (s, 1H). |

(continued)

| Example No. | Structure | NMR data δ(ppm) |
|---|---|---|
| 83 | 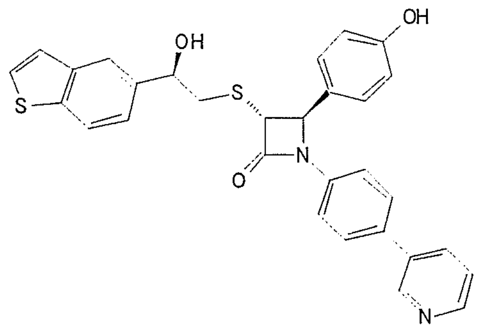 | (DMSO-d6) δ: 2.98-3.00 (m, 2H), 4.32 (d, 1H, J = 2.4 Hz), 4.81-4.85 (m, 1H), 5.00 (d, 1H, J = 2.4 Hz), 5.70 (d, 1H, J = 4.6 Hz), 6.76 (d, 2H, J = 8.5 Hz), 7.24-7.26 (m, 2H), 7.30-7.34 (m, 3H), 7.41-7.44 (m, 2H), 7.66-7.79 (m, 4H), 7.91 (d, 1H, J = 8.3 Hz), 7.97-8.00 (m, 1H), 8.50-8.52 (m, 1H), 8.81-8.82 (m, 1H),9.59(s, 1H). |

[Table 17]

| Example No. | Structure | NMR data δ(ppm) |
|---|---|---|
| 84 | 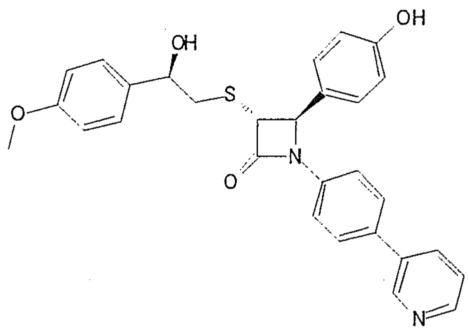 | (DMSO-d6) δ: 2.89-2.93 (m, 2H), 3.71 (s, 3H), 4.24 (d, 1H, J = 2.4 Hz), 4.63-4.65 (m, 1H), 5.01 (d, 1H, J = 2.2 Hz), 5.50 (d, 1H, J = 4.6 Hz), 6.73-6.85 (m, 4H), 7.17-7.33 (m, 6H), 7.40-7.48 (m, 1H), 7.66-7.69 (m, 2H), 7.98-8.00 (m, 1H), 8.50-8.54 (m, 1H), 8.81-8.81 (m, 1H), 9.60 (s, 1H). |
| 85 | 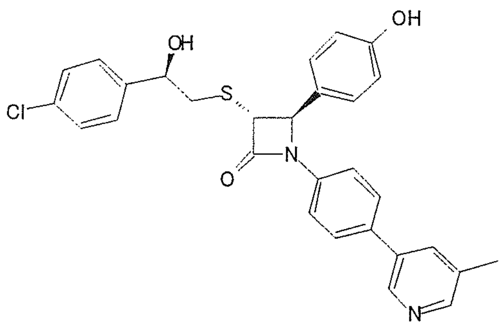 | (DMSO-d6) δ: 2.32 (s, 3H), 2.92-2.94 (m, 2H), 4.27 (d, 1H, J = 2.4 Hz), 4.71-4.75 (m, 1H), 5.04 (d, 1H, J = 2.4 Hz), 5.71 (d, 1H, J = 4.6 Hz), 6.77 (d, 2H, J = 8.5 Hz), 7.26-7.34 (m, 8H), 7.66 (d, 2H, J = 8.8 Hz), 7.81-7.82 (m, 1H), 8.35-8.35 (m, 1H), 8.60-8.61 (m, 1H), 9.60 (s, 1H). |
| 86 | 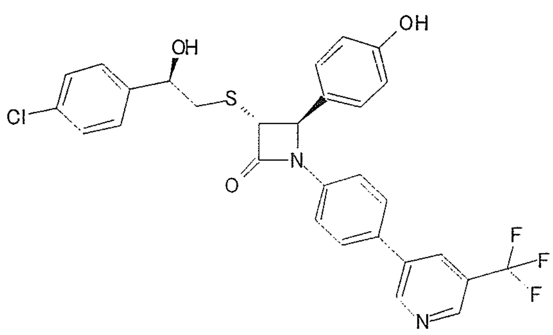 | (DMSO-d6) δ 2.93-2.94 (m, 2H), 4.30 (d, 1H, J = 2.4 Hz), 4.71-4.75 (m, 1H), 5.07 (d, 1H, J = 2.4 Hz), 5.71 (d, 1H, J = 4.6 Hz), 6.77 (d, 2H, J = 8.5 Hz), 7.27-7.29 (m, 2H), 7.33-7.35 (m, 6H), 7.80-7.82 (m, 2H), 8.38-8.39 (m, 1H), 8.90-8.91 (m, 1H), 9.13-9.14 (m, 1H), 9.61 (s, 1H). |

(continued)

| Example No. | Structure | NMR data δ(ppm) |
|---|---|---|
| 87 | 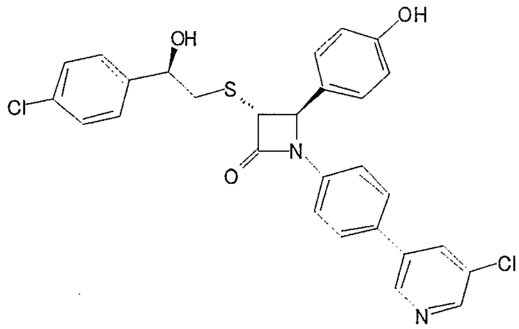 | (DMSO-d6) δ: 2.92-2.94 (m, 2H), 4.29 (d, 1H, J = 2.4 Hz), 4.71-4.75 (m, 1H), 5.06 (d, 1H, J = 2.2 Hz), 5.71 (d, 1H, J = 4.6 Hz), 6.77 (d, 2H, J = 8.5 Hz), 7.26-7.34 (m, 8H), 7.74 (d, 2H, J = 8.8 Hz), 8.16-8.17 (m, 1H), 8.56-8.56 (m, 1H), 8.79-8.80 (m, 1H),9.61 (s, 1H). |
| 88 | 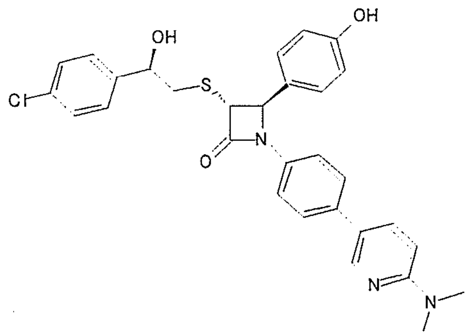 | (DMSO-d6) δ: 2.91-2.93 (m, 2H), 3.02 (s, 6H), 4.24 (d, 1H, J = 2.2 Hz), 4.70-4.74 (m, 1H), 5.00 (d, 1H, J = 2.2 Hz), 5.71 (d, 1H, J = 4.6 Hz), 6.66 (d, 1H, J = 9.0 Hz), 6.76-6.78 (m, 2H), 7.23-7.34 (m, 8H), 7.52 (d, 2H, J = 8.5 Hz), 7.72-7.75 (m, 1H), 8.34-8.34 (m, 1H), 9.59 (s, 1H). |

[Table 18]

| Example No. | Structure | NMR data δ(ppm) |
|---|---|---|
| 89 | 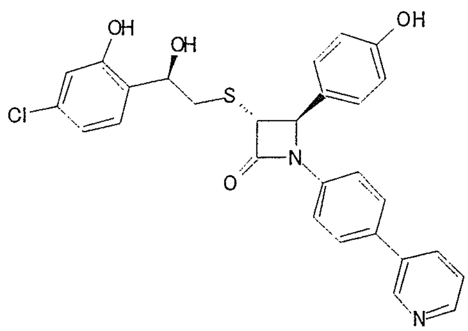 | (DMSO-d6) δ: 2.82-2.87 (m, 1H), 2.94-2.98 (m, 1H), 4.27 (d, 1H, J = 2.2 Hz), 4.96-5.02 (m, 2H), 5.53 (d, 1H, J = 4.9 Hz), 6.76-6.82 (m, 4H), 7.25-7.33 (m, 5H), 7.41-7.45 (m, 1H), 7.67 (d, 2H, J = 8.5 Hz), 7.98-8.00 (m, 1H), 8.51-8.52 (m, 1H), 8.81-8.82 (m, 1H), 9.59 (s, 1H), 10.01 (s, 1H). |
| 90 | 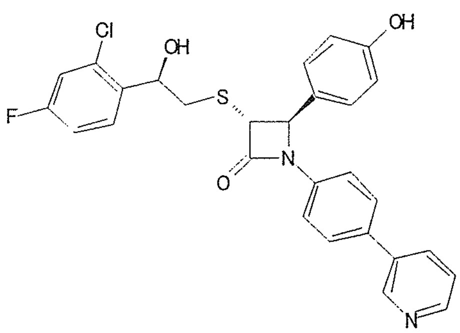 | (DMSO-d6) δ: 2.83-2.86 (m, 1H), 2.98 (dd, 1H, J = 13.3,4.0 Hz), 4.31 (d, 1H, J = 2.4 Hz), 5.02-5.08 (m, 2H), 5.91 (d, 1H, J = 4.9 Hz), 6.76-6.78 (m, 2H), 7.18-7.23 (m, 1H), 7.25-7.38 (m, 5H), 7.41-7.44 (m, 1H), 7.61 (dd, 1H, J = 8.7, 6.5 Hz), 7.66-7.69 (m, 2H), 7.98-8.00 (m, 1H), 8.50-8.52 (m, 1H), 8.81-8.82 (m, 1H), 9.60 (s, 1H). |

(continued)

| Example No. | Structure | NMR data δ(ppm) |
|---|---|---|
| 91 | 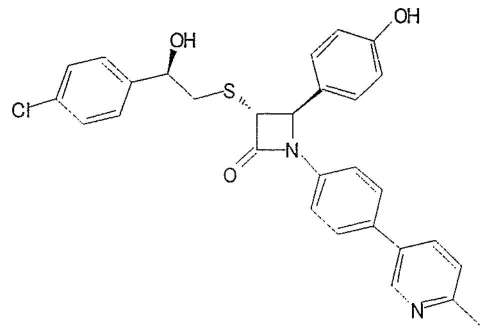 | (DMSO-d6) δ: 2.46 (s, 3H), 2.92-2.93 (m, 2H), 4.27 (d, 1H, J = 2.4 Hz), 4.70-4.75 (m, 1H), 5.03 (d, 1H, J = 2.2 Hz), 5.71 (d, 1H, J =4.6 Hz), 6.77 (d, 2H, J = 8.5 Hz), 7.26-7.34 (m, 9H), 7.63-7.65 (m, 2H), 7.86-7.89 (m, 1H), 8.67-8.67 (m, 1H), 9.60 (s, 1H). |
| 92 | 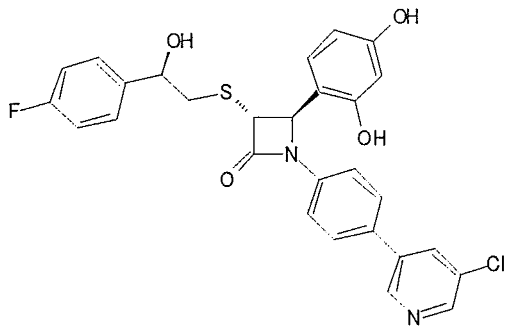 | (DMSO-d6) δ: 2.90-2.98 (m, 2H), 4.38 (d, 1H, J = 2.4 Hz), 4.68-4.72 (m, 1H), 5.07 (d, 1H, J = 2.2 Hz), 5.64 (d, 1H, J = 4.6 Hz), 6.21-6.23 (m, 1H), 6.30-6.31 (m, 1H), 7.06-7.11 (m, 3H), 7.31-7.36 (m, 4H), 7.72-7.74 (m, 2H), 8.16-8.17 (m, 1H), 8.55-8.56 (m, 1H), 8.79-8.80 (m, 1H), 9.44 (s, 1H), 9.82 (s, 1H). |
| 93 | 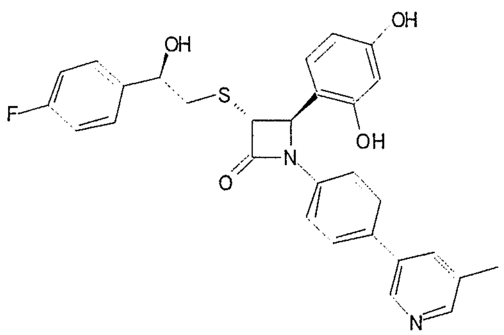 | (DMSO-d6) δ:2.32 (s, 3H), 2.90-2.99 (m, 2H), 4.36 (d, 1H, J = 2.4 Hz), 4.68-4.72 (m, 1H), 5.06 (d, 1H, J = 2.2 Hz), 5.64 (d, 1H, J = 4.6 Hz), 6.21-6.23 (m, 1H), 6.31-6.31 (m, 1H), 7.05-7.11 (m, 3H), 7.30-7.36 (m, 4H), 7.65 (d, 2H, J = 8.8 Hz), 7.81-7.82 (m, 1H), 8.34-8.35 (m, 1H), 8.61-8.61 (m, 1H), 9.43 (s, 1H), 9.81 (s, 1H). |

[Table 19]

| Example No. | Structure | NMR data δ(ppm) |
|---|---|---|
| 94 | 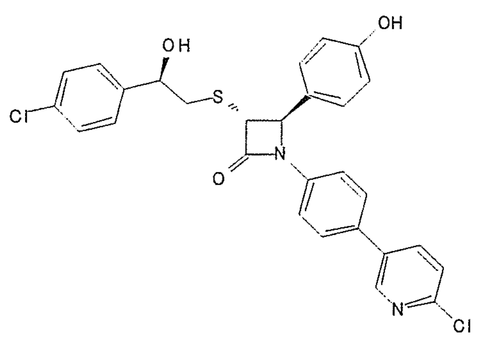 | (DMSO-d6) δ: 2.92-2.94 (m, 2H), 4.28 (d, 1H, J = 2.4 Hz), 4.70-4.75 (m, 1H), 5.04 (d, 1H, J = 2.2 Hz), 5.71 (d, 1H, J = 4.6 Hz), 6.77 (d, 2H, J = 8.5 Hz), 7.26-7.34 (m, 8H), 7.55 (d, 1H, J = 8.5 Hz), 7.69 (d, 2H, J = 8.8 Hz), 8.06-8.09 (m, 1H), 8.65-8.66 (m, 1H),9.60(s, 1H). |

(continued)

| Example No. | Structure | NMR data δ(ppm) |
|---|---|---|
| 95 | 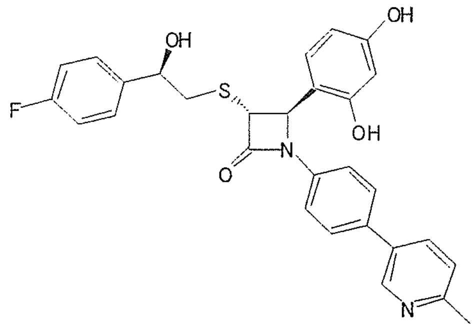 | (DMSO-d6) δ: 2.46 (s, 3H), 2.89-2.98 (m, 2H), 4.36 (d, 1H, J = 2.2 Hz), 4.68-4.72 (m, 1H), 5.06 (d, 1H, J = 2.2 Hz), 5.64 (d, 1H, J =4.6 Hz), 6.20-6.23 (m, 1H), 6.31-6.31 (m, 1H), 7.05-7.11 (m, 3H), 7.27-7.36 (m, 5H), 7.62-7.64 (m, 2H), 7.86-7.89 (m, 1H), 8.67-8.67 (m, 1H), 9.43 (s, 1H), 9.81 (s, 1H). |
| 96 | 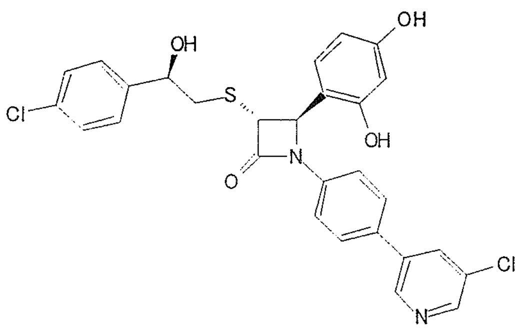 | (DMSO-d6) δ: 2.93-2.95 (m, 2H), 4.40 (d, 1H, J = 2.2 Hz), 4.68-4.73 (m, 1H), 5.07 (d, 1H, J = 2.2 Hz), 5.69 (d, 1H, J = 4.6 Hz), 6.21-6.24 (m, 1H), 6.30-6.31 (m, 1H), 7.07 (d, 1H, J = 8.3 Hz), 7.29-7.33 (m, 6H), 7.72-7.74 (m, 2H), 8.16-8.17 (m, 1H), 8.55-8.56 (m, 1H), 8.79-8.80 (m, 1H), 9.44 (s, 1H), 9.82 (s, 1H). |
| 97 | 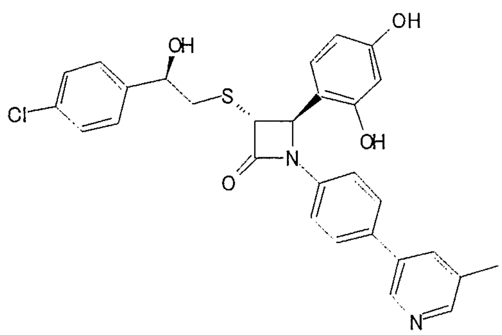 | (DMSO-d6) δ: 2.32 (s, 3H), 2.93-2.95 (m, 2H), 4.38 (d, 1H, J = 2.2 Hz), 4.69-4.73 (m, 1H), 5.06 (d, 1H, J = 2.2 Hz), 5.69 (d, 1H, J = 4.6 Hz), 6.21-6.23 (m, 1H), 6.31-6.31 (m, 1H), 7.06 (d, 1H, J = 8.5 Hz), 7.30-7.33 (m, 6H), 7.64-7.66 (m, 2H), 7.81-7.82 (m, 1H), 8.34-8.35 (m, 1H), 8.61-8.61 (m, 1H), 9.44 (s, 1H), 9.82 (s, 1H). |
| 98 | 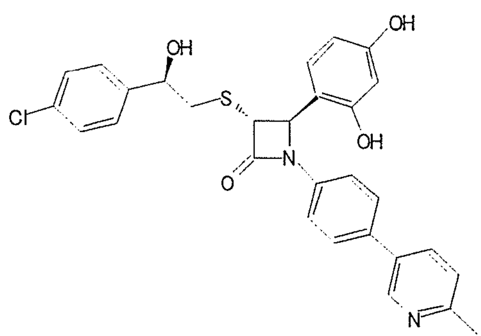 | (DMSO-d6) δ: 2.46 (s, 3H), 2.91-2.95 (m, 2H), 4.37 (d, 1H, J = 2.2 Hz), 4.68-4.73 (m, 1H), 5.05 (d, 1H, J = 2.2 Hz), 5.69 (d, 1H, J = 4.9 Hz), 6.20-6.23 (m, 1H), 6.31-6.31 (m, 1H), 7.05 (d, 1H, J = 8.3 Hz), 7.27-7.33 (m, 7H), 7.62-7.64 (m, 2H), 7.86-7.89 (m, 1H), 8.67-8.68 (m, 1H), 9.43 (s, 1H), 9.81 (s, 1H). |

[Table 20]

| Example No. | Structure | NMR data δ(ppm) |
|---|---|---|
| 99 | 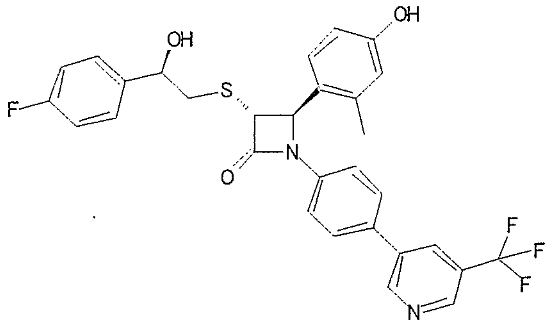 | (DMSO-d6) δ: 2.37 (s, 3H), 2.92-3.01 (m, 2H), 4.18 (d, 1H, J = 2.2 Hz), 4.75-4.79 (m, 1H), 5.27 (d, 1H, J = 2.4 Hz), 5.65-5.67 (m, 1H), 6.52-6.55 (m, 1H), 6.65-6.65 (m, 1H), 6.91 (d, 1H, J = 8.3 Hz), 7.09-7.13 (m, 2H), 7.33-7.39 (m, 4H), 7.83 (d, 2H, J = 8.8 Hz), 8.39-8.40 (m, 1H), 8.91-8.92 (m, 1H), 9.15-9.15 (m, 1H), 9.43 (s, 1H). |
| 100 | 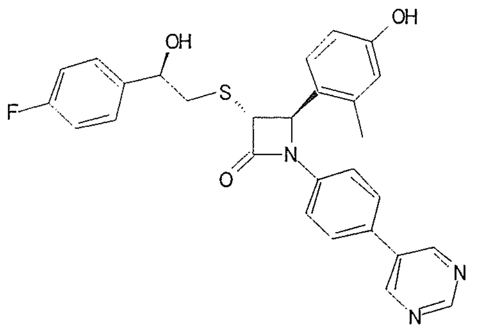 | (DMSO-d6) δ: 2.37 (s, 3H), 2.94-3.01 (m, 2H), 4.18 (d, 1H, J = 2.4 Hz), 4.75-4.79 (m, 1H), 5.27 (d, 1H, J = 2.2 Hz), 5.66 (d, 1H, J = 4.6 Hz), 6.52-6.55 (m, 1H), 6.64-6.65 (m, 1H), 6.91 (d, 1H, J = 8.3 Hz), 7.09-7.13 (m, 2H), 7.33-7.39 (m, 4H), 7.78-7.80 (m, 2H), 9.08 (s, 2H), 9.13 (s, 1H), 9.43 (s, 1H). |
| 101 | 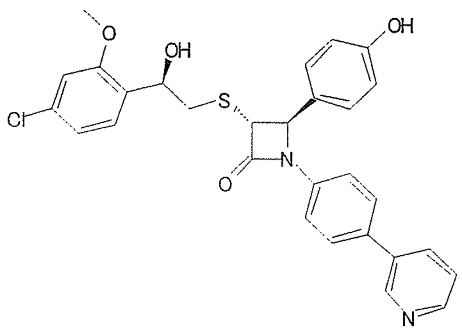 | (DMSO-d6) δ: 2.80-2.83 (m, 1H), 2.90-2.93 (m, 1H), 3.66 (s, 3H), 4.26 (d, 1H, J = 2.4 Hz), 4.97-5.01 (m, 2H), 5.58 (d, 1H, J = 4.9 Hz), 6.77-6.79 (m, 2H), 6.96-6.98 (m, 2H), 7.28-7.32 (m, 4H), 7.39-7.44 (m, 2H), 7.66-7.69 (m, 2H), 7.98-8.00 (m, 1H), 8.50-8.52 (m, 1H), 8.81-8.82 (m, 1H), 9.60 (s, 1H). |
| 102 | 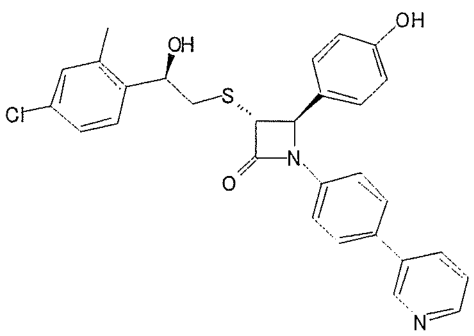 | (DMSO-d6) δ: 2.22 (s, 3H), 2.82-2.97 (m, 2H), 4.30 (d, 1H, J = 2.4 Hz), 4.90-4.95 (m, 1H), 5.01 (d, 1H, J = 2.4 Hz), 5.61 (d, 1H, J = 4.6 Hz), 6.76-6.78 (m, 2H), 7.19-7.20 (m, 2H), 7.26-7.28 (m, 2H), 7.31-7.33 (m, 2H), 7.41-7.44 (m, 2H), 7.67-7.69 (m, 2H), 7.98-8.01 (m, 1H), 8.51-8.52 (m, 1H), 8.82 (br s, 1H), 9.60 (s, 1H). |

(continued)

| Example No. | Structure | NMR data δ(ppm) |
|---|---|---|
| 103 | 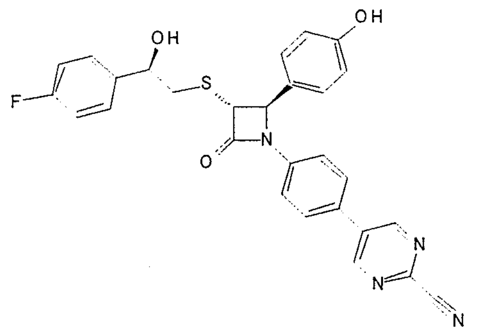 | (DMSO-d6) δ: 2.93-2.94 (m, 2H), 4.32 (d, 1H, J = 2.4 Hz), 4.71-4.74 (m, 1H), 5.09 (d, 1H, J = 2.4 Hz), 5.66 (d, 1H, J = 4.6 Hz), 6.76-6.78 (m, 2H), 7.08-7.13 (m, 2H), 7.27-7.29 (m, 2H), 7.33-7.38 (m, 4H), 7.87-7.89 (m, 2H), 9.30 (s, 2H), 9.60 (s, 1H). |

[Table 21]

| Example No. | Structure | NMR data δ(ppm) |
|---|---|---|
| 104 | 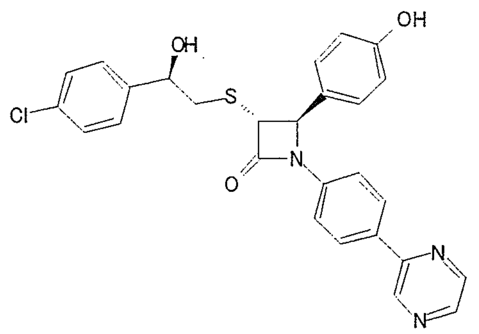 | (DMSO-d6) δ: 2.93-2.95 (m, 2H), 4.30 (d, 1H, J = 2.4 Hz), 4.72-4.74 (m, 1H), 5.07 (d, 1H, J = 2.4 Hz), 5.72 (d, 1H, J = 4.6 Hz), 6.76-6.78 (m, 2H), 7.26-7.36 (m, 8H), 8.07-8.09 (m, 2H), 8.56-8.64 (m, 2H), 9.16-9.16 (m, 1H), 9.61 (s, 1H). |
| 105 | 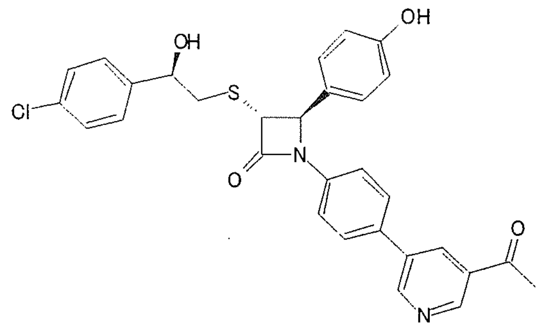 | (DMSO-d6) δ: 2.66 (s, 3H), 2.93-2.94 (m, 2H), 4.29 (d, 1H, J = 2.2 Hz), 4.71-4.75 (m, 1H), 5.06 (d, 1H, J = 2.2 Hz), 5.71 (d, 1H, J = 4.9 Hz), 6.77-6.79 (m, 2H), 7.27-7.29 (m, 2H), 7.34-7.36 (m, 6H), 7.76-7.78 (m, 2H), 8.41-8.42 (m, 1H), 9.03-9.04 (m, 2H), 9.60 (s, 1H). |
| 106 | 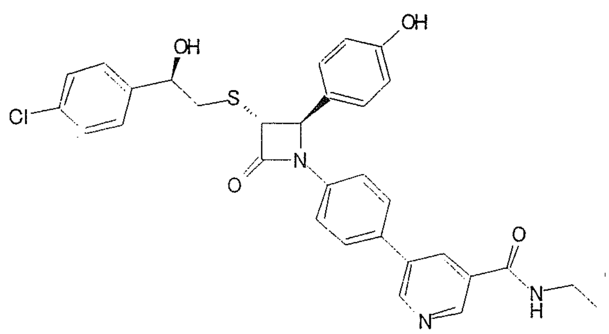 | (DMSO-d6) δ: 1.11-1.15 (m, 3H), 2.93-2.94 (m, 2H), 3.27-3.34 (m, 2H), 4.29 (d, 1H, J = 2.4 Hz), 4.71-4.75 (m, 1H), 5.05 (d, 1H, J = 2.2 Hz), 5.71 (d, 1H, J = 4.9 Hz), 6.76-6.78 (m, 2H), 7.26-7.29 (m, 2H), 7.33-7.35 (m, 6H), 7.73-7.75 (m, 2H), 8.34-8.35 (m, 1H), 8.67-8.69 (m, 1H), 8.90-8.93 (m, 2H), 9.60 (s, 1H). |

[Example 107]

Synthesis of 4-{(2R,3R)-3-[(2R)-2-acetyloxy-2-(4-fluorophenyl)ethylthio]-4-oxo-1-(4-(pyridin-3-yl)phenyl)azetidin-2-yl} phenyl acetate

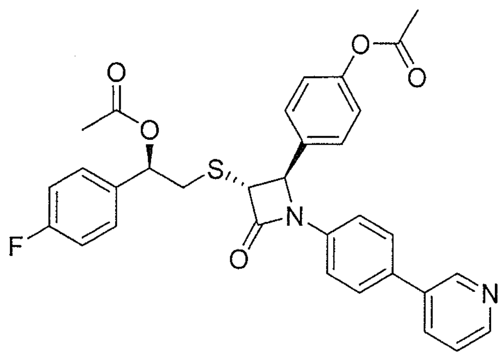

Acetic anhydride (0.571 mL) and 4-(dimethylamino)pyridine (0.807 g) were added to a solution of (3R,4R)-3-[(2R)-2-(4-fluorophenyl)-2-hydroxyethylthio]-4-(4-hydroxyphenyl)-1-(4-(pyridin-3-yl)phenyl)azetidin-2-one (1.34 g) in tetrahydrofuran (20 mL), and the resulted mixture was stirred at room temperature overnight. The reaction solution was diluted with ethyl acetate and sequentially washed with water and brine. The organic layer was dried over sodium sulfate and filtered. The filtrate was concentrated under reduced pressure and the obtained residue was purified by silica gel column chromatography (ethyl acetate/hexane = 1/1→2/1) to obtain the title compound (1.30 g, 83% yield). The NMR data of this compound is shown below.
$^1$H-NMR (400 MHz, DMSO-$d_6$): δ (ppm) = 2.08-2.08 (m, 3H), 2.26 (s, 3H), 3.16-3.22 (m, 2H), 4.37 (d, 1H, J = 2.2 Hz), 5.25-5.27 (m, 1H), 5.83-5.84 (m, 1H), 7.12-7.22 (m, 4H), 7.32-7.46 (m, 5H), 7.52 (d, 2H, J = 8.3 Hz), 7.70 (d, 2H, J = 8.3 Hz), 7.99-8.01 (m, 1H), 8.51-8.52 (m, 1H), 8.83 (s, 1H).

[Example 108]

Synthesis of (1R)-2-[(3R,4R)-4-(4-hydroxyphenyl)-2-oxo-1-(4-(pyridin-3-yl)phenyl)azetidin-3-ylthio]-1-(4-fluorophenyl) ethyl acetate

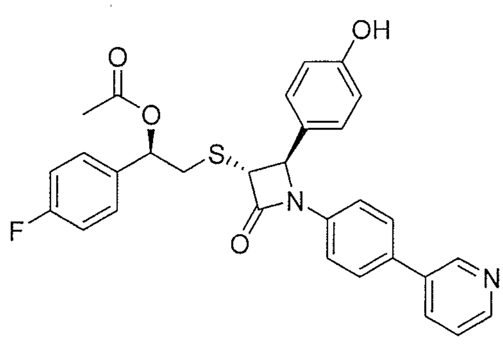

Guanidine hydrochloride (0.753 g) and sodium methoxide (0.383 g) are dissolved in methanol (20 mL) to prepare a guanidine solution. A solution of 4-{(2R,3R)-3-[(2R)-2-acetyloxy-2-(4-fluorophenyl)ethylthio]-4-oxo-1-(4-(pyridin-3-yl)phenyl)azetidin-2-yl}phenyl acetate (1.26 g) in methanol (25 mL) was cooled to 0 ˚C, and the guanidine solution (2.8 mL) was added dropwise very slowly. After the resulted mixture was stirred at 0 ˚C for about 10 minutes, acetic acid was added to quench the reaction. Ethyl acetate was added to the reaction solution and washed sequentially with water and brine. The organic layer was dried over sodium sulfate and filtered. The filtrate was concentrated under

reduced pressure, and the obtained residue was purified by silica gel column chromatography (ethyl acetate/hexane = 1/1→2/1) to obtain the title compound (0.868 g, 60% yield). The NMR data of this compound is shown below.
$^1$H-NMR (400 MHz, DMSO-$d_6$): δ (ppm) = 1.96 (s, 3H), 3.14-3.21 (m, 2H), 4.31 (d, 1H, J = 2.0 Hz), 5.07 (d, 1H, J = 2.2 Hz), 5.82-5.83 (m, 1H), 6.76-6.83 (m, 2H), 7.13-7.19 (m, 2H), 7.27-7.46 (m, 6H), 7.66-7.75 (m, 3H), 7.96-8.02 (m, 1H), 8.50-8.52 (m, 1H), 8.81-8.83 (m, 1H), 9.61 (brs, 1H) .

[Example 109]

Synthesis of (2S,3R,4S,5R,6S)-6-(4-{(2R,3R)-3-[(2R)-2-acetyloxy-2-(4-fluorophenyl)ethylthio]-4-oxo-1-(4-(pyridin-3-yl)phenyl)azetidin-2-yl}phenoxy)-4,5-diacetyloxy-2-(methoxycarbonyl)-2H-3,4,5,6-tetrahydropyran-3-yl acetate

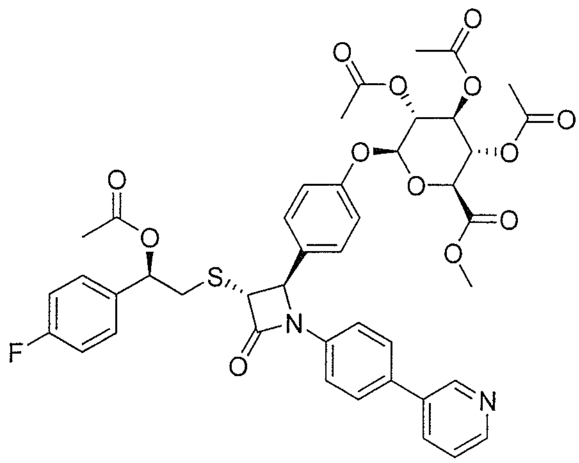

Under nitrogen atmosphere, (2S,3R,4S,5R,6R)-4,5-diacetyloxy-2-(methoxycarbonyl)-6-(2,2,2-trichloro-1-iminoethoxy)-2H-3,4,5,6-tetrahydropyran-3-yl acetate (978 mg) synthesized according to the method of Rele, S.M. et al. (J. Org. Chem. 69, 9159-9170, 2004) and a small amount of molecular sieves 3A were added to a solution of (1R)-2-[(3R,4R)-4-(4-hydroxyphenyl)-2-oxo-1-(4-(pyridin-3-yl)phenyl)azetidin-3-ylthio]-1-(4-fluorophenyl)ethyl acetate (540 mg) in dichloromethane (15 mL), and the resulted mixture was cooled to -10 °C. After boron trifluoride diethyl ether complex (0.194 mL) was slowly added dropwise, the resulted mixture was stirred at 0 °C for 2 hours, then at room temperature for further 4 hours. After triethylamine was added to quench the reaction, the resulted mixture was filtered. The filtrate was concentrated under reduced pressure and the obtained residue was purified by silica gel column chromatography (ethyl acetate/hexane = 2/1→3/1) to obtain the title compound (344 mg, 40% yield). The NMR data of this compound is shown below.
$^1$H-NMR (400 MHz, DMSO-$d_6$): δ (ppm) = 1.95-2.00 (m, 12H), 3.15-3.20 (m, 2H), 3.61 (s, 3H), 4.33 (d, 1H, J = 1.7 Hz), 4.70 (d, 1H, J = 10.0 Hz), 5.01-5.13 (m, 2H), 5.18 (d, 1H, J = 2.2 Hz), 5.44 (t, 1H, J = 9.5 Hz), 5.68 (d, 1H, J = 7.8 Hz), 5.80-5.85 (m, 1H), 7.01-7.07 (m, 2H), 7.11-7.18 (m, 2H), 7.31-7.35 (m, 2H), 7.36-7.48 (m, 5H), 7.66-7.71 (m, 2H), 7.97-8.02 (m, 1H), 8.50-8.52 (m, 1H), 8.80-8.83 (m, 1H).

[Example 110]

Synthesis of (2S,3S,4S,5R,6S)-6-(4-{(2R,3R)-3-[(2R)-2-(4-fluorophenyl)-2-hydroxyethylthio]-4-oxo-1-(4-(pyridin-3-yl)phenyl)azetidin-2-yl}phenoxy)-3,4,5-trihydroxy-2H-3,4,5,6-tetrahydropyran-2-carboxylic acid

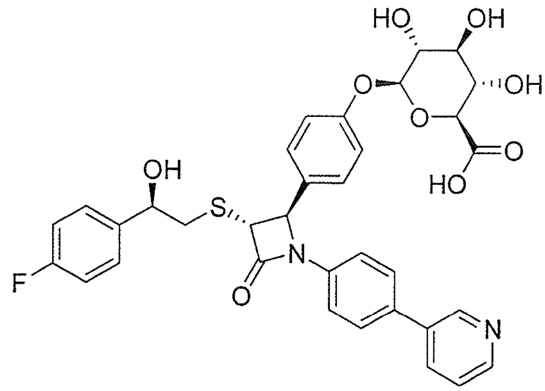

After triethylamine (2 mL) was added to a solution of (2S,3R,4S,5R,6S)-6-(4-{(2R,3R)-3-[(2R)-2-acetyloxy-2-(4-fluorophenyl)ethylthio]-4-oxo-1-(4-(pyridin-3-yl)phenyl)azetidin-2-yl}phenoxy)-4,5-diacetyloxy-2-(methoxycarbonyl)-2H-3,4,5,6-tetrahydropyran-3-yl acetate (89 mg) in methanol (2 mL), water (7 mL) was slowly added dropwise. After the resulted mixture was stirred at room temperature overnight, the solvent was distilled off under reduced pressure. A small amount of 1 N hydrochloric acid was added to the obtained residue and the precipitated solid was filtered off. The obtained solid was purified by preparative HPLC to obtain the title compound (4.4 mg, 5% yield). The NMR data of this compound is shown below.

$^1$H-NMR (400 MHz, $CD_3OD$): δ (ppm) = 2.96-3.09 (m, 2H), 3.50-3.58 (m, 2H), 3.59-3.68 (m, 1H), 4.04 (d, 1H, J = 9.8 Hz), 4.73-4.82 (m, 2H), 5.04-5.12 (m, 1H), 6.94-7.01 (m, 2H), 7.08-7.16 (m, 2H), 7.24-7.30 (m, 2H), 7.74-7.95 (m, 7H), 8.51-8.58 (m, 1H), 8.63-8.68 (m, 1H), 9.00 (brs, 1H) .

In vitro and in vivo activities of the compounds represented by formula (I) are measured according to the following methods.

[Example 111]

Cholesterol absorption inhibition assay (in vitro)

This assay measures cholesterol absorption inhibitory activity by adding mixed micelles containing [$^{14}$C]cholesterol to cells and measuring cholesterol taken in the cells as oleic acid cholesteryl ester. The assay is conducted after Caco-2 cells are seeded on multi-well plates and cultured (culture medium: DMEM containing 10% fetal bovine serum, 1% non-essential amino acids, 1% penicillin, and 1% streptomycin) for 2 weeks. To each well, the micelle solution (in an amount required to provide the following final concentrations: 600 μM of sodium oleate, 50 μM of phosphatidyl choline, 5 mM of sodium taurocholate, 2.5 μM of cholesterol, and 2.5 μM of [$^{14}$C] cholesterol) and a test compound solution (in an amount required to provide the final concentration of 0.5 to 25 μM) or a solvent as a control are added, and incubation is started at 37°C. 24 hours later, the culture medium is removed, and the lipid component is extracted with hexane/2-propanol (3:2, v/v) and applied onto thin-layer silica gel plates. After drying, each plate is eluted with a mixture of hexane, diethyl ether, and acetic acid in a ratio of 83:16:1. The plates are photoexposed on imaging plates and analyzed using BAS-2500 (FUJIFILM). The amounts of light exposure of the areas corresponding to the cholesteryl ester are calculated. Background is measured using a blank space of the plate. Inhibition rates are calculated using values obtained by subtracting the background values from both values of the control and the test sample, respectively, in accordance with the following equation. A test value is calculated as a percentage (%) with respect to the control.

[Equation 1]

$$\text{Inhibition rate (\%)} = \frac{(\text{The amount of light exposure in [A} - \text{BG]}) - (\text{The amount of light exposure in [C} - \text{BG]})}{(\text{The amount of light exposure in [A} - \text{BG]})} \times 100$$

In the Equation 1, A is the control group, BG is a background, and B is the test compound.

The following results were obtained from the measurement by this method on cholesterol absorption inhibitory activity of the compounds of the present invention. The following results were obtained by the evaluation using concentrations of 10 μM and/or 12.5 μM.

Compounds which exhibited cholesterol absorption inhibitory activity of 50% or higher at a concentration of 10 μM:

Compounds of Examples 4, 5, 7, 8, 9, 11, 12, 13, 14, 15, 16, 19, 20, 21, 22, 23, 24, 28, 29, 31, 32, 34, 36, 37, 38, 39, 43, 45, 47, 48, 50, 51, 52, 54, 55, 56, 57, and 58

Compounds which exhibited cholesterol absorption inhibitory activity of 60% or higher at a concentration of 12.5 μM: Compounds of Examples 1, 4, 5, 6, 16, 20, 22, 31, 32, 45, 48, 57, 58, 59, 61, 81, 83, 85, 86, 87, 88, 90, 91, 94, 99, and 105

[Example 112]

Measurement of lipid lowering effect in hamsters fed a high cholesterol diet:

Male Golden Syrian hamsters are divided into groups each consisting of 6 animals and fed a controlled high cholesterol diet (CE-2 containing 0.5% cholesterol; CLEA Japan, Inc.) for 28 days at most. From the start of feeding of the high cholesterol diet, the test compounds are administered by gavage once daily. For the administration, 0.2 mL of corn oil alone per 100 g (control group) or a solution (or suspension) of the test compound in corn oil at a dose of 0.01 to 10 mg/kg was given. At the same time, a group fed with a normal diet during the same study period is handled as a normal group. 4 hours after the last administration, whole blood is collected from the abdominal aorta under light anesthesia with ether and serum is separated from the blood. Further, livers are harvested, wet liver weights are measured, and part (about 0.5 g) of the harvested livers are extracted and weighed. 2-propanol in an amount 4 times the weight of the extracted livers are added, the resulted mixtures are homogenized, and the centrifuged supernatant are recovered and used as liver-derived samples.

Serum and liver TC concentrations are determined by an enzyme method using L-type Wako CHO·H (Wako Pure Chemical Industries Ltd.) or Cholestest CHO (Daiichi Pure Chemicals Co., Ltd.). Serum TG concentrations are determined by an enzyme method using L-type Wako TG·H (Wako Pure Chemical Industries Ltd.) or Autosera S TG-N (Daiichi Pure Chemicals Co., Ltd.) and a free glycerine elimination method. LDL-C concentrations are determined by a direct method using L-type Wako LDL-C·M (Wako Pure Chemical Industries Ltd.) or Cholestest LDL (Daiichi Pure Chemicals Co., Ltd.). Human serum was commonly used as a sample for the calibration curve for determinations of serum and liver TC concentrations, serum TG concentration, and serum LDL-C concentration. The effect of the test compound is shown by a suppression rate (%) of an increase in serum TC concentration due to feeding of the high cholesterol diet, a serum TG lowering rate (%), a suppression rate (%) of an increase in serum LDL-C concentration due to feeding of the high cholesterol diet, and a suppression rate (%) of an increase in liver TC concentration due to feeding of the high cholesterol diet calculated in accordance with the following equations.

[Equation 2]

$$\text{Serum TC suppression rate (\%)} = \frac{\text{Serum TC concentration in } (A - B)}{\text{Serum TC concentration in } (A - C)} \times 100$$

In the Equation 2, A is the control group, B is the test compound group, and C is the normal group.

[Equation 3]

$$\text{Serum LDL-C suppression rate (\%)} = \frac{\text{Serum LDL-C concentration in } (A - B)}{\text{Serum LDL-C concentration in } (A - C)} \times 100$$

In the Equation 3, A is the control group, B is the test compound group, and C is the normal group.

[Equation 4]

$$\text{Liver TC suppression rate (\%)} = \frac{\text{Liver TC concentration in (A} - \text{B)}}{\text{Liver TC concentration in (A} - \text{C)}} \times 100$$

In the Equation 4, A is the control group, B is the test compound group, and C is the normal group.

[Equation 5]

$$\text{Serum TG lowering rate (\%)} = \frac{\text{Serum TG concentration in (A} - \text{B)}}{\text{Serum TG concentration in A}} \times 100$$

In the Equation 5, A is the control group, and B is the test compound group.

The following results were obtained from the study by this method on the effects of lowering serum TC, LDL-C, and TG, and liver TC in the hamsters fed with a high cholesterol diet which were given the test compounds for 7 days.

[Test Example 1] A serum TC lowering effect (a dose: 0.01 mg/kg)

Compounds that exhibited a serum TC lowering effect of 30% or higher at a dose of 0.01 mg/kg:

Compounds of Examples 2, 18, 20, 22, 31, 32, 48, 51, 55, 56, 58, 59, 63, 68, 69, 70, 73, 74, 75, 76, 85, 86, 87, 91, 92, 93, 94, 95, 96, 98, 104, and 105

Compounds that exhibited a serum TC lowering effect of 55% or higher among the above compounds:

Compounds of Examples 20, 22, 32, 48, 58, 75, 86, 94, 96, 98, and 105

Compounds that exhibited a serum TC lowering effect of 65% or higher among the above compounds:

Compounds of Examples 32, 48, 96, and 105

[Test Example 2] A serum TC lowering effect (a dose: 0.1 mg/kg)

Compounds that exhibited a serum TC lowering effect of 50% or higher at a dose of 0.1 mg/kg:

Compounds of Examples 1, 2, 6, 10, 16, 18, 20, 22, 24, 27, 28, 30, 31, 32, 33, 38, 45, 48, 49, 50, 51, 53, 55, 56, 58, 59, 60, 62, 63, 64, 65, 68, 69, 70, 71, 72, 73, 74, 75, 76, 80, 81, 82, 83, 85, 86, 87, 88, 89, 90, 91, 92, 93, 94, 95, 96, 97, 98, 99, 100, 101, 102, 104, and 105

Compounds that exhibited a serum TC lowering effect of 80% or higher among the above compounds:

Compounds of Examples 1, 2, 18, 20, 31, 32, 48, 51, 53, 55, 56, 58, 59, 60, 68, 69, 70, 74, 75, 76, 85, 86, 87, 88, 91, 92, 93, 94, 95, 96, 98, 99, 104, and 105

Compounds that exhibited a serum TC lowering effect of 110% or higher among the above compounds:

Compounds of Examples 32, 55, 56, 69, 76, 86, 87, 92, and 105

[Test Example 3] A serum LDL-C lowering effect (a dose: 0.01 mg/kg)

Compounds that exhibited a serum LDL-C lowering effect of 30% or higher at a dose of 0.01 mg/kg:

Compounds of Examples 2, 16, 18, 20, 22, 31, 32, 38, 48, 55, 56, 58, 63, 69, 70, 73, 75, 85, 86, 87, 92, 93, 94, 95, 96, 98, 104, and 105

Compounds that exhibited a serum LDL-C lowering effect of 55% or higher among the above compounds:

Compounds of Examples 2, 16, 18, 31, 48, 73, 94, 96, and 105

Compounds that exhibited a serum LDL-C lowering effect of 65% or higher among the above compounds:

Compounds of Examples 2, 18, 73, 96, and 105

[Test Example 4] A serum LDL-C lowering effect (a dose: 0.1 mg/kg)

Compounds that exhibited a serum LDL-C lowering effect of 50% or higher at a dose of 0.1 mg/kg:

Compounds of Examples 1, 2, 11, 12, 13, 15, 17, 18, 20, 22, 27, 30, 32, 33, 38, 42, 45, 48, 49, 51, 53, 55, 56, 58, 59, 60, 61, 62, 63, 64, 65, 68, 69, 70, 71, 72, 73, 74, 75, 76, 80, 81, 82, 83, 85, 86, 87, 88, 89, 90, 91, 92, 93, 94, 95, 96, 97, 98, 99, 100, 101, 102, 104, 105, and 106

Compounds that exhibited a serum LDL-C lowering effect of 80% or higher among the above compounds::

Compounds of Examples 1, 2, 11, 13, 17, 20, 32, 48, 51, 55, 56, 58, 59, 69, 70, 75, 76, 85, 86, 87, 88, 91, 92, 93, 94, 95, 96, 98, 99, 104, and 105

Compounds that exhibited a serum LDL-C lowering effect of 110% or higher among the above compounds:

Compounds of Examples 1, 2, 32, 51, 55, 56, 58, 76, 86, 92, and 105

[Test Example 5] A liver TC lowering effect (a dose: 0.01 mg/kg)

Compounds that exhibited a liver TC lowering effect of 30% or higher at a dose of 0.01 mg/kg:

Compounds of Examples 2, 16, 20, 22, 31, 32, 55, 56, 58, 59, 63, 68, 70, 73, 74, 75, 86, 87, 94, 96, 98, 99, and 104

Compounds that exhibited a liver TC lowering effect of 60% or higher among the above compounds:

Compounds of Examples 31, 32, 5.9, 68, 70, 73, 86, 94, 98, 99, and 104

Compounds that exhibited a liver TC lowering effect of 90% or higher among the above compounds:

Compounds of Examples 68, 73, 86, and 98

[Test Example 6] A liver TC lowering effect (a dose: 0.1 mg/kg)

Compounds that exhibited a liver TC lowering effect of 50% or higher at a dose of 0.1 mg/kg:

Compounds of Examples 1, 2, 14, 15, 20, 21, 22, 24, 28, 30, 31, 32, 38, 48, 51, 53, 54, 55, 56, 58, 59, 61, 62, 63, 70, 71, 74, 75, 80, 81, 84, 86, 91, 92, 93, 94, 95, 97, 98, 99, 102, 104, and 105

Compounds that exhibited a liver TC lowering effect of 80% or higher among the above compounds:

Compounds of Examples 1, 2, 14, 15, 20, 21, 22, 31, 32, 38, 48, 51, 53, 54, 55, 58, 61, 62, 63, 71, 80, 81, 86, 91, 92, 93, 95, 97, 98, 99, and 105

Compounds that exhibited a liver TC lowering effect of 110% or higher among the above compounds:

Compounds of Examples 1, 14, 48, 51, 53, 63, 71, 80, 92, 95, 97, 98, and 99

[Test Example 7] a serum TG lowering effect (a dose: 0.1 mg/kg)

Compounds that exhibited a serum TG lowering effect of 20% or higher at a dose of 0.1 mg/kg:

Compounds of Examples 3, 5, 6, 7, 8, 9, 10, 14, 15, 18, 31, 32, 34, 46, 48, 49, 50, 51, 52, 55, 58, 59, 60, 67, 75, 77, 92, 93, 99, 100, and 103

Compounds that exhibited a serum TG lowering effect of 25% or higher among the above compounds:

Compounds of Examples 3, 6, 8, 9, 10, 15, 34, 48, 49, 50, 51, 52, 55, 59, 60, 67, 77, 93, 99, 100, and 103

As a result of the study by this method on the effects of lowering serum TC and LDL-C in the hamsters fed with a high cholesterol diet which were given the test compounds for 28 days, a sustained TC lowering effect and/or an LDL-C lowering effect by the test compounds was observed.

[Comparative Example 1]

Measurement of lipid lowering effect in hamsters fed a high cholesterol diet:

It is shown as follow that the compound in which phenyl at 1-position of azetidinone ring was substituted with heteroaryl but not aryl unexpectedly exhibited a enhancing lipid lowering effect. The compounds in which pyridyl of compound of example 51 and 55 was exchanged for phenyl, were used as comparative compound 1 and 2, respectively. In the same method as Example 112, the suppression rate (%) of an increase in serum TC concentration of comparative compound 1 and 2 were calculated. The following Table 22 shows the suppression rate (%) of an increase in serum TC concentration of compound of example 51 and 55 and comparative compound 1 and 2.

[Table 22]

| | $Ar^3$ | | a serum TC lowering effect at a dose of 0.1 mg/kg |
|---|---|---|---|
| 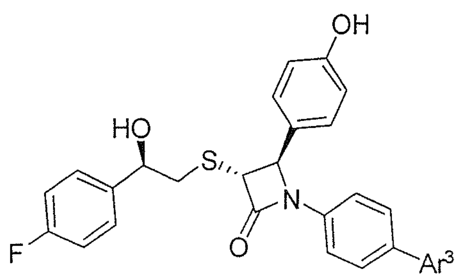 | 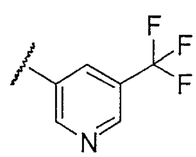 | Compound of Example 51 | 104% |
| | 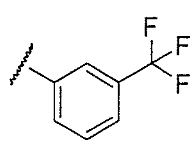 | Comparative Compound 1 | 20% |
| | 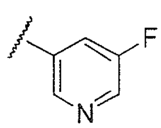 | Compound of Example 55 | 130% |
| | 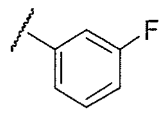 | Comparative Compound 2 | 34% |

As shown in Table 22, the compound in which phenyl at 1-position of azetidinone ring is substituted with heteroaryl exhibited a enhancing lipid lowering effect compared to the compound in which phenyl at 1-position of azetidinone ring is substituted with aryl.

[Example 113]

Measurement of lipid lowering effect in hamsters fed with a high fat diet:

A lipid lowering effect is measured also by the following methods.

Male Golden Syrian hamsters are divided into groups each consisting of 6 animals and fed a controlled high fat diet (containing 45 kcal% fat, Research Diet) for 28 days at most. From the start of feeding of the high fat diet, the test compounds are administered by gavage once daily. For the administration, 0.2 mL of corn oil alone per 100 g (control group) or a solution (or suspension) of the test compound in corn oil at a dose of 0.01 to 10 mg/kg was given. At the same time, a group fed with a normal diet during the same study period is handled as a normal group. 4 hours after the last administration, blood is collected from the abdominal aorta under light anesthesia with ether and serum is separated. Serum TC concentrations are determined by an enzyme method using L-type Wako CHO·H (Wako Pure Chemical Industries Ltd.) or Cholestest CHO (Daiichi Pure Chemicals Co., Ltd.). Serum TG concentrations are determined by an enzyme method using L-type Wako TG·H (Wako Pure Chemical Industries Ltd.) or Autosera S TG-N (Daiichi Pure Chemicals Co., Ltd.) and a free glycerine elimination method. Serum LDL-C concentrations are determined by a direct method using L-type Wako LDL-C·M (Wako Pure Chemical Industries Ltd.) or Cholestest LDL (Daiichi Pure Chemicals Co., Ltd.). Serum HDL-C concentrations are determined by a direct method using L-type Wako HDL-C·M (Wako Pure Chemical Industries Ltd.) or Cholestest N HDL (Daiichi Pure Chemicals Co., Ltd.). Human serum was commonly used as a sample for the calibration curve for determinations of serum TC concentration, TG concentration, LDL-C concentration, and HDL-C concentration. As a result, the test compounds exhibited a TC lowering effect, a TG lowering effect, an LDL-C lowering effect, or an HDL-C increasing effect.

The effects of the test compounds are calculated according to the equations described in Example 112 and the following equation.

[Equation 6]

$$\text{Serum HDL-C increasing rate (\%)} = \frac{\text{Serum HDL-C concentration in (B}-\text{A)}}{\text{Serum HDL-C concentration in A}} \times 100$$

In the Equation 6, A is the control group, and B is the test compound group.

[Example 114]

Analysis of LDL receptor expression in the liver of hamsters fed with a high cholesterol diet:

Male Golden Syrian hamsters are divided into groups each consisting of 6 animals and fed a controlled high cholesterol diet (CE-2 containing 0.5% cholesterol, CLEA Japan, Inc.) for 28 days at most. From the start of feeding of the high cholesterol diet, the test compounds are administered by gavage once daily. For the administration, 0.2 mL of corn oil alone per 100 g (control group) or a solution (or suspension) of the test compound in corn oil at a dose of 0.01 to 10 mg/kg was given. At the same time, a group fed with a normal diet during the same study period is handled as a normal group. 4 hours after the last administration, whole blood is collected from the abdominal aorta under light anesthesia with ether, the livers are then harvested, and the harvested livers are disrupted in Isogen (Nippon Gene Co., Ltd.). Nucleic acids are extracted from the disrupted livers and the total RNAs are purified with RN-easy Kit (Qiagen K.K.). After that, complementary DNAs (cDNAs) are synthesized by reverse transcription reaction and used as templates for real-time PCR. Primers (short DNA fragments) used for the real-time PCR are as shown below.
Primer that specifically amplifies LDL receptor:

5'-GATCCCCAACCTGAAGAACG-3' (SEQ ID NO: 1), and
5'-AGTCTACGGCCAGCCCATCT-3' (SEQ ID NO: 2)

Primer for 18S rRNA (small ribosome subunit) used as a housekeeping gene:

5'-ACTCAACACGGGAAACCTCA-3' (SEQ ID NO: 3), and
5'-AACCAGACAAATCGCTCCAC-3' (SEQ ID NO: 4)

Cyber Green kit (Applied Biosystem) is added to the templates and the above-described primers, and amplification of DNA by PCR (ABI-prism 7000) is measured over time to quantify the template DNA based on the amplification rate. The real-time PCR is conducted under the following conditions: 40 cycles of 50 °C for 2 minutes, 95 °C for 10 minutes, then 95 °C for 15 seconds, and 60 °C for 1 minute. The amounts of expression of the LDL receptors are calculated by correcting the amount of RNA with the amount of expression of the housekeeping gene. As a result, the enhanced expressions of the LDL receptor in the livers by the test compounds were observed.

[Industrial Applicability]

The 1-biarylazetidinone derivative represented by formula (I) and the pharmaceutically acceptable salt thereof according to the present invention have a cholesterol-lowering effect and/or a TG-lowering effect and can be used as drugs for the treatment, prevention of onset, or prevention of progress of lipid metabolism disorder, hyperlipidemia, or atherosclerosis, which is clinically applicable as a cholesterol-lowering agent.

## Claims

**1.** A compound represented by the following formula (I) or a pharmaceutically acceptable salt thereof:

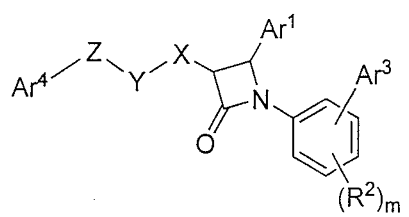

Formula (I)

wherein
X represents -S(O)$_r$- or -O-, with the proviso that r represents 0, 1, or 2;
$Ar^1$ represents aryl having 6 to 10 carbon atoms, or monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^1$;
$R^1$ represents a substituent independently selected from the group consisting of $C_1$-$C_6$ alkyl, $C_1$-$C_6$ alkyl substituted with one or more halogen, $C_1$-$C_6$ alkyl substituted with one or more -$OR^7$, halogen, cyano, nitro, -$OR^7$, -$OCOR^7$, $OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, -$NR^7SO_2R^{7a}$, -$SO_2NR^7R^{7a}$, -$S(O)_{0-2}R^7$, and glucuronide;
two independent $R^7$ or $R^7$ and $R^{7a}$ in the same $R^1$ may be bonded together to form 3 to 8-membered ring structure that may have 3 or less oxygen or nitrogen;
two adjacent independent $R^1$ may be bonded together to form 3 to 8-membered ring structure that may have 3 or less oxygen or nitrogen;
$R^2$ represents a substituent independently selected from the group consisting of $C_1$-$C_6$ alkyl, $C_1$-$C_6$ alkyl substituted with one or more halogen, halogen, cyano, and -$OR^7$;
m represents 0, 1, 2, 3, or 4;
$Ar^3$ represents monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^3$;
$R^3$ represents a substituent independently selected from the group consisting of $C_1$-$C_6$ alkyl, $C_1$-$C_6$ alkyl substituted with one or more halogen, $C_1$-$C_6$ alkyl substituted with one or more -$OR^7$, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, -$NR^7SO_2R^{7a}$, -$SO_2NR^7R^{7a}$, and -$S(O)_{0-2}R^7$;
two independent $R^7$ or $R^7$ and $R^{7a}$ in the same $R^3$ may be bonded together to form 3 to 8-membered ring structure that may have 3 or less oxygen or nitrogen;

two adjacent independent $R^3$ may be bonded together to form 3 to 8-membered ring structure that may have 3 or less oxygen or nitrogen;
$Ar^4$ represents an aryl group having 6 to 10 carbon atoms or monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^4$;
$R^4$ represents a substituent independently selected from the group consisting of $C_1$-$C_6$ alkyl, $C_1$-$C_6$ alkyl substituted with one or more halogen, $C_1$-$C_6$ alkyl substituted with one or more -$OR^7$, $C_1$-$C_6$ alkylene, halogen, cyano, nitro, -$OR^7$, -$OCOR^7$, -$OCOOR^7$, -$OCONR^7R^{7a}$, -$COOR^7$, -$COR^7$, $CONR^7R^{7a}$, -$NR^7R^{7a}$, -$NR^7COR^{7a}$, -$NR^7CONR^7R^{7a}$, -$NR^7SO_2R^{7a}$, -$SO_2NR^7R^{7a}$, -$S(O)_{0-2}R^7$, aryl having 6 to 10 carbon atoms, which may be substituted with 1 to 5 same or different $R^8$, and monocyclic or bicyclic heteroaryl having 1 to 9 carbon atoms with 1 to 4 heteroatoms selected from the group consisting of oxygen, sulfur, and nitrogen, which may be substituted with same or different 1 to 5 $R^8$;
two independent $R^7$ or $R^7$ and $R^{7a}$ in the same $R^4$ may be bonded together to form 3 to 8-membered ring structure that may have 3 or less oxygen or nitrogen;
two adjacent independent $R^4$ may be bonded together to form 3 to 8-membered ring structure that may have 3 or less oxygen or nitrogen;
Y represents a bond or -$CR^5R^{5a}$-;
$R^5$ and $R^{5a}$ each independently represents a substituent selected from the group consisting of hydrogen, $C_1$-$C_6$ alkyl, and halogen;
Z represents -$CR^6R^{6a}$-;
$R^6$ and $R^{6a}$ each independently represents a substituent selected from the group consisting of hydrogen, $C_1$-$C_6$ alkyl, $C_1$-$C_6$ alkyl substituted with one or more halogen, halogen, -$OR^7$, -$OCOR^7$, -$OCOOR^7$, -$OCONR^7R^{7a}$,-$NR^7R^{7a}$, -$NR^7COR^{7a}$, and -$NR^7CONR^7R^{7a}$, or $R^6$ and $R^{6a}$ may together form oxo group;
$R^7$ and $R^{7a}$ each independently represents hydrogen, $C_1$-$C_6$ alkyl, $C_1$-$C_6$ alkyl substituted with one or more halogen, $C_1$-$C_6$ alkyl substituted with one or more $C_1$-$C_6$ alkoxy, $C_1$-$C_6$ alkenyl, $C_1$-$C_6$ alkynyl, aryl having 6 to 10 carbon atoms, which may be substituted with 1 to 5 same or different $R^8$, monocyclic or bicyclic heteroaryl having 1 to 9 carbon atoms with 1 to 4 heteroatoms selected from the group consisting of oxygen, sulfur, and nitrogen, which may be substituted with 1 to 5 same or different $R^8$, or $C_7$-$C_{12}$ aralkyl, which may be substituted with 1 to 5 same or different $R^8$; and
$R^8$ represents $C_1$-$C_6$ alkyl, $C_1$-$C_6$ alkyl substituted with one or more halogen, $C_1$-$C_6$ alkyl substituted with one or more $C_1$-$C_6$ alkoxy, $C_1$-$C_6$ alkoxy, or halogen.

**2.** The compound or the pharmaceutically acceptable salt thereof according to claim 1, wherein
X represents -$S(O)_r$- or -O-, with the proviso that r represents 0, 1, or 2;
$Ar^1$ represents aryl having 6 to 10 carbon atoms, or monocyclic or bicyclic heteroaryl having 2 to 9 carbon atoms with 1 to 4 heteroatoms selected from the group consisting of oxygen, sulfur, and nitrogen, which may be substituted with 1 to 5 same or different $R^1$;
$R^1$ represents a substituent independently selected from the group consisting of $C_1$-$C_6$ alkyl, $C_1$-$C_6$ alkyl substituted with one or more halogen, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, -$NR^7SO_2R^{7a}$, and glucuronide;
$R^2$ represents a substituent independently selected from the group consisting of $C_1$-$C_6$ alkyl, $C_1$-$C_6$ alkyl substituted with one or more halogen, halogen, cyano, and -$OR^7$;
m represents 0, 1, 2, 3, or 4;
$Ar^3$ represents monocyclic or bicyclic heteroaryl having 2 to 9 carbon atoms with 1 to 4 heteroatoms selected from the group consisting of oxygen, sulfur, and nitrogen, which may be substituted with 1 to 5 same or different $R^3$;
$R^3$ represents a substituent independently selected from the group consisting of $C_1$-$C_6$ alkyl, $C_1$-$C_6$ alkyl substituted with one or more halogen, halogen, cyano, nitro, -$OR^7$, -$OCOR^7$, -$OCOOR^7$, -$OCONR^7R^{7a}$, -$COOR^7$, -$COR^7$, -$CONR^7R^{7a}$, -$NR^7R^{7a}$, -$NR^7COR^{7a}$, -$NR^7CONR^7R^{7a}$, and -$NR^7SO_2R^{7a}$;
$Ar^4$ represents aryl having 6 to 10 carbon atoms or monocyclic or bicyclic heteroaryl having 2 to 9 carbon atoms with 1 to 4 heteroatoms selected from the group consisting of oxygen, sulfur, and nitrogen, which may be substituted with 1 to 5 same or different $R^4$;
$R^4$ represents a substituent independently selected from the group consisting of $C_1$-$C_6$ alkyl, $C_1$-$C_6$ alkyl substituted with one or more halogen, $C_1$-$C_6$ alkylene, halogen, cyano, nitro, -$OR^7$, -$OCOR^7$, -$OCOOR^7$, -$OCONR^7R^{7a}$, - $COOR^7$, -$COR^7$, -$CONR^7R^{7a}$, -$NR^7R^{7a}$, -$NR^7COR^{7a}$, -$NR^7CONR^7R^{7a}$, and -$NR^7SO_2R^{7a}$;
two independent $R^7$ or $R^7$ and $R^{7a}$ in the same $R^4$ may be bonded together to form 3 to 8-membered ring structure that may have 3 or less oxygen or nitrogen;
two adjacent independent $R^4$ may be bonded together to form 3 to 8-membered ring structure that may have 3 or less oxygen or nitrogen;

Y represents a bond or $-CR^5R^{5a}-$;
$R^5$ and $R^{5a}$ each independently represents a substituent selected from the group consisting of hydrogen, $C_1$-$C_6$ alkyl, and halogen;
Z represents $-CR^6R^{6a}-$;
$R^6$ and $R^{6a}$ each independently represents a substituent selected from the group consisting of hydrogen, $C_1$-$C_6$ alkyl, $C_1$-$C_6$ alkyl substituted with one or more halogen, halogen, $-OR^7$, $-OCOR^7$, $-OCOOR^7$, $-OCONR^7R^{7a}$, $-NR^7R^{7a}$, $-NR^7COR^{7a}$, and $-NR^7CONR^7R^{7a}$, or $R^6$ and $R^{6a}$ may together form oxo group; and
$R^7$ and $R^{7a}$ each independently represents hydrogen, $C_1$-$C_6$ alkyl, $C_1$-$C_6$ alkyl substituted with one or more halogen, or $C_1$-$C_6$ alkyl substituted with one or more $C_1$-$C_6$ alkoxy.

**3.** The compound or the pharmaceutically acceptable salt thereof according to claim 1 or 2, wherein
$Ar^1$ represents phenyl, pyridyl, pyrimidyl, or pyridazinyl, which may be substituted with 1 to 5 same or different $R^1$, and
$R^1$ represents a substituent independently selected from the group consisting of $C_1$-$C_6$ alkyl, $C_1$-$C_6$ alkyl substituted with one or more halogen, halogen, $-OR^7$, $-COOR^7$, and glucuronide.

**4.** The compound or the pharmaceutically acceptable salt thereof according to claim 1 or 2, wherein $Ar^1$ represents phenyl or pyridyl, of which is substituted with at least one hydroxy.

**5.** The compound or the pharmaceutically acceptable salt thereof according to claim 1 or 2, wherein $Ar^1$ represents phenyl, of which at least 4-position is substituted with hydroxy.

**6.** The compound or the pharmaceutically acceptable salt thereof according to any of claims 1 to 5, wherein $R^2$ represents halogen.

**7.** The compound or the pharmaceutically acceptable salt thereof according to any of claims 1 to 5, wherein m represents 0.

**8.** The compound or the pharmaceutically acceptable salt thereof according to any of claims 1 to 7, wherein
$Ar^3$ represents pyridyl, pyrazyl, pyrimidyl, pyridazinyl, furanyl, thienyl, pyrrolidinyl, pyrazolyl, isoxazolyl, isothiazolyl, benzofuranyl, isobenzofuranyl, benzothienyl, indolyl, isoindolyl, benzothienyl, benzimidazolyl, or benzoxazolyl, which is bonded to 4-position of phenylene to which the $Ar^3$ is bonded and may be substituted with 1 to 5 same or different $R^3$, and
$R^3$ represents a substituent independently selected from the group consisting of $C_1$-$C_6$ alkyl, $C_1$-$C_6$ alkyl substituted with one or more halogen, halogen, cyano, $-OR^7$, $COOR^7$, $-COR^7$, $-CONR^7R^{7a}$, and $-NR^7R^{7a}$.

**9.** The compound or the pharmaceutically acceptable salt thereof according to any of claims 1 to 7, wherein
$Ar^3$ represents pyridyl, pyrazyl, or pyrimidyl, which is bonded to 4-position of phenylene to which the $Ar^3$ is bonded and may be substituted with 1 to 4 same or different $R^3$, and
$R^3$ represents a substituent independently selected from the group consisting of $C_1$-$C_6$ alkyl, $C_1$-$C_6$ alkyl substituted with one or more halogen, halogen, cyano, $-OR^7$, $-COR^7$, $-CONR^7R^{7a}$, and $-NR^7R^{7a}$.

**10.** The compound or the pharmaceutically acceptable salt thereof according to any of claims 1 to 7, wherein
$Ar^3$ represents 3-pyridyl, 2-pyrazyl, or 5-pyrimidyl, which is bonded to 4-position of phenylene to which the $Ar^3$ is bonded and may be substituted with 1 to 4 same or different $R^3$.

**11.** The compound or the pharmaceutically acceptable salt thereof according to any of claims 1 to 7, wherein
$Ar^3$ represents 3-pyridyl, which is bonded to 4-position of phenylene to which the $Ar^3$ is bonded and may be substituted with 1 to 4 same or different $R^3$, and
$R^3$ represents a substituent independently selected from the group consisting of $C_1$-$C_6$ alkyl, $C_1$-$C_6$ alkyl substituted with one or more halogen, halogen, cyano, $-OR^7$, $-COR^7$, $-CONR^7R^{7a}$, and $-NR^7R^{7a}$.

**12.** The compound or the pharmaceutically acceptable salt thereof according to any of claims 1 to 11, wherein
X represents -S-,
Y represents $-CH_2-$, and
Z represents -CH(OH)- or -CO-.

**13.** The compound or the pharmaceutically acceptable salt thereof according to any of claims 1 to 12, wherein
$Ar^4$ represents phenyl, pyridyl, furanyl, thienyl, thiazolyl, naphthyl, benzofuranyl, benzothienyl, or benzothiazolyl,

which may be substituted with 1 to 5 same or different $R^4$, and
$R^4$ represents a substituent independently selected from the group consisting of $C_1$-$C_6$ alkyl, $C_1$-$C_6$ alkyl substituted with one or more halogen, halogen, cyano, nitro, -$OR^7$, -$CONR^7R^{7a}$, -$NR^7R^{7a}$, -$NR^7COR^{7a}$, $C_1$-$C_4$ alkyleneoxy, and $C_1$-$C_3$ alkylenedioxy.

**14.** The compound or the pharmaceutically acceptable salt thereof according to any of claims 1 to 12, wherein
$Ar^4$ represents phenyl substituted with 1 to 5 same or different $R^9$, and
$R^4$ represents a substituent independently selected from the group consisting of $C_1$-$C_6$ alkyl, $C_1$-$C_6$ alkyl substituted with one or more halogen, halogen, cyano, hydroxy, $C_1$-$C_4$ alkyleneoxy, and $C_1$-$C_3$ alkylenedioxy.

**15.** The compound or the pharmaceutically acceptable salt thereof according to any of claims 1 to 12, wherein $Ar^4$ represents unsubstantiated benzofuranyl.

**16.** The compound or the pharmaceutically acceptable salt thereof according to claim 1 or 2, wherein
$Ar^1$ represents 4-hydroxyphenyl, 2,4-dihydroxyphenyl, 4-hydroxy-2-methylphenyl, 4-hydroxy-2-methoxyphenyl, or 2-chloro-4-hydroxyphenyl;
m represents 0;
$Ar^3$ represents pyridyl, pyrazyl, or pyrimidyl, which is bonded to 4-position of phenylene to which the $Ar^3$ is bonded and may be substituted with 1 to 4 same or different $R^3$;
$R^3$ represents a substituent independently selected from the group consisting of $C_1$-$C_6$ alkyl, $C_1$-$C_6$ alkyl substituted with one or more halogen, halogen, cyano, - $OR^7$, -$COR^7$, -$CONR^7R^{7a}$, and -$NR^7R^{7a}$;
$Ar^4$ represents phenyl substituted with 1 to 5 same or different $R^4$;
$R^4$ represents a substituent independently selected from the group consisting of $C_1$-$C_6$ alkyl, $C_1$-$C_6$ alkyl substituted with one or more halogen, halogen, cyano, hydroxy, $C_1$-$C_4$ alkyleneoxy, and $C_1$-$C_3$ alkylenedioxy;
X represents -S-;
Y represents -$CH_2$-; and
Z represents -CH(OH)- or -CO-.

**17.** The compound or the pharmaceutically acceptable salt thereof according to claim 1 or 2, wherein
$Ar^1$ represents 4-hydroxyphenyl, 2,4-dihydroxyphenyl, 4-hydroxy-2-methylphenyl, 4-hydroxy-2-methoxyphenyl, or 2-chloro-4-hydroxyphenyl;
m represents 0;
$Ar^3$ represents pyridyl, pyrazyl, or pyrimidyl, which is bonded to 4-position of phenylene to which the $Ar^3$ is bonded and may be substituted with 1 to 4 same or different $R^3$;
$R^3$ represents a substituent independently selected from the group consisting of $C_1$-$C_6$ alkyl, $C_1$-$C_6$ alkyl substituted with one or more halogen, halogen, cyano, - $OR^7$, -$COR^7$, -$CONR^7R^{7a}$, and -$NR^7R^{7a}$;
$Ar^4$ represents phenyl substituted with one or more halogen;
X represents -S-;
Y represents -$CH_2$-; and
Z represents -CH(OH)-.

**18.** The compound or the pharmaceutically acceptable salt thereof according to claim 1 or 2, wherein
$Ar^1$ represents 4-hydroxyphenyl, 2,4-dihydroxyphenyl, 4-hydroxy-2-methylphenyl, 4-hydroxy-2-methoxyphenyl, or 2-chloro-4-hydroxyphenyl;
m represents 0;
$Ar^3$ represents 3-pyridyl, 2-pyrazyl, or 5-pyrimidyl, which is bonded to 4-position of phenylene to which the $Ar^3$ is bonded and may be substituted with 1 to 4 same or different $R^3$;
$R^3$ represents a substituent independently selected from the group consisting of $C_1$-$C_6$ alkyl, $C_1$-$C_6$ alkyl substituted with one or more halogen, halogen, cyano, -$OR^7$, -$COR^7$, -$CONR^7R^{7a}$, and -$NR^7R^{7a}$;
$Ar^4$ represents phenyl substituted with 1 to 5 same or different $R^4$;
$R^4$ represents a substituent independently selected from the group consisting of $C_1$-$C_6$ alkyl, $C_1$-$C_6$ alkyl substituted with one or more halogen, halogen, cyano, hydroxy, $C_1$-$C_4$ alkyleneoxy, and $C_1$-$C_3$ alkylenedioxy;
X represents -S-;
Y represents -$CH_2$-; and
Z represents -CH(OH)-.

**19.** (3R,4R)-3-[(2R)-2-(4-fluorophenyl)-2-hydroxyethylthio]-4-(4-hydroxyphenyl)-1-[4-(pyridin-3-yl)phenyl]azetidin-2-one,

(3R,4R)-3-[(2R)-2-(4-fluorophenyl)-2-hydroxyethylthio]-4-(2,4-dihydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxyphenyl)-1-[4-(2-methoxypyridin-3-yl)phenyl]azetidin-2-one,
(3R,4R)-3-[(2R)-2-(4-fluorophenyl)-2-hydroxyethylthio]-4-(4-hydroxyphenyl)-1-[4-(pyrimidin-5-yl)phenyl]azetidin-2-one,
(3R,4R)-3-[(2R)-2-(4-fluorophenyl)-2-hydroxyethylthio]-4-(4-hydroxyphenyl)-1-[4-(4-methoxypyridin-3-yl)phenyl]azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbonitrile,
(3R,4R)-3-[(2R)-2-(4-fluorophenyl)-2-hydroxyethylthio]-4-(4-hydroxyphenyl)-1-[4-(6-methylpyridin-3-yl)phenyl]azetidin-2-one,
(3R,4R)-3-[(2R)-2-(4-fluorophenyl)-2-hydroxyethylthio]-1-[4-(6-dimethylaminopyridin-3-yl)phenyl]-4-(4-hydroxyphenyl)azetidin-2-one,
(3R,4R)-3-[(2R)-2-(4-fluorophenyl)-2-hydroxyethylthio]-4-(4-hydroxy-2-methoxyphenyl)-1-[4-(pyridin-3-yl)phenyl]azetidin-2-one,
(3R,4R)-3-[2-(4-fluorophenyl)-2-oxoethylthio]-4-(4-hydroxyphenyl)-1-[4-(pyridin-3-yl)phenyl]azetidin-2-one,
(3R,4R)-3-[(2R)-2-(3,4-dichlorophenyl)-2-hydroxyethylthio]-4-(4-hydroxyphenyl)-1-[4-(pyridin-3-yl)phenyl]azetidin-2-one,
(3R,4R)-3-[(2R)-2-(2,4-difluorophenyl)-2-hydroxyethylthio]-4-(4-hydroxyphenyl)-1-[4-(pyridin-3-yl)phenyl]azetidin-2-one,
(3R,4R)-3-((2R)-2-benzo[b]furan-5-yl-2-hydroxyethylthio)-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyridin-3-yl)phenyl]azetidin-2-one,
(3R,4R)-3-[(2R)-2-(2H-benzo[3,4-d]1,3-dioxolen-5-yl)-2-hydroxyethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(pyridin-3-yl)phenyl]azetidin-2-one,
(3R,4R)-3-[(2R)-2-hydroxy-2-(4-methylphenyl)ethylthio]-4-(4-hydroxyphenyl)-1-[4-(pyridin-3-yl)phenyl]azetidin-2-one,
(3R,4R)-3-[(2R)-2-(4-fluorophenyl)-2-hydroxyethylthio]-1-[4-(5-acetylpyridin-3-yl)phenyl]-4-(4-hydroxyphenyl)azetidin-2-one,
N-ethyl-5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carboxamide,
(3R,4R)-3-[(2R)-2-(4-fluorophenyl)-2-hydroxyethylthio]-4-(4-hydroxyphenyl)-1-[4-(5-methoxypyridin-3-yl)phenyl]azetidin-2-one,
(3R,4R)-3-[(2R)-2-(4-fluorophenyl)-2-hydroxyethylthio]-4-(4-hydroxyphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(4-hydroxyphenyl)-1-[4-(pyrazin-2-yl)phenyl]azetidin-2-one,
(3R,4R)-3-[(2R)-2-(4-fluorophenyl)-2-hydroxyethylthio]-1-[4-(5-fluoropyridin-3-yl)phenyl]-4-(4-hydroxyphenyl)azetidin-2-one,
(3R,4R)-3-[(2R)-2-(4-fluorophenyl)-2-hydroxyethylthio]-1-[4-(5-chloropyridin-3-yl)phenyl]-4-(4-hydroxyphenyl)azetidin-2-one,
5-(4-{(3R,4R)-3-[(2R)-2-(4-chlorophenyl)-2-hydroxyethylthio]-4-(4-hydroxyphenyl)-2-oxoazetidinyl}phenyl)pyridine-3-carbonitrile,
(3R,4R)-3-[(2R)-2-(4-fluorophenyl)-2-hydroxyethylthio]-4-(2-chloro-4-hydroxyphenyl)-1-[4-(pyridin-3-yl)phenyl]azetidin-2-one,
5-(4-{(3R,4R)-3-[(2R)-2-(4-chlorophenyl)-2-hydroxyethylthio]-4-(4-hydroxyphenyl)-2-oxoazetidinyl}phenyl)pyridine-2-carbonitrile,
(3R,4R)-3-[(2R)-2-(4-chlorophenyl)-2-hydroxyethylthio]-4-(2,4-dihydroxyphenyl)-1-[4-(pyridin-3-yl)phenyl]azetidin-2-one,
5-(4-{(3R,4R)-3-[(2R)-2-(4-fluorophenyl)-2-hydroxyethylthio]-4-(2,4-dihydroxyphenyl)-2-oxoazetidinyl}phenyl)pyridine-3-carbonitrile,
(3R,4R)-3-[(2R)-2-(4-fluorophenyl)-2-hydroxyethylthio]-4-(2,4-dihydroxyphenyl)-1-[4-(4-methoxypyridin-3-yl)phenyl]azetidin-2-one,
5-(4-{(3R,4R)-3-[(2R)-2-(4-chlorophenyl)-2-hydroxyethylthio]-4-(2,4-dihydroxyphenyl)-2-oxoazetidinyl}phenyl)pyridine-3-carbonitrile,
(3R,4R)-3-[(2R)-2-(4-fluorophenyl)-2-hydroxyethylthio]-4-(4-hydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyrimidin-5-yl)phenyl]azetidin-2-one,
(3R,4R)-3-[(2R)-2-(4-chlorophenyl)-2-hydroxyethylthio]-1-[4-(5-fluoropyridin-3-y1)phenyl]-4-(4-hydroxyphenyl)azetidin-2-one,
5-(4-{(3R,4R)-3-[(2R)-2-(4-fluorophenyl)-2-hydroxyethylthio]-4-(4-hydroxyphenyl)-2-oxoazetidinyl}phenyl)pyridine-3-carbaldehyde,
(3R,4R)-3-[(2R)-2-(4-chlorophenyl)-2-hydroxyethylthio]-4-(2,4-dihydroxyphenyl)-1-[4-(5-fluoropyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-fluoropyridin-3-yl)phenyl]azetidin-2-one,
(3R,4R)-3-[(2R)-2-(4-fluorophenyl)-2-hydroxyethylthio]-1-[4-(5-fluoropyridin-3-yl)phenyl]-4-(4-hydroxy-2-methylphenyl)azetidin-2-one,
(3R,4R)-3-[(2R)-2-(4-chlorophenyl)-2-hydroxyethylthio]=4-(2,4-dihydroxyphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-trifluoromethylpyridin-3-yl)phenyl]azetidin-2-one,
5-(4-{(3R,4R)-3-[(2R)-2-(4-fluorophenyl)-2-hydroxyethylthio]-4-(4-hydroxy-2-methylphenyl)-2-oxoazetidinyl}phenyl)pyridine-3-carbonitrile,
(3R,4R)-3-[(2R)-2-(4-chloro-3-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-chloro-3-fluorophenyl)-2-hydroxyethylthio]-1-[4-(5-fluoropyridin-3-yl)phenyl]-4-(4-hydroxyphenyl)azetidin-2-one,
(3R,4R)-3-((2R)-2-benzo[b]thiophen-5-yl-2-hydroxyethylthio)-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(4-hydroxyphenyl)-1-[4-(5-trifluoromethylpyridin-3-yl)phenyl]azetidin-2-one,
(3R,4R)-3-[(2R)-2-(4-chlorophenyl)-2-hydroxyethylthio]-1-[4-(5-chloropyridin-3-yl)phenyl]-4-(4-hydroxyphenyl)azetidin-2-one,
(3R,4R)-3-[(2R)-2-(4-chlorophenyl)-2-hydroxyethylthio]-1-[4-(6-dimethylaminopyridin-3-yl)phenyl]-4-(4-hydroxyphenyl)azetidin-2-one,
(3R,4R)-3-[(2R)-2-(4-chloro-2-hydroxyphenyl)-2-hydroxyethylthio]-4-(4-hydroxyphenyl)-1-[4-(pyridin-3-yl)phenyl]azetidin-2-one,
(3R,4R)-3-[(2R)-2-(2-chloro-4-fluorophenyl)-2-hydroxyethylthio]-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(6-methylpyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-chloropyridin-3-yl)phenyl]azetidin-2-one,
(3R,4R)-3-[(2R)-2-(4-fluorophenyl)-2-hydroxyethylthio]-4-(2,4-dihydroxyphenyl)-1-[4-(5-methylpyridin-3-yl)phenyl]azetidin-2-one,
(3R,4R)-3-[(2R)-2-(4-chlorophenyl)-2-hydroxyethylthio]-1-[4-(6-chloropyridin-3-yl)phenyl]-4-(4-hydroxyphenyl)azetidin-2-one,
(3R,4R)-3-[(2R)-2-(4-fluorophenyl)-2-hydroxyethylthio]-4-(2,4-dihydroxyphenyl)-1-[4-(6-methylpyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(5-chloropyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(5-methylpyridin-3-yl)phenyl]azetidin-2-one,
(3R,4R)-3-[(2R)-2-(4-chlorophenyl)-2-hydroxyethylthio]-4-(2,4-dihydroxyphenyl)-1-[4-(6-methylpyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(5-trifluoromethylpyridin-3-yl)phenyl]azetidin-2-one,
(3R,4R)-3-[(2R)-2-(4-fluorophenyl)-2-hydroxyethylthio]-4-(4-hydroxy-2-methylphenyl)-1-[4-(pyrimidin-5-yl)phenyl]azetidin-2-one,
(3R,4R)-3-[(2R)-2-(4-chloro-2-methoxyphenyl)-2-hydroxyethylthio]-4-(4-hydroxyphenyl)-1-[4-(pyridin-3-yl)phenyl]azetidin-2-one,
(3R,4R)-3-[(2R)-2-(4-chloro-2-methylphenyl)-2-hydroxyethylthio]-4-(4-hydroxyphenyl)-1-[4-(pyridin-3-yl)phenyl]azetidin-2-one,
(3R,4R)-3-[(2R)-2-(4-chlorophenyl)-2-hydroxyethylthio]-4-(4-hydroxyphenyl)-1-[4-(pyrazin-2-yl)phenyl]azetidin-2-one,
(3R,4R)-3-[(2R)-2-(4-chlorophenyl)-2-hydroxyethylthio]-1-[4-(5-acetylpyridin-3-yl)phenyl]-4-(4-hydroxyphenyl)azetidin-2-one, or a pharmaceutically acceptable salt thereof.

**20.** A pharmaceutical composition comprising the compound or the pharmaceutically acceptable salt thereof according to any of claims 1 to 19 as an active ingredient.

**21.** A cholesterol-lowering agent comprising the compound or the pharmaceutically acceptable salt thereof according to any of claims 1 to 19 as an active ingredient.

**22.** A medicament for lowering a cholesterol concentration in a blood comprising the compound or the pharmaceutically acceptable salt thereof according to any of claims 1 to 19 as an active ingredient.

**23.** A medicament for a treatment, a prevention of onset, or a prevention of progress of lipid metabolism disorder, hyperlipidemia, or atherosclerosis, comprising the compound or the pharmaceutically acceptable salt thereof according to any of claims 1 to 19 as an active ingredient.

**INTERNATIONAL SEARCH REPORT**

International application No.
PCT/JP2008/054252

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D401/10, A61K31/397, A61K31/404, A61K31/4427, A61K31/497, A61K31/506, A61K31/706, A61P3/06, A61P9/10, C07D401/14, C07D403/10, C07D405/14, C07D409/14, C07H15/26

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2007-520453 A (MICROBIA INC, US), 26 July, 2007 (26.07.07), & WO 2005/047248 A1 & US 2005/209165 A1 & EP 1682499 A1 | 1-23 |
| A | WO 2006/124713 A2 (MICROBIA INC, US), 23 November, 2006 (23.11.06), (Family: none) | 1-23 |
| A | JP 06-508637 A (SCHERING CORP), 29 September, 1994 (29.09.94), & EP 524595 A1 & WO 93/02048 A1 | 1-23 |
| A | JP 2006-501184 A (ASTRAZENECA AB), 12 January, 2006 (12.01.06), & WO 2004/005247 A1 & EP 1521742 A1 & US 2005/239766 A1 | 1-23 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 02 April, 2008 (02.04.08) | 15 April, 2008 (15.04.08) |
| Name and mailing address of the ISA/ Japanese Patent Office | Authorized officer |
| Facsimile No. | Telephone No. |

Form PCT/ISA/210 (second sheet) (April 2007)

INTERNATIONAL SEARCH REPORT

International application No.
PCT/JP2008/054252

C (Continuation). DOCUMENTS CONSIDERED TO BE RELEVANT

| Category* | Citation of document, with indication, where appropriate, of the relevant passages | Relevant to claim No. |
|---|---|---|
| A | WO 2006/137795 A1 (ASTRAZENECA AB, SE), 28 December, 2006 (28.12.06), (Family: none) | 1-23 |

Form PCT/ISA/210 (continuation of second sheet) (April 2007)

**INTERNATIONAL SEARCH REPORT**

International application No.
PCT/JP2008/054252

Continuation of A. CLASSIFICATION OF SUBJECT MATTER
(International Patent Classification (IPC))

*C07D401/10*(2006.01)i, *A61K31/397*(2006.01)i, *A61K31/404*(2006.01)i, *A61K31/4427*(2006.01)i, *A61K31/497*(2006.01)i, *A61K31/506*(2006.01)i, *A61K31/706*(2006.01)i, *A61P3/06*(2006.01)i, *A61P9/10*(2006.01)i, *C07D401/14*(2006.01)i, *C07D403/10*(2006.01)i, *C07D405/14*(2006.01)i, *C07D409/14*(2006.01)i, *C07H15/26*(2006.01)i

(According to International Patent Classification (IPC) or to both national classification and IPC)

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9508532 A
- WO 93002048 A
- WO 95026334 A
- WO 95035277 A
- WO 9616037 A
- WO 2005033100 A
- WO 2005047248 A
- WO 2005113495 A
- WO 2006124713 A
- WO 2006121861 A


### Non-patent literature cited in the description


- **André J. Tremblay.** *Arterioscler. Thromb. Vasc. Biol.,* 2006, vol. 26, 1101-1106
- **V.Charlton-Menys.** *Exp. Physiol.,* 2007, vol. 93 (1), 27-42
- **H Iso.** *Am. J. Epidemiol.,* 2001, vol. 153, 490-499
- **T Gordon.** *Arch Intern. Med.,* 1981, vol. 141, 1128-1131
- **Rele, S.M. et al.** *J. Org. Chem.,* 2004, vol. 69, 9159-9170